# EUROPEAN PATENT SPECIFICATION

(11) **EP 1 891 235 B1**
(45) Date of publication and mention of the grant of the patent: **03.11.2010**
(21) Application number: 06763551.6
(22) Date of filing: 07.06.2006
(51) Int. Cl.: C12Q 1/68

(54) **MUTATIONS ASSOCIATED WITH THE LONG QT SYNDROME AND DIAGNOSTIC USE THEREOF**
MIT DEM LONG-QT-SYNDROM ASSOZIIERTE MUTATIONEN UND DIAGNOSTISCHE VERWENDUNG DAVON
MUTATIONS ASSOCIEES AU SYNDROME DU QT LONG ET UTILISATION DIAGNOSTIQUE DE CELLES-CI

(30) Priority: 07.06.2005 IT MI20051047
(43) Date of publication of application: 27.02.2008
(73) Proprietor: Fondazione Salvatore Maugeri Clinica del Lavoro E della Riabilitazione I.R.C.C.S., 27100 Pavia (IT); Universita' Degli Studi Di Pavia, 27100 Pavia (IT)
(72) Inventor: PRIORI, Silvia Giuliana, I-20154 Milano (IT)
(74) Representative: Gervasi, Gemma
(86) International application number: PCT/EP2006/062956
(87) International publication number: WO 2006/131528

(56) References cited:
- WO-A2-2004/057030
- US-A- 5 599 673
- US-A1- 2005 003 445
- US-A1- 2005 130 190
- US-A1- 2005 142 591
- SCHULZE-BAHR E ET AL: "MOLECULAR GENETICS OF ARRHYTHMIAS - A NEW PARADIGM" ZEITSCHRIFT FUER KARDIOLOGIE, DEUTSCHE GESELLSCHAFT FUER KARDIOLOGIE, DE, vol. 89, no. SUPPL 4, 2000, pages IV-12, XP009029988 ISSN: 0300-5860

## Description

### Field of the invention

The invention relates to new genetic mutations in KCNQ1, KCNH2, SCN5A, KCNE1, KCNE2 genes and to diagnostic tests for their identification.

### State of the art

The Long QT Syndrome is an inherited disease predisposing to cardiac arrhythmias and sudden death at a young age. It is characterized by a prolonged QT interval on the electrocardiogram.

Two phenotypic variants have been recognized: an autosomal dominant variant known as Romano Ward Syndrome (RWS) and an autosomal recessive variant termed Jervell Lange Nielsen Syndrome (JLNS) (Romano C et al. Clin Ped 1963;45:656-657; Ward DC. J Irish Med Ass 1964; 54:103; Jervell A & Lange-Nielsen F. Am.Heart J 1957;54:59-61). More recently, two other forms with extra-cardiac involvement have been reported (Splawski I et al. Cell 2004;119:19-31; Plaster NM et al. Cell 2001;105:511-519).

The genetic loci associated with the first type of pathology have been located on chromosomes 3, 4, 7, 11 and 21 and the respective genes have been identified.

The gene associated with the LQT 1 locus is KCNQ1 (formerly termed KvLQT1), the gene associated with the LQT2 locus is KCNH2 (formerly termed HERG), the gene associated with the LQT3 locus is SCN5A, the gene associated with the LQT4 locus is ANK2, the gene associated with the LQT5 locus is KCNE1 (formerly termed minK) and finally the gene associated with the LQT6 locus is KCNE2 (formerly termed MIRP). All the genes involved encode ion channels (KCNQ1, KCNH2, SCN5A, KCNE1, KCNE2) except for the gene encoding the cardiac form of "ankyrin", a structural protein which anchors ion channels to the cell membrane (ANK2): however there are very few patients (4-5 families world-wide) showing an involvement of this protein, therefore it is not possible to perform genotype - phenotype relation studies in such a small population.

So far, several mutations in the genes encoding the five ion channel have been reported: for instance US2005/003445 describes mutations in KCNQ1 (or KvLQT1), KCNE1 (or Min K), KCNE2 (or MIRP), KCNH2 (or HERG) and SCN5A genes.

Currently, the diagnosis of Long QT Syndrome is primarily based on identification in a surface electrocardiogram of a heart-rate corrected QT prolongation (QTc ≥440 msec for males and QTc ≥460 for females). Prolongation of the QT interval may or may not be associated with symptoms linked to the presence of arrhythmias, such as syncopal episodes, however the finding of a prolonged QT interval remains the basic diagnostic element in the disease. However, epidemiological data have shown that only 70% of the subjects affected by this Syndrome displays a prolonged QT interval, therefore it can be deduced that genetic diagnosis is a fundamental tool for the diagnosis of disease at the presymptomatic stage. Moreover, since the type of underlying genetic defect affects the seriousness of the Long QT Syndrome and the response to the therapy, it is also evident the importance of molecular diagnosis for assessment of the arrhythmic risk and the following therapeutic choice.

However, a limit to the diffusion of molecular diagnosis is represented by the high number of mutations (for a list see http://pc4.fsm.it:81/cardmoc/) that can cause the disease, many of which are "private" mutations found in a single patient or family. So far, this has made necessary to screen the entire coding region (ORF) of all genes involved in the Syndrome. Such an approach is expensive and requires a very log time to formulate the report.

### Summary of the invention

The present disclosure relates to novel nucleotide mutations in KCNQ1, KCNH2, SCN5, KCNE1, KCNE2 genes that are associated with the full-blown Long QT Syndrome or are associated with the predisposition to said syndrome or with the susceptibility to develop arrhythmias during exposure to trigger-events (food, drugs), and to a method to identify such mutations. Said mutations affect the coding region of the above defined genes and always result in corresponding amino acid changes, leading to the expression of variant proteins with altered functionality compared to *wild type* proteins.

The invention relates to a method for identification of about 40% of the carriers of the Long QT Syndrome or of carriers of a predisposition to said Syndrome. Such method involves the detection of a group of about 70 non-private mutations as listed in claim 1. According to a further aspect, the disclosure relates to a method for identification of about 20% of the carriers of the Long QT Syndrome or of carriers of a predisposition to said syndrome, comprising the detection of a further selection of about 20 non-private mutations. The detection is performed by well known techniques for identification of point mutations, insertions, deletions, duplications.

Nucleic acids containing previously unreported mutations, vectors containing said nucleic acids and cells transformed with said vectors are also included in the invention.

According to a further aspect, the invention relates to the detection, at the amino acid sequence level, of novel mutations or of different groups of non-private mutations.

### Description of the figures

Figure 1. Figure 1 shows the distribution of QT intervals in subjects not affected by LQTS and, in red, in subjects affected by LQTS. A wide overlap of the QT interval duration curve between affected and non affected subjects is noticed, therefore only genetic analysis can allow a correct diagnosis in affected subjects with a QT within normal limits.
Figure 2. Nucleotide and amino acid sequences of *wild type* KCNQ1 cDNA.
Figure 3. Nucleotide and amino acid sequences of *wild type* KCNH2 cDNA.
Figure 4. Nucleotide and amino acid sequences of *wild type* SCN5 cDNA.
Figure 5. Nucleotide and amino acid sequences of *wild type* KCNE1 cDNA.
Figure 6. Nucleotide and amino acid sequences of *wild type* KCNE2 cDNA.

### Detailed description of the invention

For the purpose of the present invention, the following definitions have been used: ***Mutation:*** for the purpose of the present invention, it is meant by mutation, unless otherwise indicated, any change of the nucleotide sequence, involving one or more nucleotides, hence including a permutation, insertion or deletion that is absent in the DNA from control individuals or in wild type DNA corresponding to the cDNA sequences deposited in the GenBank with accession numbers: AF00571 (cDNA KvLQT1; gene: KCNQ1: AJ006345), NM005136 (cDNA: MiRP; gene: KCNE2, AB009071), NM000335 (cDNA: Nav1.5; gene: SCN5A NT 022517.17), NM000238 (cDNA: HERG; gene: KCNH2: NT 011512.10), NM00219 (KCNE1; gene: AP000324), resulting also in an amino acid sequence change in the encoded protein.

In the present invention, unless otherwise indicated, the positions of a single mutated nucleotide or of several mutated nucleotides are indicated with the number of the mutated nucleotide (like, for instance, in table 1 where the reference of the identifying sequence can be found, with the mutated nucleotide underlined) or with the respective codon or with the amino acid affected by the mutation: therefore, the name of the P345 mutation refers to the amino acid change, which is proline in *wild type,* as well as to the mutation of one of the nucleotides of the proline codon at position 345 of the amino acid sequence: in this case, the specific mutation of the nucleotide sequence is also reported.

In the present invention, the definitions **"locus"** and **"gene"** are used interchangeably and they correspond to, respectively: LQT1 locus - KCNQ1 (o KvLQT1) gene, LQT2 locus - KCNH2 (o HERG) gene, LQT3 locus - SCN5A gene, LQT5 locus - KCNE1 (o minK) gene, LQT6 locus - KCNE2 (o MiRP) gene.

QTc by QTc it is meant the QT interval corrected for the heart rate expressed as RR interval according to the formula QTc =QT / v RR.

**IQR**: Interquartile range. Values corresponding to the 75th percentile and the 25th percentile of a variable are reported under this definition.

**Long QT Syndrome (QTS)** Two phenotypic variants of the Long QT Syndrome have been recognized : an autosomal dominant variant known as Romano Ward Syndrome (RWS) and an autosomal recessive variant termed Jervell Lange Nielsen Syndrome (JLNS). In addition, two other forms with extra-cardiac involvement have been reported.

**ORF**: Open Reading Frame = the coding portion of a gene.

The present invention is based on the identification of new mutations in KCNQ1 (also termed KvLQT1), KCNH2 (also termed HERG), SCN5A, KCNE1 (also termed minK), KCNE2 (also termed MiRP) genes encoding ion channels involved in the control of cardiac electrical activity and particularly in generation of the cardiac action potential. A genetically based dysfunction of the proteins encoded by these genes can cause the Long QT Syndrome.

The oligonucleotides of the disclosure, and/or their complementary sequences, are chemically synthesized and can comprise chemically modified nucleotides (for instance phosphorothioated nucleotide) or a fluorochrome or chromophore label, preferably at the 5' and/or 3' terminus.

Such oligos can be used for gene amplification reactions or for hybridization, in homogeneous or heterogeneous phase: they can be used as such or in a form bound to a solid matrix or a two-dimensional or three-dimensional support, for instance a membrane, or to the bottom of a well in a plate or to a microchip.

The nucleic acids of the disclosure are double or single stranded: wherein single stranded molecules include also oligonucleotides and complementary DNA (cDNA) or antisense DNA.

The nucleic acids of the disclosure, particularly the cDNA and its fragments, comprising at least one mutation according to the invention, can be cloned into vectors, for instance expression vectors for the production of high amounts of recombinant protein useful for functional characterization of different variants or to set up immunoassays.

Therefore, vectors containing the nucleotide sequences and cells transformed with such vectors, and expressing the mutant proteins, are also comprised in the invention.

The nucleic acids and proteins of the disclosure are for diagnostic use in the Long QT Syndrome, particularly in the Romano Ward Syndrome and/or the Jervell Lange-Nielsen Syndrome in *in vitro* methods. Moreover, the recombinant proteins and their fragments comprising the mutation are useful for production of specific antibodies against the mutant protein, which are able to specifically bind the mutated but not the *wild type* protein. Together with the proteins, said antibodies are used to set up diagnostic immunoassays *in vitro.*

The sample is preferably represented by nucleic acids purified from a biological sample, such as cells obtained from biological fluids, as for instance blood or other tissues. The nucleic acids are preferably genomic DNA or mRNA. In the latter case, the sample can be retrotranscribed into cDNA prior to sequence analysis.

A first aspect of the invention relates to a diagnostic method based on the identification of a group of about 70 non-private mutations in KCNQ1, KCNH2 and SCN5A genes, selected among new mutations shown in Table 1, and mutations well known in the art, selected among those detected by the authors of the present invention as listed claim 1, which occur at high rate in a statistically significant sample of probands and which are able to identify about 40% of the probands.

According to a further aspect of the disclosure the method to diagnose the Long QT Syndrome of RW and/or Jervell Lange type, or the genetic predisposition to said syndrome which represents the genetic cause of QT interval alterations found in an electrocardiogram, comprises at least the identification of *hot spot* mutations as defined below. *Hot spot* mutations are the most commonly found according to the population studied and occur in at least 3 or more clinically affected individuals of different families. In the KCNQ1 gene, such *hot spot* mutations affect the codons encoding for: R190, preferably R190W, where even more preferably W is encoded by the corresponding codon in sequence SEQ ID NO: 17, R231C and more preferably R231H, where even more preferably H is encoded by the corresponding codon in sequence SEQ ID NO: 24, V254 more preferably V254M and V254L, where even more preferably L is encoded by the corresponding codon in sequence SEQ ID NO: 26, and so on according to the preferred embodiments enlisted in Table 2 for the following mutations in the KCNQ1 gene: G269, S277, G314, A341, A344; in the KCNH2 gene in codons: A561, G572 (preferably identified by sequence SEQ ID NO: 97), G628; in the SCN5A gene in codons P1332 and E1784.

*Hot spot* mutations characterize 24% of the probands with electrocardiographic alterations; therefore the present invention comprises a method for identification of *hot spot* mutations as defined in the present invention which make use of methods well known in the art. In the KCNQ1 gene, the rate of said *hot spot* mutations in the sample is the following for each indicated codon: 190 (n=12), 231 (n=4) 254 (n=4), 269 (n=4), 277 (n=5), 314 (n=4), 341 (n=6), 344 (n=9), in the KCNH2 gene it is for codon 561 (n=7), 572 (n=4) and 628 (n=7); in the SCN5A gene is the following for each indicated codon: 1332 (n=5) and 1784 (n=3).

It should be noted that mutations in KCNQ1 and KCNH2 (LQT1 and LQT2) genes are more common in patients with Long QT Syndrome; they account for the genetic cause in 90% of these pathologies.

The preferential search identification of mutations in one of the *hot spot* codons, or of the mutations listed in Table 2, allows a rapid and highly cost-effective diagnosis of the Long QT Syndrome, with remarkable reduction of costs and expansion of the diagnostic potential to the general population.

In a particularly preferred aspect, the invention relates to a method for the molecular diagnosis (carried out on the nucleic acids of the patient) of the Long QT Syndrome, particularly the inherited forms Romano-Ward and/or Jervell Lange, comprising the identification of a group of non-private mutations (i.e. found in at least two individuals belonging to different families) affecting the following codons or groups of codons: as listed in claim 1

where the presence of a mutation in at least one of the above-mentioned codons or positions indicates the presence of a functional abnormality of the ion channel and that the subject carrying such mutation is affected by or predisposed to the Long QT Syndrome, preferably of the Romano-Ward and/or Jervell Lange type.

Table 2 outlines the group of most representative mutations used for the diagnostic methods described above.

In a further embodiment the disclosure also relates to a two-dimensional or three-dimensional support comprising oligonucleotides capable of selectively detecting the mutations defined in Table 2. According to an even more preferred embodiment, said support comprises polynucleotides or oligonucleotides comprising at least one of the preferred nonanucleotides chosen from those that are most commonly found in the sequences of the probands and reported in Table 1: SEQ ID NO: 13, SEQ ID NO: 16, SEQ ID NO: 17, SEQ ID NO: 21, SEQ ID NO: 24, SEQ ID NO: 26, SEQ ID NO: 27, SEQ ID NO: 28, SEQ ID NO: 29, SEQ ID NO: 34, SEQ ID NO: 39, SEQ ID NO: 40, SEQ ID NO: 41, SEQ ID NO: 46, SEQ ID NO: 55, SEQ ID NO: 56, SEQ ID NO: 59, SEQ ID NO: 67, SEQ ID NO: 69, SEQ ID NO: 70, SEQ ID NO: 71, SEQ ID NO: 75, SEQ ID NO: 79, SEQ ID NO: 80, SEQ ID NO: 85, SEQ ID NO: 88, SEQ ID NO: 97, SEQ ID NO: 98, SEQ ID NO: 99, SEQ ID NO: 106, SEQ ID NO: 110, SEQ ID NO: 111, SEQ ID NO: 113, SEQ ID NO: 125, SEQ ID NO: 128, SEQ ID NO: 133, SEQ ID NO: 134, SEQ ID NO: 140, SEQ ID NO: 142, or their complementary sequences. Alternatively, an expert in the field can design the sequence of oligonucleotides capable of detecting the mutations defined in the present invention, for example by software tools.

According to a further embodiment, the disclosure comprises a support wherein polynucleotides and/or oligonucleotides include at least one of the nonanucleotides with sequence from SEQ ID NO: 11 to SEQ ID NO: 149 or at least one of their complementary oligonucleotides.

Table 2 shows both the position of the mutated nucleotide and the position of the codon which, as result of the mutation, is different from the *wild type.* In Tables 1 and 2 it is also possible to identify, in patients affected by the Long QT Syndrome, one or more amino acids preferably found as result of mutations in the codons of a gene.

According to a further aspect the disclosure comprises the use of a oligonucleotide comprising anyone of the nonamer with wild type sequence corresponding to those identified in SEQ ID NO: 11-149 and in Table 2 for the diagnosis of a full-blown Long QT Syndrome or for the diagnosis of a genetic predisposition to the Long QT Syndrome in *in vitro* methods.

For the purpose of the present invention it is intended to be comprised within the disclosure of the present invention any nucleotidic mutation leading to any amino acid change different from *wild type,* in the same position, as herein disclosed, with the exclusion of the mutations already well-known (see references of Table 2) regardless of the codons that are preferably generated as the result of the specific mutation.

Thus, just as an example, the present invention comprises all the mutations affecting, in the case of the KCNQ1 gene, the histidine codon at position 258 (*wild type*) which, according to the invention, changes from a histidine codon to a different codon that, according to a preferred embodiment, is an arginine (Arg or R) codon, if the mutation affects the second nucleotide of the CAC codon (His), thus changing from A into G (A→G) and producing a CGC codon which encodes for Arg; in the same starting codon a different change, C→A produces a AAC codon which enclodes for Asp as result of a mutation of the first nucleotide of the codon. In this case the mutation according to the invention identifies any amino acid at position 258 that is different from histidine, and preferably identifies arginine or asparagine. Table 2 shows the preferred embodiments for each mutation.

All nucleotide mutations (generally in the third-nucleotide of the codon) which, due to the degeneracy of the genetic code, change the codon giving rise to the same amino acid, identical to the amino acid preferred in the protein sequence, are also intended to be comprised in the present invention. Just as an example, in the case of the mutation of codon 258 in the KCNQ1 gene, already used in the previous example, all the permutations, due to genetic code degeneracy, that change the CAC codon into any of the codons encoding Arginine, or, in the second case, into any of the codons encoding Asp, are intended to be comprised in the invention. Each preferred embodiment of the mutations identified as being related to the Long QT Syndrome found and used in the method of the invention enlisted in Table 1 which reports the mutated nucleotide and/or amino acid according to the nucleotide or amino acid numbering of the corresponding *wild type* gene sequence which is reported as annex in the Sequence List from SEQ ID NO: 1 to 10.

The identification, according to the invention, of the most representative mutations in subjects at risk for the Long QT Syndrome can be carried out also on the protein product, corresponding to the various amino acid variants, using methods well known in the art, as for instance specific antibodies or differences in the electrophoretic migration pattern.

Based on the findings of the authors of the present invention, which have been also confirmed in an independent sample, at least 40% of mutation carriers (or probands) carries a mutation of one of the codons or one of the mutations reported in Table 2. Therefore, this molecular method represents the first level of molecular screening for the Long QT Syndrome.

The molecular method according to the invention involves also a second level of investigation carried out preferentially on subjects that are found to be negative at the first level screening. Such second level of investigation consists in the characterization of sequences of the Open Reading Frames in KCNQ1 and KCNH2 genes. Finally, the molecular method according to the invention involves a third level for subjects that turned out to be negative in the second level, comprising the analysis of the genes responsible for the less prevalent genetic variants of LQTS, that is for SCN5A, KCNE1 and KCNE2 genes. Said third level can include a confirmation of the sequences of SCN5A, KCNE1 and KCNE2 gene ORFs, for instance by direct sequencing following gene amplification with primer oligonucleotides which can be derived, by methods well known in art, from the published sequence. According to a preferred embodiment, the primers used for direct sequencing of exons are listed in Table 4.

The identification performed with molecular methods according to the present invention can be associated with other measurements or other clinical diagnostic/prognostic methods.

In this respect, the authors of the present invention have evaluated in parallel the sensitivity and specificity of several QTc cut-off values: a cut-off value of 440 ms turned out to have 81% specificity, 89% sensitivity and 91% positive predictive accuracy for the diagnosis of Long QT Syndrome. Instead, specific cut-off values for gender (=440 ms for males and = 460 ms for females) proved to be very specific (96%) but not very sensitive (72%).

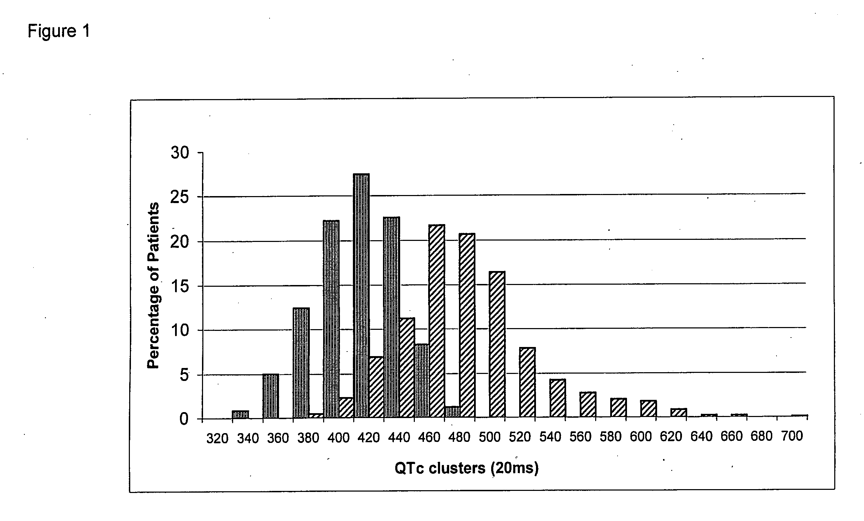

QTc duration correlates with the presence of genetic mutations: it is in fact decreasingly long for probands, family members carrying the disease at the genetic level and healthy family members (p<0.0001; Table 5). However the distribution of QTc values is very similar among individuals affected and unaffected by genetic mutations, even though the epidemiological data have shown that only 70% of subjects affected by the Syndrome has a prolonged QT interval (the overlap between the two populations is shown in Figure 1). Therefore, it is demonstrated that the genetic diagnosis is an important tool for diagnosis of the disease at the presymptomatic stage. Moreover, since the type of underlying genetic defect affects the severity of the long QT Syndrome and the response to therapy, it is also evident the importance of molecular diagnosis for assessment of the arrhythmic risk and the following therapeutic choice (drugs, implantable defibrillator).

The data provided in the Table 3 and shown in Figure 1, highlight the penetrance values of the disease (i.e. the percentage of carriers showing a prolongation of the QT interval in the surface electrocardiogram): from these data it is deduced that 30% of the carriers of at least one mutation according to the invention have a QT interval that is not different from normal. Therefore the effectiveness of a molecular screening for genotyping, like the one proposed here, is clear. Where necessary, such screening can be also coupled to a further investigation at the level of population screening, in order to identify genetic defects at birth, and/or identify iatrogenic long QT susceptibility, and/or screen competitive athletes and other populations in which the identification of a subclinical form of congenital long QT can prevent arrhythmic events, cardiac arrest and sudden cardiac death. This is made possible by the identification of a susceptibility to develop arrhythmias during exposure to trigger events (food, drugs etc) and the avoidance of conditions known to entail a higher risk, as for instance harmful life style habits, use of drugs and food/drinks contraindicated in subjects carrying such genetic defects.

The three levels of investigation in the method of the invention allow a significant saving of time and reagents: the first level of investigation is limited to the screening of about 70 mutations identified in Tables 2 A and 2B which are present in at least 40% of the patients that can be genotyped on the basis of mutations found so far. This way, a quick diagnosis is obtained that has limited costs and is nevertheless significant. Such analysis can be easily performed also in the forms of genetic screening at birth, screening for competitive athletes or screening for patients that need to be treated with drugs that can cause a prolongation of then QT interval. In fact, in all these categories of patients and sports persons, the analysis of the whole codifying portion of all disease genes is possible but is not used on a routine basis due to costs and excessive time length required.

The molecular method in its various embodiments makes use of well known methods for identification of mutations. Any method for detection of nucleotide mutations in a nucleic acid sequence can be used on the basis of the sequence information herein provided.

Among well known methods, the following are reported, although the list is not exhaustive: a) recognition of an enzymatic digestion pattern based for instance on the use of restriction enzymes by which the DNA fragment derived from the sample is selectively cut generating alternative patterns for mutated and "*wild type*" sequences, b) use of direct sequencing of nucleic acids, c) use of methods based on hybridization (or base pairing) with homologous or highly homologous sequences, d) use of the selective removal of specific sequences by methods of chemical or enzymatic breakage. The above techniques may or may not be used in association with steps of gene amplifications and may comprise, according to a particularly preferred embodiment, the use of solid platforms (microchip) with high/medium or low density binding of oligonucleotide probes (microarrays). Among hybridization-based methods beside the Southern Blotting technique, the following methods can also be used: Single Strand Conformation Polymorphism (SSCP Orita et al. 1989), *clamped* denaturing gel electrophoresis (CDGE, Sheffield et al. 1991), Denaturing Gradient Gel Electrophoresis (DGGE), heteroduplex analysis (HA, White et al. 1992), Chemical Mismatch Cleavage (CMC, Grompe et al., 1989), ASO (Allele Specific Oligonucleotides), RNase protection method (DB Thompson and J Sommercorn J. Biol. Chem., Mar 1992; 267:5921 - 5926) and TCGE temporal gradient capillary electrophoresis Integrated platform for detection of DNA sequence variants using capillary array electrophoresis (Qingbo Li et al., Electrophoresis 2002, vol. 23, 1499-1511.

In the case of DNA direct sequencing (genes and ORFs) the primers used according to a preferred embodiment are those reported in Table 4. Insertions and deletions can be identified by methods well known in the art, such as RFLP (Restriction Fragment Length Polymorphism). Other methods are reported, for instance, in Sambrook et al. Molecular Cloning: a Laboratory Manual, Cold Spring Harbor Laboratory Press NY. USA, 1989, or in Human Molecular Genetics, ed Strachan T. and Read A.P., 2nd ed. 1999, BIOS Scientific Publisher.

In a preferred embodiment the method of the invention, when based on nucleic acid hybridization, comprises the use of oligonucleotide probes derived from KCNQ1, KCNH2, SCN5A, KCNE1, KCNE2 genes, and comprising the nonanucleotides with sequence from SEQ ID NO: 11 to SEQ ID NO: 149 in the Sequence List or their complementary sequences, or oligonucleotides suitable to identify the mutations disclosed in the present invention.

Alternatively, the invention relates to oligonucleotides with *wild type* sequence, which are however suitable to distinguish, in a biological sample under appropriate conditions of hybridization stringency, the mutations or groups of mutations according to the invention because they cannot perfectly match with the mutated sequence present in the sample.

Considering that about 10-15% of subjects with iatrogenic (i.e. caused by drugs) torsions of the tip and sudden death show a Long QT Syndrome mutation as genetic substratum (Yang P et al Circulation. 2002 Apr 23;105(16):1943-8, Napolitano et al J Cardiovasc Electrophysiol. 2000 Jun;11(6):691-6), one embodiment of the proposed method is definitively the identification of subjects with contraindication for drugs that prolong the QT interval by blocking the Ikr current (the so-called" pre-prescription genotyping"). These include antibiotics, prokinetic drugs, antipsychotic drugs, antidepressants, antiarrhythmic drugs and drugs belonging to other therapeutic classes.

Therefore, the method of the invention allows to avoid the risks associated with drug administration to subjects carrying the subclinical form of the Long QT Syndrome and to assess the sensitivity of a subject to the following drugs:
Albuterol, Alfuzosin, Haloperidol, Amantadine, Amiodarone, Amitriptyline, Amoxapine, Amphetamine/dextroamphetamine, AmpicillinArsenic trioxide, Atomoxetine, Azithromycin, Bepridil, Quinidine, Chloral hydrate, Chloroquine, Chlorpromazine, Ciprofloxacin, Cisapride, Citalopram, Clarithromycin, Clomipramine, Cocaine, Desipramine, Dextroamphetamine, Disopiramide, Dobutamine, Dofetilide, Dolasetron, Domperidone, Dopamine, Doxepin, Droperidol, Ephedrine, Epinephrine, Erythromycin, Felbamate, Fenfluramine, Phentermine, Phenylephrine, Phenylpropanolamine, Flecainide, Fluconazole, Fluoxetine, Foscarnet, Fosphenytoin, Galantamine, Gatifloxacin, Gemifloxacin, Granisetron, Halofantrine, Ibutilide, Imipramine, Indapamide, Isoproterenol, Isradipine, Itraconazole, Ketoconazole, Levalbuterol, Levofloxacin, Levomethadyl, Lithium, Mesoridazine, Metaproterenol, Methadone, Methylphenidate, Mexiletine, Midodrine, Moexipril/HCTZ, Moxifloxacin, Nicardipine, Norepinephrine, Nortriptyline, Octreotide, Ofloxacin, Ondansetron, Paroxetine, Pentamidine, Pimozide, Procainamide, Procainamide, Protriptyline, Pseudoephedrine, Quetiapine, Risperidone, Ritodrine, Roxithromycin, Salmeterol, Sertraline, Sibutramine, Solifenacin, Sotalol, Sparfloxacin, Tacrolimus, Tamoxifen, Telithromycin, Terbutaline, Thioridazine, Tizanidine, Trimethoprim-Sulfamethoxazole, Trimipramine, Vardenafil, Venlafaxine, Voriconazole, Ziprasidone, where the presence of at least one of the mutations identified in Table 1 or of one of the mutations identified in Table 2 indicates a sensitivity to one of the drugs mentioned above.

The assay can also be used to identify a possible susceptibility to develop arrhythmias during exposure to trigger events other than drugs (e.g. food).

Also disclosed are kits for the realization of the diagnostic or prognostic methods according to each of the aspects described above. Therefore, said kits are obtained according to a preferred embodiment comprising at least one of the oligonucleotides in Table 1, having sequence from SEQ ID NO: 11 to SEQ ID NO: 149 (mutant sequences), and/or their complementary sequences, and optionally other reagents such as buffers, enzymes, etc. necessary to carry out the method of the invention. According to a preferred embodiment, the kit comprises a set of at least 2 oligonucleotides each including at least one of the following nonanucleotides (identified by the SEQ ID NO) or of their complementary sequences, where said nonanucleotides are chosen from:
- KCNQ1 gene or locus: SEQ ID NO: 13 (L137), SEQ ID NO: 16 (R174P), SEQ ID NO: 17 (R190W), SEQ ID NO: 21 (I204M), SEQ ID NO: 24 (R231H), SEQ ID NO: 26(V254L), SEQ ID NO: 27(H258N), SEQ ID NO: 28 (H258R), SEQ ID NO: 29 (L262V), SEQ ID NO: 34 (V280E), SEQ ID NO: 39 (T322M), SEQ ID NO: 40 (P343L), SEQ ID NO: 41 (P343R), SEQ ID NO: 46 (R360T), SEQ ID NO: 55 (R518G), SEQ ID NO: 56 (R518P), SEQ ID NO: 59 (I567T), SEQ ID NO: 52, SEQ ID NO: 53;
- KCNH2 gene or locus: SEQ ID NO: 67 (Y43C), SEQ ID NO: 69 (E58A), SEQ ID NO: 70 (E58G), SEQ ID NO: 71 (E58D), SEQ ID NO: 75 (dellAQ), SEQ ID NO: 85 (W412stop), SEQ ID NO: 88 (S428L), SEQ ID NO: 97 (G572D), SEQ ID NO: 98 (R852L), SEQ ID NO: 99 (D609H), SEQ ID NO: 106 (S660L), SEQ ID NO: 113 (S818P), SEQ ID NO: 79, SEQ ID NO: 80, SEQ ID NO: 110, SEQ ID NO: 111, SEQ ID NO: 125, SEQ ID NO: 126, SEQ ID NO: 128;
- SCN5A gene or locus: SEQ ID NO: 133 (A413E), SEQ ID NO: 134 (A413T), SEQ ID NO: 140 (R1644C), SEQ ID NO: 142 (Y1767C).

Alternatively the kit comprises oligonucleotides suitable to detect a group of at least 20 of the mutations reported in Table 2, where said oligonucleotides are designed with methods or software well known in the art. According to a preferred embodiment, the kit to realize the diagnostic method of the invention comprises oligonucleotides suitable to detect the following mutations, identified, according to the numbering of codons or amino acids in the KCNQ1 gene:
L137F, where F is preferably identified with the corresponding codon in SEQ ID NO: 13; R174C and R174P, where P is preferably identified with the corresponding codon in SEQ ID NO: 16; G179S, R190W where W is preferably identified with the corresponding codon in SEQ ID NO: 17; and R190Q, I204M where M is preferably identified with the corresponding codon in SEQ ID NO: 20; R231C and R231H where H is preferably identified with the corresponding codon in SEQ ID NO: 24; D242N, V254L where L is preferably identified with the corresponding codon in SEQ ID NO: 26; and V254M, H258N where N is preferably identified with the corresponding codon in SEQ ID NO: 27; and H258R where R is preferably identified with the corresponding codon in SEQ ID NO: 28; R259C, L262V where V is preferably identified with the corresponding codon in SEQ ID NO: 29; G269D and G269S, S277L, V280E where E is preferably identified with the corresponding codon in SEQ ID NO: 34; A300T, W305S and W305stop, G314D and G314S, Y315C, T322M where M is preferably identified with the corresponding codon in SEQ ID NO: 39; G325R, A341E and A341V, P343C where C is preferably identified with the corresponding codon in SEQ ID NO: 40; and P343R where R is preferably identified with the corresponding codon in SEQ ID NO: 41; A344E, R360T where T is preferably identified with the corresponding codon in SEQ ID NO: 46; R518G where G is preferably identified with the corresponding codon in SEQ ID NO: 55; and R518P where P is preferably identified with the corresponding codon in SEQ ID NO: 56; and R518stop, R539W, I567T where T is preferably identified with the corresponding codon in SEQ ID NO: 59; R591H, R594Q and, according to nucleotide numbering, with the mutations: 1514 +1G>A, (SEQ ID NO: 52), 1513-1514delCA corresponding to SEQ ID NO: 53, with the mutation 921+1 G>A and with the mutation 921+2 T>C;

- in the KCNH2 gene, according to the numbering of codons or amino acids: Y43C where C is preferably identified with the corresponding codon in SEQ ID NO: 67; E58A where A is preferably identified with the corresponding codon in SEQ ID NO: 69; and E58G where G is preferably identified with the corresponding codon in SEQ ID NO: 70; and E58D where D is preferably identified with the corresponding codon in SEQ ID NO: 71; and E58K, deI82-84IAQ preferably identified with SEQ ID NO: 75; W412stop, S428L where L is preferably identified with the corresponding codon in SEQ ID NO: 88; R534C and R534L where L is preferably identified with the corresponding codon in SEQ ID NO: 91; L552S, A561T and A561V, G572C and G572D where D is preferably identified with the corresponding codon in SEQ ID NO: 97; R582C and R582L where L is preferably identified with the corresponding codon in SEQ ID NO: 98; G604S, D609H where H is preferably identified with the corresponding codon in SEQ ID NO: 99; and D609G, T613M, A614V, T623I, G628S, S660L where L is preferably identified with the corresponding codon in SEQ ID NO: 106; R752W, S818L, R823W and, according to nucleotide numbering, with the mutations: 453deIC, 453-454insCC, 576deIG (SEQ ID NO: 79), 578-582deICCGTG (SEQ ID NO: 80), G2398+3A>G (SEQ ID NO: 110), G2398+3A>T (SEQ ID NO: 111), 3093-3106deI (SEQ ID NO: 125), 3093-3099del/insTTCGC (SEQ ID NO: 126), and 3100deIC (SEQ ID NO: 128);
- in the SCN5A gene: A413E where E is preferably identified with the corresponding codon in SEQ ID NO: 133; and A413T where T is preferably identified with the corresponding codon in SEQ ID NO: 134; T1304M, P1332L, 1505-1507deIKPQ, R1623Q, R1644C where C is preferably identified with the corresponding codon in SEQ ID NO: 140; Y1767C where C is preferably identified with the corresponding codon in SEQ ID NO: 142, E1784K.

According to a different embodiment, the kit comprises a set of at least 20 mutations among those defined in Table 2 and optionally other reagents such as buffers, enzymes, etc. necessary to carry out the method of the invention.

**Table 1. New mutations identified in probands.**

| **Gene** | | | **Nucleotide** | **Coding Effect (by aa* number)** | **Region** | **Type of mutation** |
|---|---|---|---|---|---|---|
| Oligo IDN | **KCNQ1** | | | | | |
| 11 | C CCG ACG GG | | G136A | A46T | N-TERM | Missense |
| 12 | CGCTCTTAC | | 151-152insT | L50fs+233x | N-TERM | Insertion |
| 13 | C TGC TTC AT | | C409T | L137F | S1 | Missense |
| 14 | C ATC AAG CA | | G436A | E146K | S1-S2 | Missense |
| 15 | GTG GAC CGC | | T518A | V173D | S2-S3 | Missense |
| 16 | GTC CCC CTC | | G521C | R174P | S2-S3 | Missense |
| 17 | G GGG TGG CT | | C568T | R190W | S2-S3 | Missense |
| 18 | CTG CCC TTT | | G575C | R192P | S2-S3 | Missense |
| 19 | GCCCGAAGC | | 584del | R195fs+41X | S2-S3 | Deletion |
| 20 | C ATC CGTGA | | G604C | D202H | S3 | Missense |
| 21 | TC ATG GTG G | | C612G | I204M | S3 | Missense |
| 22 | GCC TTC ATG | | C626T | S209F | S3 | Missense |
| 23 | C TGC ATG GG | | G643A | V215M | S3 | Missense |
| 24 | ATC CAC TTC | | G692A | R231H | S4 | Missense |
| 25 | ATG CCA CAC | | T716C | L239P | S4 | Missense |
| 26 | C TCC TTG GT | | G760T | V254L | S4-S5 | Missense |
| 27 | C ATC AAC CG | | C772A | H258N | S4-S5 | Missense |
| 28 | ATC CGC CGC | | A773G | H258R | S4-S5 | Missense |
| 29 | AG GAG GTG AT | | C784G | L262V | S4-S5 | Missense |
| 30 | CACCTGTAC | | 796del | T265fs+22X | S5 | Deletion |
| 31 | CTG GAC CTC | | G815A | G272D | S5 | Missense |
| 32 | TCTCGTACT | | 828-830del | S277del | S5 | Deletion |
| 33 | TCC TGG TAC | | C830G | S277W | S5 | Missense |
| 34 | TTT GAG TAC | | T839A | V280E | S5 | Missense |
| 35 | GAC GAG GTG | | C860A | A287E | S5-PORE | Missense |
| 36 | A GAT ACG CT | | G904A | A302T | PORE | Missense |
| 37 | CAG GAC ACA | | T923A | V308D | PORE | Missense |
| 38 | TAT GAG GAC | | G947A | G316E | PORE | Missense |
| 39 | CAG ATG TGG | | C965T | T322M | PORE-S6 | Missense |
| 40 | CTC CTA GCG | | C1028T | P343L | S6 | Missense |
| 41 | CTC CGA GCG | | C1028G | P343R | S6 | Missense |
| 42 | T GGC CCG GG | | T1045C | S349P | S6 | Missense |
| 43 | C TCG CGG TT | | G1048C | G350R | S6 | Missense |
| 44 | GGG TCT GCC | | T1052C | F351 S | S6 | Missense |
| 45 | GTGCAGCAG | | 1067-1072del | QT 356-357del | C-TERM | Deletion |
| 46 | CAG ACG CAG | | G1079C | R360T | C-TERM | Missense |
| 47 | GCA GAC TCA | | C1115A | A372D | C-TERM | Missense |
| 48 | TGG ATG ATC | | A1178T | K393M | C-TERM | Missense |
| 49 | GGGGGTGAC | | 1291-1292insG | G430fs+31x | C-TERM | Missense |
| 50 | GGGTGGACT | | 1292-1293insG | V431fs+31X | C-TERM | Insertion |
| 51 | AGACTGCTG | | 1486-1487 del | T495fs+18X | C-TERM | Deletion |
| 52 | CACAATGAG | | 1514+1 G>A | S504sp | C-TERM | Splice Error |
| 53 | CTCAGTGAG | | 1513-1514del | S504fs+9X | C-TERM | Deletion |
| 54 | GGCCACATT | | 1538delC | T513fs+78X | C-TERM | Deletion |
| 55 | C ATT GGA CG | | C1552G | R518G | C-TERM | Missense |
| 56 | ATT CCA CGC | | G1553C | R518P | C-TERM | Missense |
| 57 | CAG GAC CAC | | G1643A | G548D | C-TERM | Missense |
| 58 | ATG GCG CGC | | T1661C | V554A | C-TERM | Missense |
| 59 | TCC ACT GGG | | T1700C | I567T | C-TERM | Missense |
| 60 | AAGCCTCAC | | 1710delC | P570fs+22X | C-TERM | Deletion |
| 61 | TG TTA ATC T | | C1719A | F573L | C-TERM | Missense |
| 62 | ATCTCTCAG | | 1725-1728del | S575fs+16X | C-TERM | Missense |
| 63 | GAT CAC GGC | | G1748A | R583H | C-TERM | Insertion |
| 64 | C AGC GAC AC | | A1756G | N586D | C-TERM | Missense |
| 65 | CCCCCAGAG | | 1893delC | P631fs+33X | C-TERM | Deletion |
| 66 | GGGCCACAT | | 1909delC | H637fs+29X | C-TERM | Deletion |
| | | **KCNH2** | | | | |
| 67 | ATC TGC TGC | | A128G | Y43C | N-TERM | Missense |
| 68 | TTC TGC GAG | | G146A | C49Y | N-TERM | Missense |
| 69 | GCC GCG GTG | | A173C | E58A | N-TERM | Missense |
| 70 | GCC GGG GTG | | A173G | E58G | N-TERM | Missense |
| 71 | CC GAC GTG A | | G174C | E58D | N-TERM | Missense |
| 72 | C GAC CTC CT | | T202C | F68L | N-TERM | Missense |
| 73 | G CAC GGG CCG | | G211C | G71R | N-TERM | Missense |
| 74 | CGC ACG CAG | | C221T | T74M | N-TERM | Missense |
| 75 | GCAGGCAC | | 244-252del | IAQ82-84del | N-TERM | Deletion |
| 76 | GATGATGGT | | 308-310ins ATG | 103InsD | N-TERM | Insertion |
| 77 | TGTGCCCGT | | 337-339del | V113del | N-TERM | Deletion |
| 78 | CGGTTCGCCG | | 566del/Ins+TTCGC | A185fs+143X | N-TERM | Deletion/Insertion |
| 79 | CCGGGGCC | | 576delG | G192fs+7X | N-TERM | Deletion |
| 80 | GGGGGTGGT | | 578-582del | G192fs+135X | N-TERM | Deletion |
| 81 | GCCCCCGGC | | 735-6lnsCC | P245fs+114X | N-TERM | Insertion |
| 82 | ATCGTCCCG | | C751T | P251S | N-TERM | Missense |
| 83 | G CTG TAG G | | C1171T | Q391X | N-TERM | Nonsense |
| 84 | CGTGTCG GAC | | G1229C | W410S | S1 | Missense |
| 85 | G GAC TGA CTC | | G1235A | W412X | S1 | Nonsense |
| 86 | C ACA CAC TAC | | C1277A | P426H | S1-S2 | Missense |
| 87 | A CCC CAC TCG | | T1279C | Y427H | S1-S2 | Missense |
| 88 | C TAC TTG GCT | | C1283T | S428L | S1-S2 | Missense |
| 89 | C GTG TAC ATC | | G1378T | D460Y | S2 | Missense |
| 90 | C ATC CAC ATG | | G1501C | D501H | S3 | Missense |
| 91 | G GTG CTC GTG | | G1601T | R534L | S4 | Missense |
| 92 | CGGATCGCT | | 1613-1619del | R537fs+24X | S4 | Deletion |
| 93 | GCC TCC ATC | | G1697C | C566S | S5 | Missense |
| 94 | TGCATTGGT | | 1701delC | 1567fs+26X | S5 | Deletion |
| 95 | C ATC CGG TAC | | T1702C | W568R | S5 | Missense |
| 96 | C GCC GTC GC | | A1711 G | 1571V | S5 | Missense |
| 97 | C ATC GAC AA | | G1715A | G572D | S5 | Missense |
| 98 | TCACTCATC | | G1745T | R582L | S5-PORE | Missense |
| 99 | AAG CAC AAG | | G1825C | D609H | S5-PORE | Missense |
| 100 | GCG TTC TAC | | C1843T | L615F | PORE | Missense |
| 101 | GC AGG CTC A | | C1863G | S621 R | PORE | Missense |
| 102 | CCC GCCAGC | | G1877C | G626A | PORE | Missense |
| 103 | TCA GAC AAG | | G1911C | E637D | PORE-S6 | Missense |
| 104 | C TGC TTC AT | | G1930T | V644F | S6 | Missense |
| 105 | ATC TGC GGC | | T1967G | F656C | S6 | Missense |
| 106 | TG TTG GCC A | | C1979T | S660L | S6 | Missense |
| 107 | CAG CCC CTC | | G2087C | R696P | S6-CNBD | Missense |
| 108 | TGACGAGTG | | 2164-2181dup | E722-D727 | S6-CNBD | Duplication |
| 109 | CTTCCAGGG | | 2231deIG | F743fs+12X | S6-CNBD | Deletion |
| 110 | GGGTGTGGG | | G2398+3A>G | L799sp | CNBD | Splice Error |
| 111 | GGGTTTGGG | | G2398+3A>T | L799sp | CNBD | Splice Error |
| 112 | CCTGTGTAT | | G2398T | G800W | CNBD | Missense |
| 113 | AAGCCGAAC | | T2452C | S818P | CNBD | Missense |
| 114 | T GGC TAG TC | | A2494T | K832X | CNBD | Nonsense |
| 115 | TTC CAC CTG | | A2581C | N861H | C-TERI1II | Missense |
| 116 | GGGTGCAAC | | 2638-2648del | G879fs+35X | C-TERM | Deletion |
| 117 | TCCGACGGA | | 2676-2682del | R892fs+79X | C-TERM | Deletion |
| 118 | CCGGCCGGG | | 2732-2766deal | P910+16X | C-TERM | Deletion |
| 119 | GGCGGGCCG | | 2738-2739insCGGGC | A913fs+62X | C-TERM | Insertion |
| 120 | GGGGCCGTG | | 2775delG | G925fs+47X | C-TERM | Deletion |
| 121 | CG TGA GGG G | | G2781A | W927X | C-TERM | Nonsense |
| 122 | CCCGGGTGG | | 2895-2905del | G965fs+148X | C-TERM | Deletion |
| 123 | CCG GTG GGT | | C2903T | P968L | C-TERM | Missense |
| 124 | CGATGACCCGC | | C3045A | C1015X | C-TERM | Nonsense |
| 125 | CCCGGGGCG | | 3093-31 06del | G1031fs+86X | C-TERM | Deletion |
| 126 | GGGTTCGCC | | 3093- 3099del/insTTCGC | G1031fs+20X | C-TERM | Deletion/Insertion |
| 127 | GGCGCCCCG | | 3099delG | R1033+22X | C-TERM | Missense |
| 128 | GCGGCCCGG | | 3100delC | P1034 fs+63X | C-TERM | Deletion |
| 129 | CAGGTGGAG | | 3154delC | R1051fs+4X | C-TERM | Deletion |
| 130 | CCCCACCCT | | 3304lnsC | P1101fs+16X | C-TERM | Insertion |
| 131 | CCCACGACG | | 3397-3398del | T1133fs+135X | C-TERM | Deletion |
| | | **SCN5A** | | | | |
| 132 | CTG TAC AGA | | C3457T | H1153Y | C-TERM | Missense |
| 133 | GGTCGAA ATG | | C1238A | A413E | IS6 | Missense |
| 134 | GGTCACAAT | | G1237A | A413T | IS6 | Missense |
| 135 | TGCC GAG GG | | C1717G | Q573E | I-II | Missense |
| 136 | TCCC AGA AC | | G1735A | G579R | I-II | Missense |
| 137 | AAC CAT CTC | | G2066A | R689H | I-II | Missense |
| 138 | T GCC ACG AAG | | T4493C | M1498T | III-IV | Missense |
| 139 | TC TTC CCA GTC | | G4877C | R1626P | IV-S4 | Missense |
| 140 | GATC TGC ACG | | C4930T | R1644C | IV-S4 | Missense |
| 141 | C AAC GTC GTG | | A4978G | 11660V | IV-S5 | Missense |
| 142 | ATG TGC ATT | | A5300G | Y1767C | IV-S6 | Missense |
| 143 | CACC AAG CC | | G5360A | S1787N | C-TERM | Missense |
| 144 | GAG GGC GAC | | A5369G | D1790G | C-TERM | Missense |
| 145 | TTC CAC AGG | | G5738A | R1913H | C-TERM | Missense |
| | | **KCNE1** | | | | |
| 146 | CCC CAC AGC | | G107A | R36H F53C | S1 | Missense |
| 147 | GGA TGC TTC | | T158G | | S1 | Missense |
| | | **KCNE2** | | | | |
| 148 | GTGGTG ATG | | A166G+169InsATG | M56V+ M57ins | S1 | Missense/Insertion |
| 149 | CTGTAGGTG | | 156-161del | 52-s4del.YLM>X | S1 | Deletion |

**Table 2A. Mutations found in at least 40% of probands.**

| **MISSENSE MUTATIONS AND IN-FRAME INSERTIONS OR DELETIONS NUMBERING REFERS TO THE AMINO ACID SEQUENCE** | | |
|---|---|---|
| **KCNQ1** | **KCNH2** | **SCN5A** |
| L137 → (Z-L) F (SEQ ID NO: 13) | Y93 → (Z -Y) C (SEQ ID NO: 67) | A413 → (Z -A) E (SEQ ID NO: 133),T (SEQ ID NO: 134) |
| R174 → (Z-R) C¹, P (SEQ ID NO: 16) | E58 - (Z -E) A, G, D (SEQ ID NO: 69, 70, 71) , K² | T1304 → (Z -T) M³ |
| G179 → (Z-G) S⁴ | del 82-84 : IAQ (SEQ ID NO: 75) | P1332 V (Z -P) L⁵ |
| R190 → (Z-R) W (SEQ ID NO: 17), Q¹ | W412 → (Z -W); X (SEQ ID NO: 85) | del 1505-1507 : KPQ⁶ |
| I209 → (Z -I) M (SEQ ID NO: 21) | S428 → (Z -S) L (SEQ ID NO: 88), X⁷ | R1623 → (Z -R) Q⁸ |
| R231 → (Z -R) C⁹, H (SEQ ID NO:24) | R534 → (Z -R) C¹⁰ | R1644 → (Z -R) C (SEQ ID NO:140) ,H⁴ |
| D242 → (Z -D) N¹⁰ | L552 → (Z -L) S⁴ | Y1767 → (Z -Y) C (SEQ ID NO: 142) |
| V254 → (Z -V) L (SEQ ID NO: 26), M¹ | A561 → (Z -A) T⁴, V⁴ | E1784 → (Z -E) K⁴ |
| H258 → (Z-H) N(SEQ ID NO: 27), R(SEQ ID NO: 28) | G572 → (Z -G) C⁴, D (SEQ ID NO: 97) | |
| R259 → (Z-R) C¹¹ | R582 → (Z -R) C¹², L (SEQ ID NO: 98) | |
| L262 → (Z -L) V (SEQ ID NO: 29) | G604 → (Z-G) S⁴ | |
| G269 → (Z-G) D¹, S¹³ | D609 → (Z -D) H (SEQ ID NO: 99), G¹⁶ | |
| S277 → (Z-S) L¹⁴ | T613 → (Z -T) M⁴ | |
| V280 → (Z -V) E(SEQ ID NO: 34) | A614 → (Z -A) V⁷ | |
| A300 → (Z -A) T¹⁵ | T623 → (Z -T) I¹⁶ | |
| W305 → (Z -W) S¹⁷, X¹⁸ | G628 → (Z -G) S⁴ | |
| **KCNQ1** | **KCNH2** | **SCNSA** |
| G314 → (Z -G) D19, S¹ | S660 → (Z -S) L (SEQ ID NO: 106) | |
| Y315 → (Z -Y) C²⁰ | R752 → (Z -R) W⁴ | |
| T322 → (Z -T) M (SEQ ID NO: 39) | S818 → (Z -S) L²¹, P (SEQ ID NO: 113) | |
| G325 → (Z -G) R¹ | R823 → (Z -R) W⁴ | |
| A341 → (Z -A) E⁴, V⁴ | | |
| P343 → (Z -P) L, R (SEQ ID NO: 40, 41) | | |
| A344 → (Z -A) E¹⁶ | | |
| R360 → (Z -R) T (SEQ ID NO: 46) | | |
| R518 → (Z -R) G,P (SEQ ID NO: 55, 56), X⁴ | | |
| R539 → (Z -F) W²² | | |
| 1567 - (Z -I) T (SEQ ID NO: 59) | | |
| R591 - (Z -R) H²³ | | |
| R594 → (Z -R) Q⁴ | | |

| **TABLE 2B: SPLICING AND FRAMESHIFTS MUTATIONS (NUMBERING REFERS TO THE NUCLEOTIDE SEQUENCE)** | | |
|---|---|---|
| **KCNQ1** | **KCNH2** | |
| 1514+1 G>A (SEQ ID NO: 52) | 453delC²⁴; 453-454insCC¹⁶ | |
| 1513-1514delCA(SEQ ID NO: 53) | 576delG (SEQ ID NO: 79) | |
| 921 + 1 G>A⁴ | 578-582del CCGTG (SEQ ID NO: 80) | |
| 921+2 T>C⁴ | G2398+3A>G (SEQ ID NO: 110) | |
| | G2398+3A>T (SEQ ID NO: 111) | |
| | 3093-3106del (SEQ ID NO: 125) | |
| | 3093-3099del/insTTCGC (SEQ ID NO: 126) | |
| | 3100delC (SEQ ID NO: 128) | |

Legend to tables 1 and 2.

### Table 1.

Mutation abbreviations:
del: deletion. 796del indicates that the nucleotide at position 796 is deleted. When more than one nucleotide is deleted, as for instance in 828-830 (SEQ ID NO: 32), all the intervening nucleotides and the extreme nucleotides are deleted (e.g. 828, 829, 830). The deleted nucleotide/nucleotides can be also specified (e.g. in SEQ ID NO: 129, 3154delC indicates that the cytosine at position 3154 is deleted).
ins: insertion. The explanation is similar to del.

Generally, the nucleotide or amino acid number refers to the residue (nucleotide or amino acid) affected by the mutation. However, for frame shift mutations (fs), the amino acid residue indicated with the one-letter code and the number corresponding to its position in the protein sequence, corresponds to the last residue identical to *wild type.*

Moreover, for frame-shift mutations, that easily generate a stop codon upstream of the natural stop codon, it is also indicated the number of amino acids out of frame (different from the natural protein sequence) that follow the last *wild type* amino acid (for instance, R195fs+41X, SEQ ID NO: 19, indicates that the nucleotide mutation generates a frame-shift due to which Arg at position 195 of the KCNQ1 gene is the last *wild type* amino acid, followed by a tail of 41 amino acids different from *wild type*).

Coding for splicing (sp) errors: the number indicates the last nucleotide of the exon; the number after the + sign indicates the position, relative to this nucleotide, of the intronic base that is substituted. For instance, 921+2 T>C indicates a T to C substitution of the second intronic base after the last exonic nucleotide at position 921.

### Table 2A

Z: any amino acid; (Z-W): any amino acid but W. Therefore, the detection of the mutation refers to the codon identified by the number: Detection may refer to any codon different from *wild type* (e.g.. for KCNQ1, the detection of R518 refers to any codon not encoding the *wild type* amino acid isoleucine, and preferably refers to any codon encoding glycine or proline, even more preferably refers to glycine and proline codons as identified by SEQ ID NO: 55 and SEQ ID NO: 56).

### Table 2A and 2B:

X.: STOP codon. Other abbreviations and symbols have the same meaning as in Table 1.

Numbering refers to the number of the *wild type* amino acid (Table 2A) or nucleotide (Table 2B) as reported in the Sequence List.

Superscript numbers refer to references for mutations already described. The mutations identified in the present invention are characterized by the corresponding identification n° (SEQ ID NO) of Table 1.

Additional references for the symbols used are reported in: Antonarakis SE. Recommendations for a nomenclature system for human gene mutations. Nomenclature Working group. Hum. Mutat., 1998; 11:1-3.

References for Table 2:
1. Donger,C, et al. Circulation. 1997; 96:2778-2781.
2. Lupoglazoff,JM, et al. Circulation. 2001; 103:1095-1101.
3. Wattanasirichaigoon, D, et al.. Am J Med Genet. 1999; 86:470-476.
4. Splawski,l, et al.. Circulation. 2000; 102:1178-1185.
5. Kehl,HG, et al. Circulation. 2004; 109:e205-e206.
6. Wang,Q, et al.. Cell. 1995; 80 :805-811.
7. Priori,SG, et al. Circulation. 1999; 99:529-533.
8. Kambouris,NG, et al. Circulation. 1998; 97:640-644.
9. Lupoglazoff,JM et al. J Am Coll Cardiol. 2004; 43:826-830.
10. ltoh,T, et al. Hum.Genet. 1998; 102:435-439.
11. Kubota,T, et al. J Cardiovasc Electrophysiol. 2000; 11:1048-1054.
12. Jongbloed,RJ, et al. Hum.Mutat. 1999; 13:301-310.
13. Ackerman,MJ, et al N.Engl.J Med. 1999; 341:1121-1125.
14. Liu,W, et al. Hum.Mutat. 2002; 20:475-476.
15. Priori,SG, et al. Circulation. 1998; 97:2420-2425.
16. Tester,DJ, et al.. Heart Rhythm. 2005; 2:507-517.
17. Neyroud,N, et al. Eur.J.Hum.Genet. 1998; 6:129-133.
18. Chen,S et al. Clin Genet. 2003; 63:273-282.
19. Choi,G, et al. Circulation. 2004; 110:2119-2124.
20. Napolitano,C et al. J.Cardiovasc.Electrophysiol. 2000; 11:691-696.
21. Berthet,M, et al. Circulation. 1999; 99:1464-1470.
22. Chouabe,C, et al. Cardiovasc Res. 2000; 45:971-980.
23. Neyroud,N, et al. Circ.Res. 1999; 84:290-297.
24. Swan,H, et al J Am.Coll.Cardiol. 1999; 34:823-829.

**Table 3**

| | N | QTc (ms) | Mean | IQR | % penetrance |
|---|---|---|---|---|---|
| LQT1 | 450 | 465±41 | 461 | 440-488 | 64** |
| LQT2 | 279 | 486±48* | 477 | 455-511 | 81# |
| LQT3 | 63 | 489±49* | 481 | 460-515 | 83# |
| LQT5 | 20 | 447±36 | 439 | 424-467 | 40 |
| LQT6 | 5 | 434±24 | 425 | 414-459 | 40 |

| | | | | | |
|---|---|---|---|---|---|
| QT values in ms. IQR = Inter Quartile Range in ms; *p<0.005 vs. *KCNQ1*/*KCNE1;* ** p<0.04 vs. *KCNE1;* # p<0.001 vs. *KCNQ1*/*KCNE1*/*KCNE2* | | | | | |

**Table 4. Sequencing primers (SEQ ID NO: 150-212)**

| GENE KCNQ1 | PRIMER ID | SEQUENCE | Length |
|---|---|---|---|
| | KV11.1 | cactcaaggccgagcctgcct | 21 |
| " | KVSNA | gccccacaccatctccttcg | 20 |
| " | KV6NI | taccctaacccgggccac | 18 |
| " | KVDFn | gaggagaagtgatgcgtgtc | 20 |
| " | KVDRn | ggcaggacctgggcaccctc | 20 |
| " | KV1A1F | cttcgctgcagctcccggtg | 20 |
| " | KV1A2R | acgcgcgggtctaggctcac | 20 |
| " | KK2F | gactgccgtgtccctgtcttg | 21 |
| " | KK2R | gccatgccttca6atgctacg | 21 |
| " | KK72Rn | ctgagggcaggaaggctcag | 20 |
| " | KK14F1 | ctgtctgtcccacagacgac | 20 |
| " | KK14Rn | ctgggcccagagtaactgac | 20 |
| " | KK15Fn | cggcccaccccagcacttggc | 21 |
| " | KK15Rn | gaaccaccgcaggccggcgcg | 21 |
| " | KK16Fn | cgtctgcctttgtccccg | 18 |
| " | KK16Rn | cactcttggcctcccctc | 18 |
| KCNE1 | MINK F | ctgcagcagtggaccctta | 19 |
| " | MINK 1R | agcttcttggagcggatgta | 20 |
| " | MINK 2F | gtcctcatggtactgggatt | 20 |
| " | MINK R | tttagccagtggtggggtt | 19 |
| KCNE2 | MINK2-F2 | ccgttttcctaaccttgttcgcct | 24 |
| " | MINK2-R2 | gccacgatgatgaaagagaacattcc | 26 |
| " | MINK2-F3 | gtcatcctgtacctcatggtgat | 23 |
| " | MINK2-R3 | tggacgtcagatgttagcttggtg | 24 |
| SCN5A | SCN2.1 Fnew | ccc tgc tct ctg tcc ctg ggc | 21 |
| " | SCN2.1 Rnew | gca gcc cct ctc ggc tct cc | 20 |
| " | SCN2.2Fnew | cat ggc aga gaa gca agc ccg | 21 |
| " | SCN6Fnew | cct cct ctg act gtg tgt ctc c | 22 |
| " | SCN10Fnew | cca gtg agg gtg acc tct gcc | 21 |
| " | SCN10Rnew | ggc tta gag gct cct cgg tgg | 21 |
| " | SCN16F | gag cca gag acc ttc aca agg | 21 |
| " | SCN17.1Fnew | gct tgg cat ggt gca gtg cct tgg | 24 |
| " | SCN17.1Rnew | gag gca cct tct ccg tct ctg g | 22 |
| SCNSA | SCN20Fnew | cat tag atg tgg gca ttc aca ggc | 24 |
| " | SCN20Rnew | cca gcc gtc cct gcc aca acc | 21 |
| " | SCN21Fnew | ggt cca ggc ttc atg tcc acc | 21 |
| " | SCN21Rnew | ggc aat ggg ttt ctc ctt cct g | 22 |
| " | SCN22Fnew | ggg gag ctg ttc cca tcc tcc c | 22 |
| " | SCN22Rnew | cgc ctc cca ctc cct ggt ggg | 21 |
| " | SCN23.1F | ttg aaa agg aaa tgt gct ctg gg | 23 |
| " | SCN23.1R | ttg ttc acg atg gtg tag ttc a | 22 |
| " | SCN23.2F | cca gac aga ggg aga ctt gcc | 21 |
| " | SCN25Fnew | ccc agc ctg tct gat ctc cct g | 22 |
| " | SCN25Rnew | cca ccc tac cca gcc cag tgg | 21 |
| KCNH2 | HM1F | catgggctcaggatgccggt | 20 |
| " | KCNH2-1R | cattgactcgcacttgccgacg | 22 |
| " | H2F | cgctcacgcgcactctcctc | 20 |
| " | KH2R | ttgaccccgcccctggtcgt | 20 |
| " | H3F | ccactgagtgggtgccaaggg | 21 |
| " | H3R | gagaccacgaacccctgagcc | 21 |
| " | H4.1 F | cccacgaccacgtgcctctcc | 21 |
| " | H8R | gcctgccacccactggcc | 18 |
| " | KH9F | atggtggagtagagtgtgggtt | 22 |
| " | KH9R | agaaggctcgcacctcttgag | 21 |
| " | KH10F | gagaaggtgcctgctgcctgg | 21 |
| " | KH10R | acagctggaagcaggaggatg | 21 |
| " | H11F | ggcaggagagcactgaaagggc | 22 |
| " | H11R | ggtaaagcagacacggcccacc | 22 |
| " | H12A F | gttctcctcccctctctgaggc | 22 |
| " | H12B R | gggtagacgcaccaccgctgc | 21 |
| " | H13F | gcagcggtggtgcgtctaccc | 21 |
| GENE | PRIMER ID | SEQUENCE | Length |
| " | H13R | gacctggaccagactccagggc | 22 |
| " | H14R | gggtacatcgaggaagcagg | 20 |

### EXPERIMENTAL PART

### Methods

Probands and their relatives were subjected to genetic analysis by molecular screening of the coding regions of genes associated with LQTS (Romano Ward variant). The diagnosis of probands was based on conventional clinical criteria (personal clinical history, evaluation of the QT interval by standard 12-lead ECG,

Holter recording, with a cycloergometer exercise test ).

Relatives were evaluated by genetic analysis independently from the diagnosis based on the clinical phenotype.

**Methods for sample analysis:** the entire coding regions of KCNQ1, KCNH2, SCN5A, KCNE1 and KCNE2 genes were examined in a sample of 1621 subjects belonging to 430 families, using primers designed from intronic regions close to splicing sites (Table 4).

The population study comprised 430 LQTS probands with RWS and 1115 members of their families, and their data were collected by the Molecular Cardiology Laboratories of the Maugeri Foundation.

An informed consent was obtained from all subjects undergoing molecular analysis and, in the case of minors, from their legal tutors.

A group of 75 genotyped probands were used to verify the non-familial mutations identified in the previously examined population.

The study was approved by the Institutional Review Board of the Maugeri Foundation according to IRB regulations for all study subjects.

### Statistical Analysis

Statistical analyses, when performed, were carried out with the SPSS Statistical Package (v. 12.01). The Kolmogorov-Smirnov test was used to determine the normal distribution of variables. Parametric tests (unpaired t-test and ANOVA with Bonferroni multiple-comparison correction) were used to compare normally distributed variables; instead the Kruskal-Wallis and the Mann-Whitney tests were used for not normally distributed variables. Chi-square test or Fisher exact test has been used for categorical variables. The data obtained are the mean ± standard deviation (SD). For data that do not follow a normal distribution, the median and the interquartile range (25% and 75%) are reported.

### Genotyping

Molecular analysis was performed on genomic DNA extracted from peripheral lymphocytes using methods well known in the art. The entire "open reading frames" of KCNQ1, KCNH2, SCNSA, KCNE1 and KCE2 genes were amplified by PCR with primers designed from intronic sequences flanking the exons ("exon-flanking intronic primer"). Primers are well known in the art or listed in Table 4. Amplicons were analysed by Denaturing High Performance Liquid Chromatography (DHPLC, Wave® Transgenomics) Each amplicon was subjected to DHPLC at least at two different temperatures, depending on the "melting" profile of the amplicon. When an abnormal chromatogram was detected, the amplicon was sequenced by a 310 Automated Genetic Analyzer® (Applied Biosystems) and/or cloned by PCR in a plasmid vector (Topo® cloning, Invitrogen). All DNA sequence variations that were absent in 400 control subjects (corresponding to 800 chromosomes) were defined as mutations.

### Example 1. Clinical characterization of the sample.

ECGs and clinical data were collected and analyzed in blind relative to the genetic status of the samples. QTc (QTc = QT / vRR) data were measured using the ECG recorded during the first visit.

The demographic characteristics of the sample are shown in the following table.

**Table 5**

| | Probands | Family members | | |
|---|---|---|---|---|
| | | Genetically affected | Genetically not affected | P-value |
| N | 310 | 521 | 594 | |
| Males (%) | 147 (47) | 231 (44) | 281 (47) | |
| Age, years (mean) | 21±20(16) | 33±20 (35) | 29±19 | <0.002** |
| QTc * (mean) | 495±49 (490) | 461±40 (458) | 406±27 (408) | <0.001** |

| | | | | |
|---|---|---|---|---|
| *In ms. ** Post-hoc analysis between groups. *QTc duration and occurrence by genotype.* | | | | |

The QTc value in the various populations examined has been reported in Table 3. As can be deduced from the table, in the mutation carriers the QTc was 474±46 msec (median value: 467 msec, IQR: 444-495 msec) while, among healthy family members, it was 406±27 msec (median value: 409 msec, IQR 390-425 msec).

Incomplete penetrance was defined as the percentage of mutation carriers having a QTc longer than normal (e.g., QTc = 440 ms for males and = 460 ms for females).

The mean penetrance of the disease in the study population turned out to be 70%, but decreased to 57% among family members carrying the mutation. Patients with LQT2 and LQT3 showed a higher penetrance compared to patients with LQT1 and LQT5, whereas penetrance for patients with LQT6 could not be determined due to the low number (see Table 5).

QTc duration was decreasingly long for probands, family members carrying the disease at the genetic level and healthy family members (p<0.0001; Table 5).

However the distribution of QTc values was very similar between subjects affected by genetic mutations and subjects without mutations. (Instead, the QTc and the penetrance of LQTS were significantly different between carriers of multiple mutations (n=26) and family members with only one mutation (n=49): QTc 495±58msec, IQR 450-523 msec versus 434±31msec, IQR: 411-452 msec, p<0.001; 30%, 15/49 versus 77% 20/26; p<0.0001).

The analysis of 1411 subjects (296 probands, 521 genetically affected and 594 genetically unaffected family members) to determine the sensitivity and the specificity of different QTc cut-off intervals in individuals carrying genetic mutations, revealed that the best cut-off value for sensitivity and specificity is a QTc value = 440 ms (81% specificity, 89% sensitivity and 91% positive predictive accuracy for diagnosis). Specific cut-off values for gender (=440 ms for males and =460 ms for females) proved to be very specific (96%) but poorly sensitive (72%).

### Example 2. Genetic characterization of the sample.

Genetic analysis of the sample revealed that 310/430 (72%) of the probands and 521 family members were carriers of 235 different mutations (139 of which were novel) that can determine the LQTS Syndrome.

From these 310 probands, the genetic analysis was extended to a total of 1115 family members: a mutation was found in 521 of them, while 594 were found to be healthy. In total, the study revealed 831 carriers of a mutation predisposing to LQTS symptoms and 594 healthy family members.

The mutation rate in the different genes was distributed as follows: 49% KCNQ1; 39% KCNH2; 10% SCN5A; 1.7% KCNE1; 0.7% KCNE2. About 90% of genotyped patients had mutations in KCNQ1 and KCNH2 genes, while 44% of the probands carried common mutations. These statistics were verified again with an independent set of 75 genotyped probands (fig 1).

All the mutations identified and characterized for the first time in the present invention are reported in Table 1.

Two-hundred-ninety-six probands carried heterozygous mutations and 14 (4.5%) carried more than one genetic defect. Twelve probands turned out to be heterozygous for 2 (n=11) or 3 (n=1) combinations of mutations, 2 turned out to be homozygous RWS patients. In the group of 296 probands with a single genetic defect, KCNQ1 mutations were most represented (n=144; 49%), followed by KCNH2 mutations (n=115; 39%), SCN5A mutations (n=30; 10%), KCNE1 mutations (n=5; 1.7%) and at last KCNE2 mutations (n=2; 0.7%).

In conclusion, 98% of the genotypic mutations detected in LQTS probands were identified in KCNQ1, KCNH2 and SCN5A genes. Twenty-nine of 247 probands, whose parents were both available for genetic analysis, turned out to be carriers of sporadic mutations (12%).

Overall, 235 different mutations were identified, of which 139 (*KCNQ1* n= 56, *KCNH2* n=67, *SCNSA* n=13, *KCNE1* n=2, *KCNE2* n=2) were identified in this study for the first time. Missense mutations accounted for 72% (170/235) of the genetic defects. The remaining 28% comprised small intragenic deletions (n=33; 14.1%), splice errors (n=6; 2.7%), non-sense mutations (n= 12; 5.1%), insertions (n=11; 4.7%), duplications or insertions/deletions (n=3; 1.4%). The most frequently mutated codons (hot-spots) were: in *KCNQ1:* codon 190.(n= 12), 231 (n=4) 254 (n=4), 269 (n=4), 277 (n=5), 314 (n=4), 341 (n=6), 344 (n= 9) and codons 561 (n=7), 572 (n=4) and 628 (n=7) in KCNH2, 1332 (n=5) and 1784 (n=3) in SCNSA.

In total, 74/296 (25%) of the probands were genotyped based on *hot-spot* mutations and 129/296 of the probands (44%) carried one of the non-private mutations reported in Table 2 (i.e. mutations identified in more than one family).

### Intra-locus variability

The distribution of mutations in the protein coding regions of the various LQTS genes, using the subdivision in regions already reported in other studies (e.g. Splawski I. et al. Circulation, 2000 102:1178-1185), was the following: mutations in the "pore region" and in the transmembrane region were identified in 61% of the patients, while in 32% of the patients the mutation was in a C-terminal region; only 7% of the patients carried a N-terminal mutation.

### Annex 1

### cDNA Sequence List:

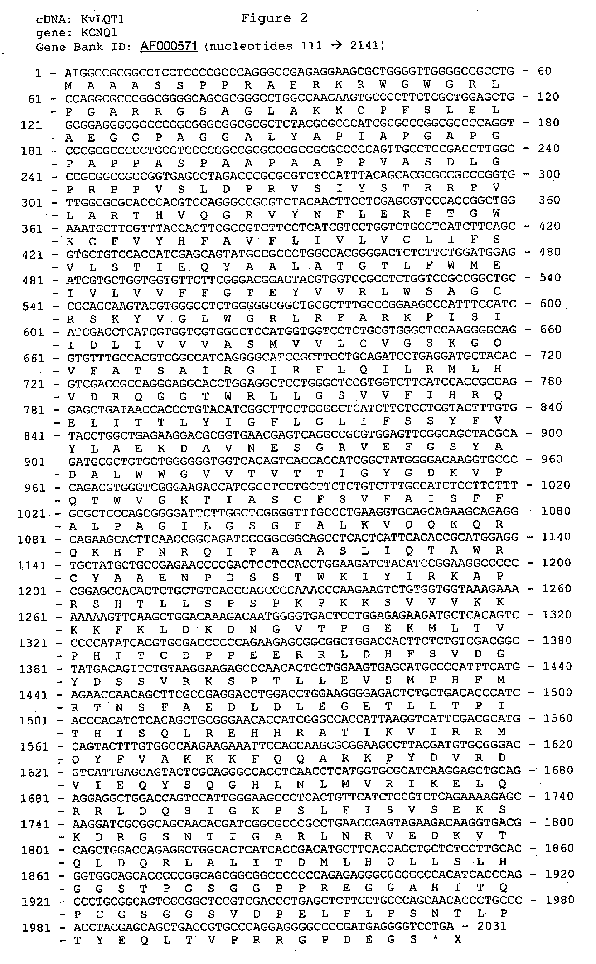

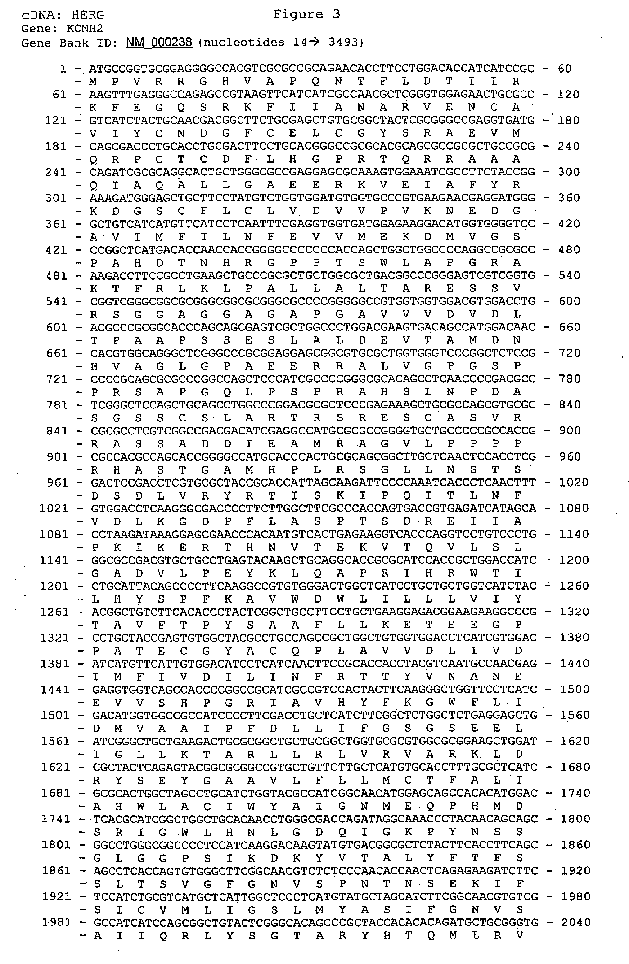

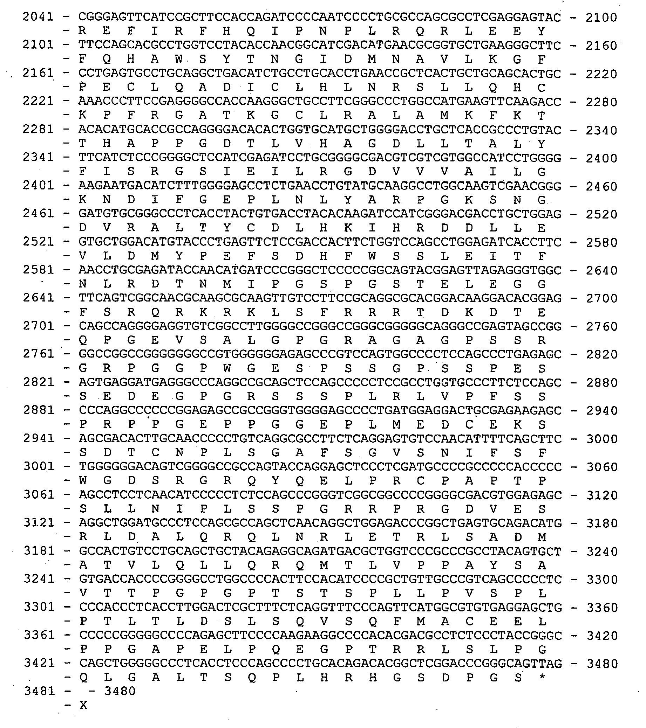

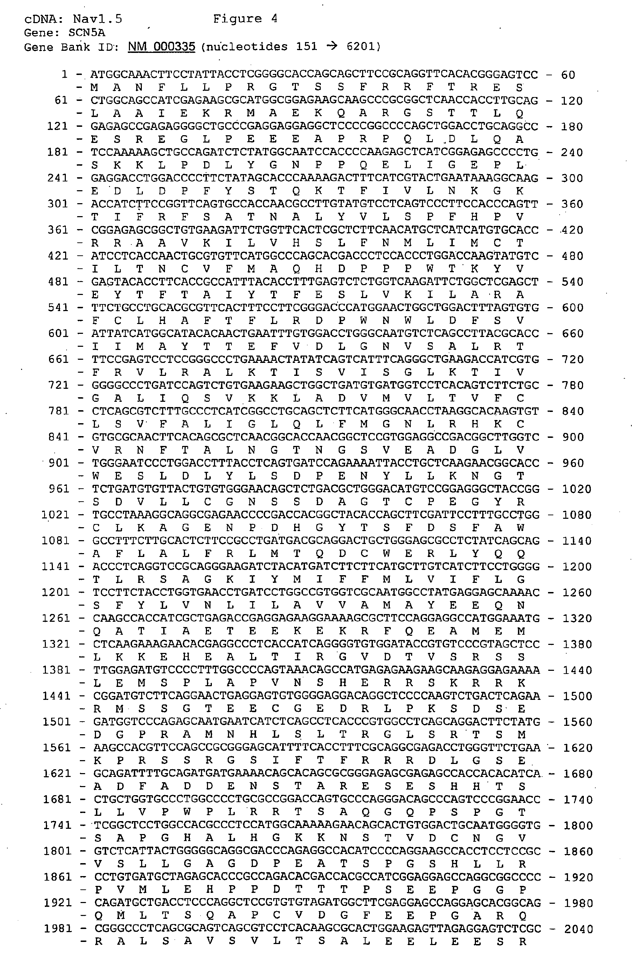

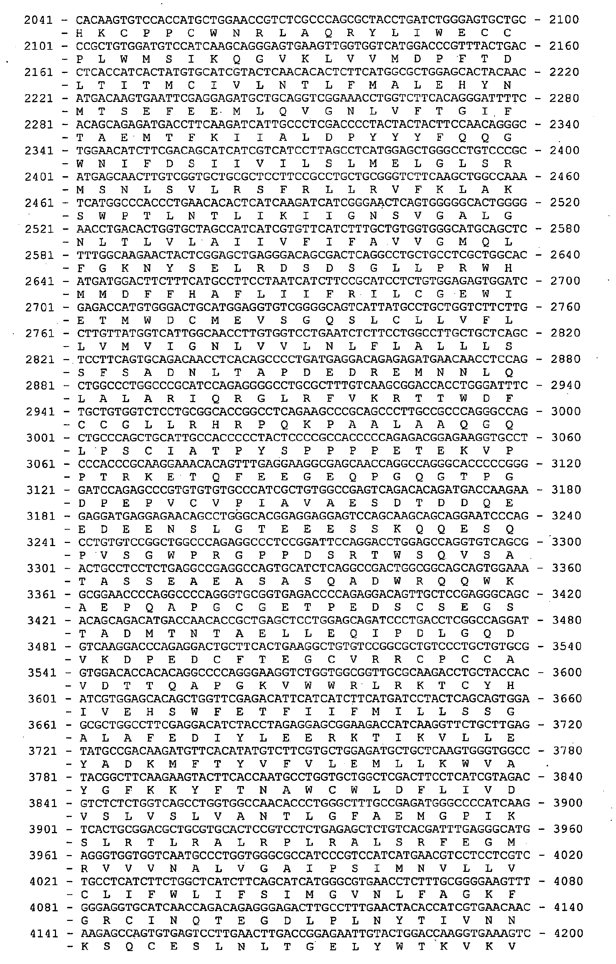

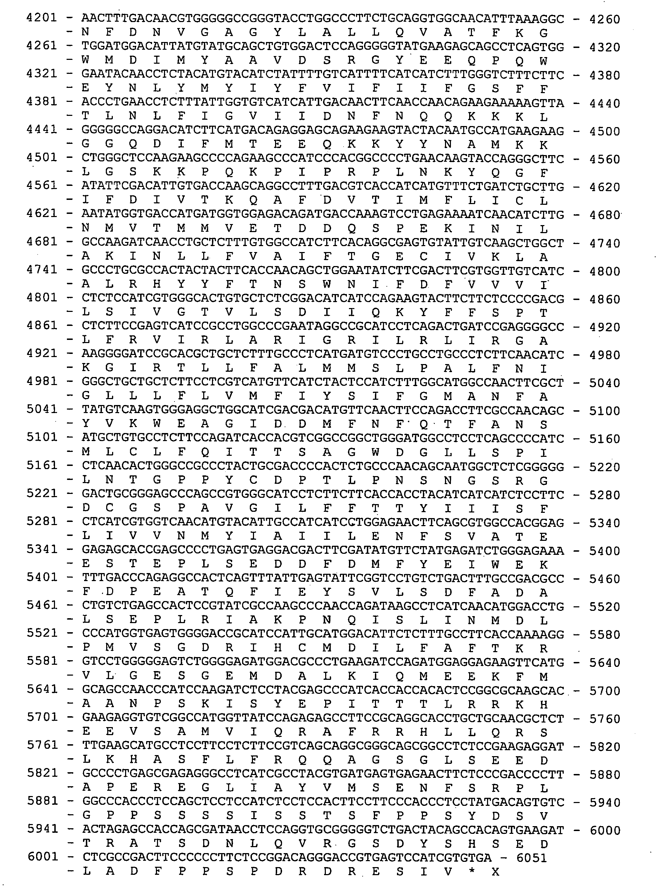

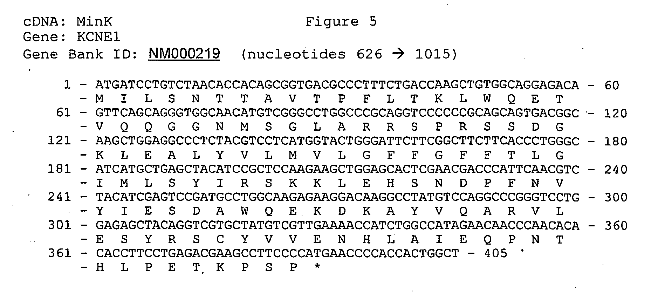

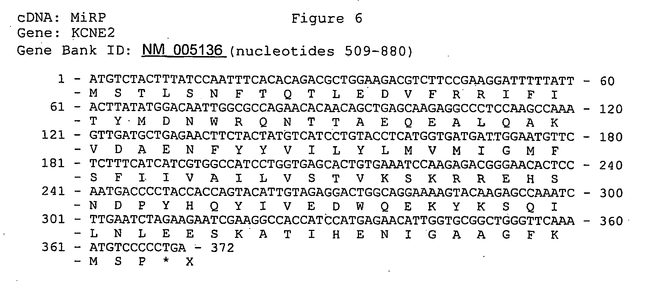

- SEQ ID NO: 1: KvLQT1 cDNA (GenBank Acc. N° AF000571); SEQ ID NO: 2: KvLQT1 protein (see fig. 2)
- SEQ ID NO: 3: KCNH2 cDNA (GenBank Acc. N° NM000238); SEQ ID NO: 4: KCNH2 protein (see fig. 3)
- SEQ ID NO: 5: SCNSA cDNA (GenBank Acc. N° NM000335); SEQ ID NO: 6: SCNSA protein (see fig. 4)
- SEQ ID NO: 7: KCNE1 cDNA (GenBank Acc. N° NM000219); SEQ ID NO: 8: KCNE1 protein (see fig.5)
- SEQ ID NO: 9 KCNE2 cDNA (GenBank Acc. N° NM000335); SEQ ID NO: 10: KCNE2 protein (see fig. 6).

The oligonucleotides of the invention, comprising the mutations identified and characterized in the present invention, are numbered from 11 to 149 and are reported in Table 1.

## Claims

1. Method for *in vitro* diagnosis of the predisposition to the Long QT Syndrome or for the diagnosis of the full-blown Long QT Syndrome, comprising the detection in a DNA sample of a group of non-private mutations in KVLQT1, KCNH2 and SCN5A genes, corresponding to the following amino acids or nucleotide positions:
- in the KCNQ1 gene, according to amino acid numbering: L137F, R174C and R174P, G179S, R190W and R190Q, I204M, R231C and R231H, D242N, V254L and V254M, H258N and H258R, R259C, L262V, G269D and G269S, S277L, V280E, A300T, W305S and W305stop, G314D and G314S, Y315C, T322M, G325R, A341E and A341V, P343L and P343R, A344E, R360T, R518G, R518P, R518stop, R539W, I567T, R591H, R594Q and,
- in the KCNQ1 gene, according to the nucleotide numbering, the mutations: 1514 +1G>A, (corresponding to the mutation of SEQ ID NO: 52), 1513-1514delCA corresponding to SEQ ID NO: 53, the mutation 921 +1 G>A and the mutation 921+2 T>C;
- in the KCNH2 gene, according to the amino acid numbering, the mutations: Y43C, E58A and E58G and E58D and E58K, deI82-84IAQ, W412stop, S428L, S428Stop, R534C and R534L, L552S, A561 T and A561 V, G572C and G572D, R582C and R582L, G604S, D609H and D609G, T613M, A614V, T623I, G628S, S660L, R752W, S818L and S818P, R823W and,
- in the KCNH2 gene, according to the nucleotide numbering, the mutations: 453delC, 453-454insCC, 576deIG (SEQ ID NO: 79), 578-582deICCGTG (SEQ ID NO: 80), G2398+3A>G (SEQ ID NO: 110), G2398+3A>T (SEQ ID NO: 111), 3093-3106del (SEQ ID NO: 125), 3093-3099del/insTTCGC (SEQ ID NO: 126), and 3100delC (SEQ ID NO: 128);
- in the SCN5A gene, according to the amino acid numbering, the mutations: A413E and A413T, T1304M, P1332L, 1505-1507delKPQ, R1623Q, R1644H and R1644C, Y1767C, E1784K
where the presence of at least one change in the sample from *wild type* correlates with the QT Syndrome or with the predisposition to said syndrome.

2. The method according to claim 1 wherein corresponding mutations are identified with oligonucleotides comprising the following nonanucleotides or complementary sequences thereof:
- KCNQ1: SEQ ID NO: 13 (L137F), SEQ ID NO: 16 (R174P), SEQ ID NO: 17 (R190W), SEQ ID NO: 21 (I204M), SEQ ID NO: 24 (R231H), SEQ ID NO: 26(V254L), SEQ ID NO: 27(H258N), SEQ ID NO: 28 (H258R), SEQ ID NO: 29 (L262V), SEQ ID NO: 34 (V280E), SEQ ID NO: 39 (T322M), SEQ ID NO: 40 (P343L), SEQ ID NO: 41 (P343R), SEQ ID NO: 46 (R360T), SEQ ID NO: 55 (R518G), SEQ ID NO: 56 (R518P), SEQ ID NO: 59 (1567T), SEQ ID NO: 52, SEQ ID NO: 53;
- KCNH2: SEQ ID NO: 67 (Y43C), SEQ ID NO: 69 (E58A), SEQ ID NO: 70 (E58G), SEQ ID NO: 71 (E58D), SEQ ID NO: 75 (del IAQ), SEQ ID NO: 85 (W412stop), SEQ ID NO: 88 (S428L), SEQ ID NO: 97 (G572D), SEQ ID NO: 98 (R852L), SEQ ID NO: 99 (D609H), SEQ ID NO: 106 (S660L), SEQ ID NO: 113 (S818P), SEQ ID NO: 79, SEQ ID NO: 80, SEQ ID NO: 110, SEQ ID NO: 111, SEQ ID NO: 113, SEQ ID NO: 125, SEQ ID NO: 126, SEQ ID NO: 128;
- SCN5A: SEQ ID NO: 133 (A413E), SEQ ID NO: 134 (A413T), SEQ ID NO: 140 (R1644C), SEQ ID NO: 142 (Y1767C).

3. The method according to claims 1 and 2 wherein said mutations are detected according to one of the following techniques:
- restriction pattern of a DNA fragment from the sample comprising the mutation, optionally in parallel with a sample corresponding to the *wild type* sequence,
- hybridization of nucleic acids of the sample with specific probes under selective conditions,
- PCR,
- Oligonucleotide Ligation Assay,
- electrophoresis showing the migration pattern of the nucleic acids of the sample,
- direct sequencing,
- Denaturing High Performance Liquid chromatography,
wherein, according to each of the above mentioned methods, the presence of a mutation in the sample is optionally further confirmed by comparison with a pattern obtained from control nucleic acids not carrying the mutation (or *wild type)* or by hybridization under selective conditions.

4. Method according to claim 1 further comprising the sequence characterization of the KCNQ1 and/or KCNH2 genes.

5. Method according to claim 4 further comprising the sequence characterization of the SCN5A, KCNE1 and/or KCNE2 genes.

6. Method according to claim 5 wherein the sequence of the said genes is **characterized by** direct sequencing with at least one of the oligonucleotide primers listed in Table 4.

7. The method according to claims 1-6 wherein said sample is genomic DNA.

8. The method according to claims 1-6 further comprising a step of reverse transcription of a RNA sample into cDNA.

9. The method according to claims 1-8 for diagnosis of the iatrogenic Long QT and wherein the presence of at least one of the mutations as defined in claim 1 indicates sensitivity to a drug selected in the group consisting of: antibiotics, prokinetic drugs, antipsychotic drugs, antidepressants, and antiarrhythmic drugs.

## Patentansprüche

1. Verfahren für eine *in vitro*-Diagnose der Prädisposition für das Long-QT-Syndrom oder für die Diagnose des voll aufgeblühten Long-QT-Syndrom, umfassend den Nachweis einer Gruppe von nicht-privaten Mutationen in den Genen KVLQT1, KCNH2 und SCN5A in einer DNS-Probe, entsprechend den folgenden Aminosäure- oder Nukleotid-Positionen:
- in dem Gen KCNQ1, entsprechend der Aminosäure-Nummerierung: L137F, R174C und R174P, G179S, R190W, und R190Q, I204M, R231C und R231H, D242N, V254L und V254M, H258N und H258R, R259C, L262V, G269D und G269S, S277L, V280E, A300T, W305S und W305stop, G314D und G314S, Y315C, T322M, G325R, A341E und A341V, P343L und P343R, A344E, R360T, R518G, R518P, R518stop, R539W, I567T, R591H, R594Q; und,
- in dem Gen KCNQ1, entsprechend der Nukleotid-Nummerierung, die Mutationen: 1514 + 1G>A (entsprechend der Mutation von Seq.-ID-Nr. 52), 1513-1514delCA entsprechend Seq.-ID-Nr. 53, die Mutation 921 + 1 G>A und die Mutation 921 + 2 T>C;
- in dem Gen KCNH2, entsprechend der Aminosäure-Nummerierung, die Mutationen: Y43C, E58A und E58G und E58D und E58K, del82-84IAQ, W412stop, S428L, S428Stop, R534C und R534L, L552S, A561T und A561V, G572C und G572D, R582C und R582L, G604S, D609H und D609G, T613M, A614V, T623I, G628S, S660L, R752W, S818L und S818P, R823W; und
- in dem Gen KCNH2, entsprechend der Nukleotid-Nummerierung, die Mutationen: 453delC, 453-454insCC, 576delG (Seq.-ID-Nr. 79), 578-582delCCGTG (Seq.-ID-Nr. 80), G2398 + 3A>G (Seq.-ID-Nr. 110), G2398 + 3A>T (Seq.-ID-Nr. 111), 3093-3106del (Seq.-ID-Nr. 125), 3093-3099del/insTTCGC (Seq.-ID-Nr. 126) und 3100delC (Seq.-ID-Nr. 128);
- in dem Gen SCN5A, entsprechend der Aminosäure-Nummerierung die Mutationen: A413E und A413T, T1304M, P1332L, 1505-1507delKPQ, R1623Q, R1644H und R1644C, Y1767C, E1784K
worin das Vorhandensein wenigstens einer Änderung in der Probe von dem Wild-Typ korreliert mit dem QT-Syndrom oder mit der Prädisposition für das Syndrom.

2. Verfahren nach Anspruch 1, worin entsprechende Mutationen mit Oligonukleotiden identifiziert werden, die die folgenden Nonanukleotide oder dazu komplementäre Sequenzen umfassen:
- KCNQ1: Seq.-ID-Nr. 13 (L137F), Seq.-ID-Nr. 16 (R174P), Seq.-ID-Nr. 17 (R190W), Seq.-ID-Nr. 21 (I204M), Seq.-ID-Nr. 24 (R231H), Seq.-ID-Nr.26 (V254L), Seq.-ID-Nr. 27 (H258N), Seq.-ID-Nr. 28 (H258R), Seq.-ID-Nr. 29 (L262V), Seq.-ID-Nr. 34 (V280E), Seq.-ID-Nr. 39 (T322M), Seq.-ID-Nr. 40 (P343L), Seq.-ID-Nr. 41 (P343R), Seq.-ID-Nr. 46 (R360T), Seq.-ID-Nr. 55 (R518G), Seq.-ID-Nr. 56 (R518P), Seq.-ID-Nr. 59 (I567T), Seq.-ID-Nr. 52, Seq.-ID-Nr. 53;
- KCNH2: Seq.-ID-Nr. 67 (Y43C), Seq.-ID-Nr. 69 (E58A), Seq.-ID-Nr. 70 (E58G), Seq.-ID-Nr. 71 (E58D), Seq.-ID-Nr. 75 (del IAQ), Seq.-ID-Nr. 85 (W412stop), Seq.-ID-Nr. 88 (S428L), Seq.-ID-Nr. 97 (G572D), Seq.-ID-Nr. 98 (R852L), Seq.-ID-Nr. 99 (D609H), Seq.-ID-Nr. 106 (S660L), Seq.-ID-Nr. 113 (S818P), Seq.-ID-Nr. 79, Seq.-ID-Nr. 80, Seq.-ID-Nr. 110, Seq.-ID-Nr. 111, Seq.-ID-Nr. 113, Seq.-ID-Nr. 125, Seq.-ID-Nr. 126, Seq.-ID-Nr. 128;
- SCN5A: Seq.-ID-Nr. 133 (A413E), Seq.-ID-Nr. 134 (A413T), Seq.-ID-Nr. 140 (R1644C), Seq.-ID-Nr. 142 (Y1767C).

3. Verfahren nach irgendeinem der Ansprüche 1 und 2, worin die Mutationen nachgewiesen werden entsprechend einer der folgenden Verfahrensweisen:
- Restriktionsmuster eines DNS-Fragments von der Probe, die die Mutation umfasst, gegebenenfalls parallel mit einer Probe entsprechend der Wild-Typ-Sequenz;
- Hybridisierung von Nukleinsäuren der Probe mit spezifischen Sonden unter selektiven Bedingungen;
- PCR;
- Oligonukleotid-Ligations-Assay;
- Elektrophorese, die das Migrationsmuster der Nukleinsäuren der Probe zeigt;
- direktes Sequenzieren;
- denaturierende Hochleistungs-Flüssigkeits-Chromatographie;
worin gemäß jeder der oben genannten Verfahrensweisen das Vorhandensein einer Mutation in der Probe gegebenenfalls weiter bestätigt wird durch Vergleich mit einem Muster, das von Kontroll-Nukleinsäuren erhalten wurde, die die Mutation nicht tragen (oder vom Wild-Typ) oder durch Hybridisierung unter selektiven Bedingungen.

4. Verfahren nach Anspruch 1, weiter umfassend die Sequenz-Charakterisierung der KCNQ1- und/oder KCNH2-Gene.

5. Verfahren nach Anspruch 4, weiter umfassend die Sequenz-Charakterisierung der Gene SCN5A, KCNE1 und/oder KCNE2.

6. Verfahren nach Anspruch 5, worin die Sequenz der Gene **gekennzeichnet** wird durch direktes Sequenzieren mit wenigstens einem der Oligonukleotid-Primer, die in Tabelle 4 aufgelistet sind.

7. Verfahren nach den Ansprüchen 1 bis 6, worin die Probe Genom-DNS ist.

8. Verfahren nach den Ansprüchen 1 bis 6, weiter umfassend einen Schritt der reversen Transkription einer RNS-Probe in eine cDNS.

9. Verfahren nach den Ansprüchen 1 bis 8 zur Diagnose der iatrogenen Long-QT, und worin das Vorhandensein wenigstens einer der Mutationen, wie sie in Anspruch 1 definiert sind, eine Empfindlichkeit für ein Arzneimittel anzeigt, das gewählt ist aus der Gruppe, die besteht aus Antibiotika, prokinetischen Arzneimitteln, antipsychotischen Arzneimitteln, Antidepressiva und antiarrhythmetischen Arzneimitteln.

## Revendications

1. Procédé de diagnostic *in vitro* de la prédisposition au syndrome du QT long ou de diagnostic du syndrome du QT long déclaré, comprenant la détection dans un échantillon d'ADN d'un groupe de mutations qui ne sont pas privées dans les gènes KVLQT1, KCNH2 et SCN5A correspondant aux acides aminés ou aux positions nucléotidiques suivant(e)s :
- dans le gène KCNQ1, selon la numérotation des acides aminés : L137F, R174C et R174P, G179S, R190W et R190Q, I204M, R231C et R231H, D242N, V254L et V254M, H258N et H258R, R259C, L262V, G269D et G269S, S277L, V280E, A300T, W305S et W305stop, G314D et G314S, Y315C, T322M, G325R, A341E et A341V, P343L et P343R, A344E, R360T, R518G, R518P, R518stop, R539W, I567T, R591H, R594Q et,
- dans le gène KCNQ1, selon la numérotation des nucléotides, les mutations : 1514 + 1 G>A, (correspondant à la mutation de la SEQ ID NO : 52), 1513 - 1514delCA correspondant à la SEQ ID NO : 53, la mutation 921 + 1 G>A et la mutation 921 + 2 T>C ;
- dans le gène KCNH2, selon la numérotation des acides aminés, les mutations : Y43C, E58A et E58G et E58D et E58K, del82-84IAQ, W412stop, S428L, S428stop, R534C et R534L, L552S, A561T et A561V, G572C et G572D, R582C et R582L, G604S, D609H et D609G, T613M, A614V, T623I, G628S, S660L, R752W, S818L et S818P, R823W et,
- dans le gène KCNH2, selon la numérotation des nucléotides, les mutations : 453delC, 453-454insCC, 576delG (SEQ ID NO : 79), 578-582delCCGTG (SEQ ID NO : 80), G2398 + 3 A>G (SEQ ID NO : 110), G2398 + 3 A>T (SEQ ID NO : 111), 3093-3106del (SEQ ID NO : 125), 3093-3099del/insTTCGC (SEQ ID NO : 126), et 3100delC (SEQ ID NO : 128) ;
- dans le gène SCN5A, selon la numérotation des acides aminés, les mutations : A413E et A413T, T1304M, P1332L, 1505-1507delKPQ, R1623Q, R1644H et R1644C, Y1767C, E1784K,
dans lequel la présence d'au moins un changement dans l'échantillon par rapport au type sauvage est en corrélation avec le syndrome du QT ou avec la prédisposition audit syndrome.

2. Procédé selon la revendication 1, dans lequel les mutations correspondantes sont identifiées comme étant des oligonucléotides comprenant les nonanucléotides suivants ou leurs séquences complémentaires :
- KCNQ1 : SEQ ID NO : 13 (L137F), SEQ ID NO : 16 (R174P), SEQ ID NO : 17 (R190W), SEQ ID NO : 21 (I204M), SEQ ID NO : 24 (R231H), SEQ ID NO : 26 (V254L), SEQ ID NO : 27 (H258N), SEQ ID NO : 28 (H258R), SEQ ID NO : 29 (L262V), SEQ ID NO : 34 (V280E), SEQ ID NO : 39 (T322M), SEQ ID NO : 40 (P343L), SEQ ID NO : 41 (P343R), SEQ ID NO : 46 (R360T), SEQ ID NO : 55 (R518G), SEQ ID NO : 56 (R518P), SEQ ID NO : 59 (I567T), SEQ ID NO : 52, SEQ ID NO : 53 ;
- KCNH2 : SEQ ID NO : 67 (Y43C), SEQ ID NO : 69 (E58A), SEQ ID NO : 70 (E58G), SEQ ID NO : 71 (E58D), SEQ ID NO : 75 (del IAQ), SEQ ID NO : 85 (W412stop), SEQ ID NO : 88 (S428L), SEQ ID NO : 97 (G572D), SEQ ID NO : 98 (R852L), SEQ ID NO : 99 (D609H), SEQ ID NO : 106 (S660L), SEQ ID NO : 113 (S818P), SEQ ID NO : 79, SEQ ID NO : 80, SEQ ID NO : 110, SEQ ID NO : 111, SEQ ID NO : 113, SEQ ID NO : 125, SEQ ID NO : 126, SEQ ID NO : 128 ;
- SCN5A : SEQ ID NO : 133 (A413E), SEQ ID NO : 134 (A413T), SEQ ID NO : 140 (R1644C), SEQ ID NO : 142 (Y1767C).

3. Procédé selon les revendications 1 et 2, dans lequel lesdites mutations sont détectées selon l'une des techniques suivantes :
- profil de restriction d'un fragment d'ADN provenant de l'échantillon comprenant la mutation, éventuellement en parallèle avec un échantillon correspondant à la séquence de type sauvage,
- hybridation des acides nucléiques de l'échantillon avec des sondes spécifiques dans des conditions sélectives,
- amplification en chaîne par polymérase (PCR),
- dosage basé sur la ligature de sondes oligonucléotidiques,
- électrophorèse présentant le motif de migration des acides nucléiques de l'échantillon,
- séquençage direct,
- chromatographie liquide haute performance dénaturante,
dans lequel, selon chacun des procédés mentionnés ci-dessus, la présence d'une mutation dans l'échantillon est éventuellement encore confirmée par comparaison avec un motif obtenu à partir d'acides nucléiques de contrôle ne portant pas la mutation (ou de type sauvage) ou par hybridation dans des conditions sélectives.

4. Procédé selon la revendication 1, comprenant en outre la caractérisation de la séquence du(des) gène(s) KCNQ1 et/ou KCNH2.

5. Procédé selon la revendication 4, comprenant en outre la caractérisation de la séquence du(des) gène(s) SCN5A, KCNE1 et/ou KCNE2.

6. Procédé selon la revendication 5, dans lequel la séquence desdits gènes est **caractérisée par** un séquençage direct avec au moins une des amorces oligonucléotidiques énumérées dans le tableau 4.

7. Procédé selon les revendications 1 à 6, dans lequel ledit échantillon est de l'ADN génomique.

8. Procédé selon les revendications 1 à 6, comprenant en outre une étape de transcription inverse d'un échantillon d'ARN en ADNc.

9. Procédé selon les revendications 1 à 8, destiné au diagnostic du syndrome du QT long d'origine iatrogène et dans lequel la présence d'au moins une des mutations telles que définies dans la revendication 1 indique une sensibilité à un médicament choisi dans le groupe comprenant : les antibiotiques, les médicaments prokinétiques, les médicaments antipsychotiques, les antidépresseurs, et les médicaments anti-arythmiques.


### SEQUENCE LISTING

<110> FONDAZIONE SALVATORE MAUGERI CLINICA DEL LAVORO E DELLA RIABILITAZIONE I.R.C.C.S. UNIVERSITA' DEGLI STUDI DI PAVIA
<120> Mutations associated with the Long QT Syndrome and diagnostic use thereof
<130> 6655PTWO
<150> MI2005A001047
   <151> 2005-06-07
<160> 212
<170> PatentIn version 3.3
<210> 1
   <211> 2031
   <212> DNA
   <213> Homo sapiens
<220>
   <221> CDS
   <222> (1)..(2031)
   <223> KvLQT1 cDNA (GenBank Acc. N° AF000571): nucleotides 111 to 2141
<400> 1
   atg gcc gcg gcc tcc tcc ccg ccc agg gcc gag agg aag cgc tgg ggt
   48
   tgg ggc cgc ctg cca ggc gcc cgg cgg ggc agc gcg ggc ctg gcc aag
   96
   aag tgc ccc ttc tcg ctg gag ctg gcg gag ggc ggc ccg gcg ggc ggc
   144
   gcg ctc tac gcg ccc atc gcg ccc ggc gcc cca ggt ccc gcg ccc cct
   192
   gcg tcc ccg gcc gcg ccc gcc gcg ccc cca gtt gcc tcc gac ctt ggc
   240
   ccg cgg ccg ccg gtg agc cta gac ccg cgc gtc tcc att tac agc acg
   288
   cgc cgc ccg gtg ttg gcg cgc acc cac gtc cag ggc cgc gtc tac aac
   336
   ttc ctc gag cgt ccc acc ggc tgg aaa tgc ttc gtt tac cac ttc gcc
   384
   gtc ttc ctc atc gtc ctg gtc tgc ctc atc ttc agc gtg ctg tcc acc
   432
   atc gag cag tat gcc gcc ctg gcc acg ggg act ctc ttc tgg atg gag
   480
   atc gtg ctg gtg gtg ttc ttc ggg acg gag tac gtg gtc cgc ctc tgg
   528
   tcc gcc ggc tgc cgc agc aag tac gtg ggc ctc tgg ggg cgg ctg cgc
   576
   ttt gcc cgg aag ccc att tcc atc atc gac ctc atc gtg gtc gtg gcc
   624
   tcc atg gtg gtc ctc tgc gtg ggc tcc aag ggg cag gtg ttt gcc acg
   672
   tcg gcc atc agg ggc atc cgc ttc ctg cag atc ctg agg atg cta cac
   720
   gtc gac cgc cag gga ggc acc tgg agg ctc ctg ggc tcc gtg gtc ttc
   768
   atc cac cgc cag gag ctg ata acc acc ctg tac atc ggc ttc ctg ggc
   816
   ctc atc ttc tcc tcg tac ttt gtg tac ctg gct gag aag gac gcg gtg
   864
   aac gag tca ggc cgc gtg gag ttc ggc agc tac gca gat gcg ctg tgg
   912
   tgg ggg gtg gtc aca gtc acc acc atc ggc tat ggg gac aag gtg ccc
   960
   cag acg tgg gtc ggg aag acc atc gcc tcc tgc ttc tct gtc ttt gcc
   1008
   atc tcc ttc ttt gcg ctc cca gcg ggg att ctt ggc tcg ggg ttt gcc
   1056
   ctg aag gtg cag cag aag cag agg cag aag cac ttc aac cgg cag atc
   1104
   ccg gcg gca gcc tca ctc att cag acc gca tgg agg tgc tat gct gcc
   1152
   gag aac ccc gac tcc tcc acc tgg aag atc tac atc cgg aag gcc ccc
   1200
   cgg agc cac act ctg ctg tca ccc agc ccc aaa ccc aag aag tct gtg
   1248
   gtg gta aag aaa aaa aag ttc aag ctg gac aaa gac aat ggg gtg act
   1296
   cct gga gag aag atg ctc aca gtc ccc cat atc acg tgc gac ccc cca
   1344
   gaa gag cgg cgg ctg gac cac ttc tct gtc gac ggc tat gac agt tct
   1392
   gta agg aag agc cca aca ctg ctg gaa gtg agc atg ccc cat ttc atg
   1440
   aga acc aac agc ttc gcc gag gac ctg gac ctg gaa ggg gag act ctg
   1488
   ctg aca ccc atc acc cac atc tca cag ctg cgg gaa cac cat cgg gcc
   1536
   acc att aag gtc att cga cgc atg cag tac ttt gtg gcc aag aag aaa
   1584
   ttc cag caa gcg cgg aag cct tac gat gtg cgg gac gtc att gag cag
   1632
   tac tcg cag ggc cac ctc aac ctc atg gtg cgc atc aag gag ctg cag
   1680
   agg agg ctg gac cag tcc att ggg aag ccc tca ctg ttc atc tcc gtc
   1728
   tca gaa aag agc aag gat cgc ggc agc aac acg atc ggc gcc cgc ctg
   1776
   aac cga gta gaa gac aag gtg acg cag ctg gac cag agg ctg gca ctc
   1824
   atc acc gac atg ctt cac cag ctg ctc tcc ttg cac ggt ggc agc acc
   1872
   ccc ggc agc ggc ggc ccc ccc aga gag ggc ggg gcc cac atc acc cag
   1920
   ccc tgc ggc agt ggc ggc tcc gtc gac cct gag ctc ttc ctg ccc agc
   1968
   aac acc ctg ccc acc tac gag cag ctg acc gtg ccc agg agg ggc ccc
   2016
   gat gag ggg tcc tga
   2031
<210> 2
   <211> 676
   <212> PRT
   <213> Homo sapiens
<400> 2
<210> 3
   <211> 3480
   <212> DNA
   <213> Homo sapiens
<220>
   <221> CDS
   <222> (1)..(3480)
   <223> KCNH2 cDNA (GenBank Acc. N° NM000238): nucleotides 14 to 3493
<400> 3
atg ccg gtg cgg agg ggc cac gtc gcg ccg cag aac acc ttc ctg gac
   48
   acc atc atc cgc aag ttt gag ggc cag agc cgt aag ttc atc atc gcc
   96
   aac gct cgg gtg gag aac tgc gcc gtc atc tac tgc aac gac ggc ttc
   144
   tgc gag ctg tgc ggc tac tcg cgg gcc gag gtg atg cag cga ccc tgc
   192
   acc tgc gac ttc ctg cac ggg ccg cgc acg cag cgc cgc gct gcc gcg
   240
   cag atc gcg cag gca ctg ctg ggc gcc gag gag cgc aaa gtg gaa atc
   288
   gcc ttc tac cgg aaa gat ggg agc tgc ttc cta tgt ctg gtg gat gtg
   336
   gtg ccc gtg aag aac gag gat ggg gct gtc atc atg ttc atc ctc aat
   384
   ttc gag gtg gtg atg gag aag gac atg gtg ggg tcc ccg gct cat gac
   432
   acc aac cac cgg ggc ccc ccc acc agc tgg ctg gcc cca ggc cgc gcc
   480
   aag acc ttc cgc ctg aag ctg ccc gcg ctg ctg gcg ctg acg gcc cgg
   528
   gag tcg tcg gtg cgg tcg ggc ggc gcg ggc ggc gcg ggc gcc ccg ggg
   576
   gcc gtg gtg gtg gac gtg gac ctg acg ccc gcg gca ccc agc agc gag
   624
   tcg ctg gcc ctg gac gaa gtg aca gcc atg gac aac cac gtg gca ggg
   672
   ctc ggg ccc gcg gag gag cgg cgt gcg ctg gtg ggt ccc ggc tct ccg
   720
   ccc cgc agc gcg ccc ggc cag ctc cca tcg ccc cgg gcg cac agc ctc
   768
   aac ccc gac gcc tcg ggc tcc agc tgc agc ctg gcc cgg acg cgc tcc
   816
   cga gaa agc tgc gcc agc gtg cgc cgc gcc tcg tcg gcc gac gac atc
   864
   gag gcc atg cgc gcc ggg gtg ctg ccc ccg cca ccg cgc cac gcc agc
   912
   acc ggg gcc atg cac cca ctg cgc agc ggc ttg ctc aac tcc acc tcg
   960
   gac tcc gac ctc gtg cgc tac cgc acc att agc aag att ccc caa atc
   1008
   acc ctc aac ttt gtg gac ctc aag ggc gac ccc ttc ttg gct tcg ccc
   1056
   acc agt gac cgt gag atc ata gca cct aag ata aag gag cga acc cac
   1104
   aat gtc act gag aag gtc acc cag gtc ctg tcc ctg ggc gcc gac gtg
   1152
   ctg cct gag tac aag ctg cag gca ccg cgc atc cac cgc tgg acc atc
   1200
   ctg cat tac agc ccc ttc aag gcc gtg tgg gac tgg ctc atc ctg ctg
   1248
   ctg gtc atc tac acg gct gtc ttc aca ccc tac tcg gct gcc ttc ctg
   1296
   ctg aag gag acg gaa gaa ggc ccg cct gct acc gag tgt ggc tac gcc
   1344
   tgc cag ccg ctg gct gtg gtg gac ctc atc gtg gac atc atg ttc att
   1392
   gtg gac atc ctc atc aac ttc cgc acc acc tac gtc aat gcc aac gag
   1440
   gag gtg gtc agc cac ccc ggc cgc atc gcc gtc cac tac ttc aag ggc
   1488
   tgg ttc ctc atc gac atg gtg gcc gcc atc ccc ttc gac ctg ctc atc
   1536
   ttc ggc tct ggc tct gag gag ctg atc ggg ctg ctg aag act gcg cgg
   1584
   ctg ctg cgg ctg gtg cgc gtg gcg cgg aag ctg gat cgc tac tca gag
   1632
   tac ggc gcg gcc gtg ctg ttc ttg ctc atg tgc acc ttt gcg ctc atc
   1680
   gcg cac tgg cta gcc tgc atc tgg tac gcc atc ggc aac atg gag cag
   1728
   cca cac atg gac tca cgc atc ggc tgg ctg cac aac ctg ggc gac cag
   1776
   ata ggc aaa ccc tac aac agc agc ggc ctg ggc ggc ccc tcc atc aag
   1824
   gac aag tat gtg acg gcg ctc tac ttc acc ttc agc agc ctc acc agt
   1872
   gtg ggc ttc ggc aac gtc tct ccc aac acc aac tca gag aag atc ttc
   1920
   tcc atc tgc gtc atg ctc att ggc tcc ctc atg tat gct agc atc ttc
   1968
   ggc aac gtg tcg gcc atc atc cag cgg ctg tac tcg ggc aca gcc cgc
   2016
   tac cac aca cag atg ctg cgg gtg cgg gag ttc atc cgc ttc cac cag
   2064
   atc ccc aat ccc ctg cgc cag cgc ctc gag gag tac ttc cag cac gcc
   2112
   tgg tcc tac acc aac ggc atc gac atg aac gcg gtg ctg aag ggc ttc
   2160
   cct gag tgc ctg cag gct gac atc tgc ctg cac ctg aac cgc tca ctg
   2208
   ctg cag cac tgc aaa ccc ttc cga ggg gcc acc aag ggc tgc ctt cgg
   2256
   gcc ctg gcc atg aag ttc aag acc aca cat gca ccg cca ggg gac aca
   2304
   ctg gtg cat gct ggg gac ctg ctc acc gcc ctg tac ttc atc tcc cgg
   2352
   ggc tcc atc gag atc ctg cgg ggc gac gtc gtc gtg gcc atc ctg ggg
   2400
   aag aat gac atc ttt ggg gag cct ctg aac ctg tat gca agg cct ggc
   2448
   aag tcg aac ggg gat gtg cgg gcc ctc acc tac tgt gac cta cac aag
   2496
   atc cat cgg gac gac ctg ctg gag gtg ctg gac atg tac cct gag ttc
   2544
   tcc gac cac ttc tgg tcc agc ctg gag atc acc ttc aac ctg cga gat
   2592
   acc aac atg atc ccg ggc tcc ccc ggc agt acg gag tta gag ggt ggc
   2640
   ttc agt cgg caa cgc aag cgc aag ttg tcc ttc cgc agg cgc acg gac
   2688
   aag gac acg gag cag cca ggg gag gtg tcg gcc ttg ggg ccg ggc cgg
   2736
   gcg ggg gca ggg ccg agt agc cgg ggc cgg ccg ggg ggg ccg tgg ggg
   2784
   gag agc ccg tcc agt ggc ccc tcc agc cct gag agc agt gag gat gag
   2832
   ggc cca ggc cgc agc tcc agc ccc ctc cgc ctg gtg ccc ttc tcc agc
   2880
   ccc agg ccc ccc gga gag ccg ccg ggt ggg gag ccc ctg atg gag gac
   2928
   tgc gag aag agc agc gac act tgc aac ccc ctg tca ggc gcc ttc tca
   2976
   gga gtg tcc aac att ttc agc ttc  tgg ggg gac agt cgg  ggc cgc cag
   3024
   tac cag  gag ctc cct cga tgc  ccc gcc ccc acc ccc  agc ctc ctc
   3069
   aac atc  ccc ctc tcc agc ccg  ggt cgg cgg ccc cgg  ggc gac gtg
   3114
   gag agc  agg ctg gat gcc ctc  cag cgc cag ctc aac  agg ctg gag
   3159
   acc cgg  ctg agt gca gac atg  gcc act gtc ctg cag  ctg cta cag
   3204
   agg cag  atg acg ctg gtc ccg  ccc gcc tac agt gct  gtg acc acc
   3249
   ccg ggg  cct ggc ccc act tcc  aca tcc ccg ctg ttg  ccc gtc agc
   3294
   ccc ctc  ccc acc ctc acc ttg  gac tcg ctt tct cag  gtt tcc cag
   3339
   ttc atg  gcg tgt gag gag ctg  ccc ccg ggg gcc cca  gag ctt ccc
   3384
   caa gaa  ggc ccc aca cga cgc  ctc tcc cta ccg ggc  cag ctg ggg
   3429
   gcc ctc  acc tcc cag ccc ctg  cac aga cac ggc tcg  gac ccg ggc
   3474
   agt tag
   3480
<210> 4
   <211> 1159
   <212> PRT
   <213> Homo sapiens
<400> 4
<210> 5
   <211> 6051
   <212> DNA
   <213> Homo sapiens
<220>
   <221> CDS
   <222> (1)..(6051)
   <223> SCN5A cDNA (GenBank Acc. N° NM000335): nucleotides 151 to 6201

<400> 5
   atg gca aac ttc cta tta cct cgg ggc acc agc agc ttc cgc agg ttc
   48
   Met Ala Asn Phe Leu Leu Pro Arg Gly Thr Ser Ser Phe Arg Arg Phe
   1 5 10 15
   aca cgg gag tcc ctg gca gcc atc gag aag cgc atg gcg gag aag caa
   96
   Thr Arg Glu Ser Leu Ala Ala Ile Glu Lys Arg Met Ala Glu Lys Gln
   20 25 30
   gcc cgc ggc tca acc acc ttg cag gag agc cga gag ggg ctg ccc gag
   144
   Ala Arg Gly Ser Thr Thr Leu Gln Glu Ser Arg Glu Gly Leu Pro Glu
   35 40 45
   gag gag gct ccc cgg ccc cag ctg gac ctg cag gcc tcc aaa aag ctg
   192
   Glu Glu Ala Pro Arg Pro Gln Leu Asp Leu Gln Ala Ser Lys Lys Leu
   50 55 60
   cca gat ctc tat ggc aat cca ccc caa gag ctc atc gga gag ccc ctg
   240
   Pro Asp Leu Tyr Gly Asn Pro Pro Gln Glu Leu Ile Gly Glu Pro Leu
   65 70 75 80
   gag gac ctg gac ccc ttc tat agc acc caa aag act ttc atc gta ctg
   288
   Glu Asp Leu Asp Pro Phe Tyr Ser Thr Gln Lys Thr Phe Ile Val Leu
   85 90 95
   aat aaa ggc aag acc atc ttc cgg ttc agt gcc acc aac gcc ttg tat
   336
   Asn Lys Gly Lys Thr Ile Phe Arg Phe Ser Ala Thr Asn Ala Leu Tyr
               100                 105                 110
   gtc ctc agt ccc ttc cac cca gtt cgg aga gcg gct gtg aag att ctg
   384
   Val Leu Ser Pro Phe His Pro Val Arg Arg Ala Ala Val Lys Ile Leu
           115                 120                 125
   gtt cac tcg ctc ttc aac atg ctc atc atg tgc acc atc ctc acc aac
   432
   Val His Ser Leu Phe Asn Met Leu Ile Met Cys Thr Ile Leu Thr Asn
       130                 135                 140
   tgc gtg ttc atg gcc cag cac gac cct cca ccc tgg acc aag tat gtc
   480
   Cys Val Phe Met Ala Gln His Asp Pro Pro Pro Trp Thr Lys Tyr Val
   145                 150                 155                 160
   gag tac acc ttc acc gcc att tac acc ttt gag tct ctg gtc aag att
   528
   Glu Tyr Thr Phe Thr Ala Ile Tyr Thr Phe Glu Ser Leu Val Lys Ile
                   165                 170                 175
   ctg gct cga gct ttc tgc ctg cac gcg ttc act ttc ctt cgg gac cca
   576
   Leu Ala Arg Ala Phe Cys Leu His Ala Phe Thr Phe Leu Arg Asp Pro
               180                 185                 190
   tgg aac tgg ctg gac ttt agt gtg att atc atg gca tac aca act gaa
   624
   Trp Asn Trp Leu Asp Phe Ser Val Ile Ile Met Ala Tyr Thr Thr Glu
           195                 200                 205
   ttt gtg gac ctg ggc aat gtc tca gcc tta cgc acc ttc cga gtc ctc
   672
   Phe Val Asp Leu Gly Asn Val Ser Ala Leu Arg Thr Phe Arg Val Leu
       210                 215                 220
   cgg gcc ctg aaa act ata tca gtc att tca ggg ctg aag acc atc gtg
   720
   Arg Ala Leu Lys Thr Ile Ser Val Ile Ser Gly Leu Lys Thr Ile Val
   225                 230                 235                 240
   ggg gcc ctg atc cag tct gtg aag aag ctg gct gat gtg atg gtc ctc
   768
   Gly Ala Leu Ile Gln Ser Val Lys Lys Leu Ala Asp Val Met Val Leu
                   245                 250                 255
   aca gtc ttc tgc ctc agc gtc ttt gcc ctc atc ggc ctg cag ctc ttc
   816
   Thr Val Phe Cys Leu Ser Val Phe Ala Leu Ile Gly Leu Gln Leu Phe
               260                 265                 270
   atg ggc aac cta agg cac aag tgt gtg cgc aac ttc aca gcg ctc aac
   864
   Met Gly Asn Leu Arg His Lys Cys Val Arg Asn Phe Thr Ala Leu Asn
           275                 280                 285
   ggc acc aac ggc tcc gtg gag gcc gac ggc ttg gtc tgg gaa tcc ctg
   912
   Gly Thr Asn Gly Ser Val Glu Ala Asp Gly Leu Val Trp Glu Ser Leu
       290                 295                 300
   gac ctt tac ctc agt gat cca gaa aat tac ctg ctc aag aac ggc acc
   960
   Asp Leu Tyr Leu Ser Asp Pro Glu Asn Tyr Leu Leu Lys Asn Gly Thr
   305                 310                 315                 320
   tct gat gtg tta ctg tgt ggg aac agc tct gac gct ggg aca tgt ccg
   1008
   Ser Asp Val Leu Leu Cys Gly Asn Ser Ser Asp Ala Gly Thr Cys Pro
                   325                 330                 335
   gag ggc tac cgg tgc cta aag gca ggc gag aac ccc gac cac ggc tac
   1056
   Glu Gly Tyr Arg Cys Leu Lys Ala Gly Glu Asn Pro Asp His Gly Tyr
               340                 345                 350
   acc agc ttc gat tcc ttt gcc tgg gcc ttt ctt gca ctc ttc cgc ctg
   1104
   Thr Ser Phe Asp Ser Phe Ala Trp Ala Phe Leu Ala Leu Phe Arg Leu
           355                 360                 365
   atg acg cag gac tgc tgg gag cgc ctc tat cag cag acc ctc agg tcc
   1152
   Met Thr Gln Asp Cys Trp Glu Arg Leu Tyr Gln Gln Thr Leu Arg Ser
       370                 375                 380
   gca ggg aag atc tac atg atc ttc ttc atg ctt gtc atc ttc ctg ggg
   1200
   Ala Gly Lys Ile Tyr Met Ile Phe Phe Met Leu Val Ile Phe Leu Gly
   385                 390                 395                 400
   tcc ttc tac ctg gtg aac ctg atc ctg gcc gtg gtc gca atg gcc tat
   1248
   Ser Phe Tyr Leu Val Asn Leu Ile Leu Ala Val Val Ala Met Ala Tyr
                   405                 410                 415
   gag gag caa aac caa gcc acc atc gct gag acc gag gag aag gaa aag
   1296
   Glu Glu Gln Asn Gln Ala Thr Ile Ala Glu Thr Glu Glu Lys Glu Lys
               420                 425                 430
   cgc ttc cag gag gcc atg gaa atg ctc aag aaa gaa cac gag gcc ctc
   1344
   Arg Phe Gln Glu Ala Met Glu Met Leu Lys Lys Glu His Glu Ala Leu
           435                 440                 445
   acc atc agg ggt gtg gat acc gtg tcc cgt agc tcc ttg gag atg tcc
   1392
   Thr Ile Arg Gly Val Asp Thr Val Ser Arg Ser Ser Leu Glu Met Ser
       450                 455                 460
   cct ttg gcc cca gta aac agc cat gag aga aga agc aag agg aga aaa
   1440
   Pro Leu Ala Pro Val Asn Ser His Glu Arg Arg Ser Lys Arg Arg Lys
   465                 470                 475                 480
   cgg atg tct tca gga act gag gag tgt ggg gag gac agg ctc ccc aag
   1488
   Arg Met Ser Ser Gly Thr Glu Glu Cys Gly Glu Asp Arg Leu Pro Lys
                   485                 490                 495
   tct gac tca gaa gat ggt ccc aga gca atg aat cat ctc agc ctc acc
   1536
   Ser Asp Ser Glu Asp Gly Pro Arg Ala Met Asn His Leu Ser Leu Thr
               500                 505                 510
   cgt ggc ctc agc agg act tct atg aag cca cgt tcc agc cgc ggg agc
   1584
   Arg Gly Leu Ser Arg Thr Ser Met Lys Pro Arg Ser Ser Arg Gly Ser
           515                 520                 525
   att ttc acc ttt cgc agg cga gac ctg ggt tct gaa gca gat ttt gca
   1632
   Ile Phe Thr Phe Arg Arg Arg Asp Leu Gly Ser Glu Ala Asp Phe Ala
       530                 535                 540
   gat gat gaa aac agc aca gcg cgg gag agc gag agc cac cac aca tca
   1680
   Asp Asp Glu Asn Ser Thr Ala Arg Glu Ser Glu Ser His His Thr Ser
   545                 550                 555                 560
   ctg ctg gtg ccc tgg ccc ctg cgc cgg acc agt gcc cag gga cag ccc
   1728
   Leu Leu Val Pro Trp Pro Leu Arg Arg Thr Ser Ala Gln Gly Gln Pro
                   565                 570                 575
   agt ccc gga acc tcg gct cct ggc cac gcc ctc cat ggc aaa aag aac
   1776
   Ser Pro Gly Thr Ser Ala Pro Gly His Ala Leu His Gly Lys Lys Asn
               580                 585                 590
   agc act gtg gac tgc aat ggg gtg gtc tca tta ctg ggg gca ggc gac
   1824
   Ser Thr Val Asp Cys Asn Gly Val Val Ser Leu Leu Gly Ala Gly Asp
           595                 600                 605
   cca gag gcc aca tcc cca gga agc cac ctc ctc cgc cct gtg atg cta
   1872
   Pro Glu Ala Thr Ser Pro Gly Ser His Leu Leu Arg Pro Val Met Leu
       610                 615                 620
   gag cac ccg cca gac acg acc acg cca tcg gag gag cca ggc ggc ccc
   1920
   Glu His Pro Pro Asp Thr Thr Thr Pro Ser Glu Glu Pro Gly Gly Pro
   625                 630                 635                 640
   cag atg ctg acc tcc cag gct ccg tgt gta gat ggc ttc gag gag cca
   1968
   Gln Met Leu Thr Ser Gln Ala Pro Cys Val Asp Gly Phe Glu Glu Pro
                   645                 650                 655
   gga gca cgg cag cgg gcc ctc agc gca gtc agc gtc ctc aca agc gca
   2016
   Gly Ala Arg Gln Arg Ala Leu Ser Ala Val Ser Val Leu Thr Ser Ala
               660                 665                 670
   ctg gaa gag tta gag gag tct cgc cac aag tgt cca cca tgc tgg aac
   2064
   Leu Glu Glu Leu Glu Glu Ser Arg His Lys Cys Pro Pro Cys Trp Asn
   675 680 685
   cgt ctc gcc cag cgc tac ctg atc tgg gag tgc tgc ccg ctg tgg atg
   2112
   Arg Leu Ala Gln Arg Tyr Leu Ile Trp Glu Cys Cys Pro Leu Trp Met
   690 695 700
   tcc atc aag cag gga gtg aag ttg gtg gtc atg gac ccg ttt act gac
   2160
   Ser Ile Lys Gln Gly Val Lys Leu Val Val Met Asp Pro Phe Thr Asp
   705 710 715 720
   ctc acc atc act atg tgc atc gta ctc aac aca ctc ttc atg gcg ctg
   2208
   Leu Thr Ile Thr Met Cys Ile Val Leu Asn Thr Leu Phe Met Ala Leu
   725 730 735
   gag cac tac aac atg aca agt gaa ttc gag gag atg ctg cag gtc gga
   2256
   Glu His Tyr Asn Met Thr Ser Glu Phe Glu Glu Met Leu Gln Val Gly
   740 745 750
   aac ctg gtc ttc aca ggg att ttc aca gca gag atg acc ttc aag atc
   2304
   Asn Leu Val Phe Thr Gly Ile Phe Thr Ala Glu Met Thr Phe Lys Ile
   755 760 765
   att gcc ctc gac ccc tac tac tac ttc caa cag ggc tgg aac atc ttc
   2352
   Ile Ala Leu Asp Pro Tyr Tyr Tyr Phe Gln Gln Gly Trp Asn Ile Phe
   770 775 780
   gac agc atc atc gtc atc ctt agc ctc atg gag ctg ggc ctg tcc cgc
   2400
   Asp Ser Ile Ile Val Ile Leu Ser Leu Met Glu Leu Gly Leu Ser Arg
   785 790 795 800
   atg agc aac ttg tcg gtg ctg cgc tcc ttc cgc ctg ctg cgg gtc ttc
   2448
   Met Ser Asn Leu Ser Val Leu Arg Ser Phe Arg Leu Leu Arg Val Phe
   805 810 815
   aag ctg gcc aaa tca tgg ccc acc ctg aac aca ctc atc aag atc atc
   2496
   Lys Leu Ala Lys Ser Trp Pro Thr Leu Asn Thr Leu Ile Lys Ile Ile
   820 825 830
   ggg aac tca gtg ggg gca ctg ggg aac ctg aca ctg gtg cta gcc atc
   2544
   Gly Asn Ser Val Gly Ala Leu Gly Asn Leu Thr Leu Val Leu Ala Ile
   835 840 845
   atc gtg ttc atc ttt gct gtg gtg ggc atg cag ctc ttt ggc aag aac
   2592
   Ile Val Phe Ile Phe Ala Val Val Gly Met Gln Leu Phe Gly Lys Asn
   850 855 860
   tac tcg gag ctg agg gac agc gac tca ggc ctg ctg cct cgc tgg cac
   2640
   Tyr Ser Glu Leu Arg Asp Ser Asp Ser Gly Leu Leu Pro Arg Trp His
   865                 870                 875                 880
   atg atg gac ttc ttt cat gcc ttc cta atc atc ttc cgc atc ctc tgt
   2688
   Met Met Asp Phe Phe His Ala Phe Leu Ile Ile Phe Arg Ile Leu Cys
                   885                 890                 895
   gga gag tgg atc gag acc atg tgg gac tgc atg gag gtg tcg ggg cag
   2736
   Gly Glu Trp Ile Glu Thr Met Trp Asp Cys Met Glu Val Ser Gly Gln
               900                 905                 910
   tca tta tgc ctg ctg gtc ttc ttg ctt gtt atg gtc att ggc aac ctt
   2784
   Ser Leu Cys Leu Leu Val Phe Leu Leu Val Met Val Ile Gly Asn Leu
           915                 920                 925
   gtg gtc ctg aat ctc ttc ctg gcc ttg ctg ctc agc tcc ttc agt gca
   2832
   Val Val Leu Asn Leu Phe Leu Ala Leu Leu Leu Ser Ser Phe Ser Ala
       930                 935                 940
   gac aac ctc aca gcc cct gat gag gac aga gag atg aac aac ctc cag
   2880
   Asp Asn Leu Thr Ala Pro Asp Glu Asp Arg Glu Met Asn Asn Leu Gln
   945                 950                 955                 960
   ctg gcc ctg gcc cgc atc cag agg ggc ctg cgc ttt gtc aag cgg acc
   2928
   Leu Ala Leu Ala Arg Ile Gln Arg Gly Leu Arg Phe Val Lys Arg Thr
                   965                 970                 975
   acc tgg gat ttc tgc tgt ggt ctc ctg cgg cac cgg cct cag aag ccc
   2976
   Thr Trp Asp Phe Cys Cys Gly Leu Leu Arg His Arg Pro Gln Lys Pro
               980                 985                 990
   gca gcc ctt gcc gcc cag ggc cag  ctg ccc agc tgc att  gcc acc ccc
   3024
   Ala Ala Leu Ala Ala Gln Gly Gln  Leu Pro Ser Cys Ile  Ala Thr Pro
           995                 1000                 1005
   tac tcc  ccg cca ccc cca gag  acg gag aag gtg cct  ccc acc cgc
   3069
   Tyr Ser  Pro Pro Pro Pro Glu  Thr Glu Lys Val Pro  Pro Thr Arg
       1010                 1015                 1020
   aag gaa  aca cag ttt gag gaa  ggc gag caa cca ggc  cag ggc acc
   3114
   Lys Glu  Thr Gln Phe Glu Glu  Gly Glu Gln Pro Gly  Gln Gly Thr
       1025                 1030                 1035
   ccc ggg  gat cca gag ccc gtg  tgt gtg ccc atc gct  gtg gcc gag
   3159
   Pro Gly  Asp Pro Glu Pro Val  Cys Val Pro Ile Ala  Val Ala Glu
       1040                 1045                 1050
   tca gac aca gat gac caa gaa gag gat gag gag aac agc ctg ggc 3204
   Ser Asp Thr Asp Asp Gln Glu Glu Asp Glu Glu Asn Ser Leu Gly
   1055 1060 1065
   acg gag gag gag tcc agc aag cag cag gaa tcc cag cct gtg tcc 3249
   Thr Glu Glu Glu Ser Ser Lys Gln Gln Glu Ser Gln Pro Val Ser
   1070 1075 1080
   ggc tgg ccc aga ggc cct ccg gat tcc agg acc tgg agc cag gtg 3294
   Gly Trp Pro Arg Gly Pro Pro Asp Ser Arg Thr Trp Ser Gln Val
   1085 1090 1095
   tca gcg act gcc tcc tct gag gcc gag gcc agt gca tct cag gcc 3339
   Ser Ala Thr Ala Ser Ser Glu Ala Glu Ala Ser Ala Ser Gln Ala
   1100 1105 1110
   gac tgg cgg cag cag tgg aaa gcg gaa ccc cag gcc cca ggg tgc 3384
   Asp Trp Arg Gln Gln Trp Lys Ala Glu Pro Gln Ala Pro Gly Cys
   1115 1120 1125
   ggt gag acc cca gag gac agt tgc tcc gag ggc agc aca gca gac 3429
   Gly Glu Thr Pro Glu Asp Ser Cys Ser Glu Gly Ser Thr Ala Asp
   1130 1135 1140
   atg acc aac acc gct gag ctc ctg gag cag atc cct gac ctc ggc 3474
   Met Thr Asn Thr Ala Glu Leu Leu Glu Gln Ile Pro Asp Leu Gly
   1145 1150 1155
   cag gat gtc aag gac cca gag gac tgc ttc act gaa ggc tgt gtc 3519
   Gln Asp Val Lys Asp Pro Glu Asp Cys Phe Thr Glu Gly Cys Val
   1160 1165 1170
   cgg cgc tgt ccc tgc tgt gcg gtg gac acc aca cag gcc cca ggg 3564
   Arg Arg Cys Pro Cys Cys Ala Val Asp Thr Thr Gln Ala Pro Gly
   1175 1180 1185
   aag gtc tgg tgg cgg ttg cgc aag acc tgc tac cac atc gtg gag 3609
   Lys Val Trp Trp Arg Leu Arg Lys Thr Cys Tyr His Ile Val Glu
   1190 1195 1200
   cac agc tgg ttc gag aca ttc atc atc ttc atg atc cta ctc agc 3654
   His Ser Trp Phe Glu Thr Phe Ile Ile Phe Met Ile Leu Leu Ser
   1205 1210 1215
   agt gga gcg ctg gcc ttc gag gac atc tac cta gag gag cgg aag 3699
   Ser Gly Ala Leu Ala Phe Glu Asp Ile Tyr Leu Glu Glu Arg Lys
   1220 1225 1230
   acc atc  aag gtt ctg ctt gag  tat gcc gac aag atg  ttc aca tat
   3744
   Thr Ile  Lys Val Leu Leu Glu  Tyr Ala Asp Lys Met  Phe Thr Tyr
       1235                 1240                 1245
   gtc ttc  gtg ctg gag atg ctg  ctc aag tgg gtg gcc  tac ggc ttc
   3789
   Val Phe  Val Leu Glu Met Leu  Leu Lys Trp Val Ala  Tyr Gly Phe
       1250                 1255                 1260
   aag aag  tac ttc acc aat gcc  tgg tgc tgg ctc gac  ttc ctc atc
   3834
   Lys Lys  Tyr Phe Thr Asn Ala  Trp Cys Trp Leu Asp  Phe Leu Ile
       1265                 1270                 1275
   gta gac  gtc tct ctg gtc agc  ctg gtg gcc aac acc  ctg ggc ttt
   3879
   Val Asp  Val Ser Leu Val Ser  Leu Val Ala Asn Thr  Leu Gly Phe
       1280                 1285                 1290
   gcc gag  atg ggc ccc atc aag  tca ctg cgg acg ctg  cgt gca ctc
   3924
   Ala Glu  Met Gly Pro Ile Lys  Ser Leu Arg Thr Leu  Arg Ala Leu
       1295                 1300                 1305
   cgt cct  ctg aga gct ctg tca  cga ttt gag ggc atg  agg gtg gtg
   3969
   Arg Pro  Leu Arg Ala Leu Ser  Arg Phe Glu Gly Met  Arg Val Val
       1310                 1315                 1320
   gtc aat  gcc ctg gtg ggc gcc  atc ccg tcc atc atg  aac gtc ctc
   4014
   Val Asn  Ala Leu Val Gly Ala  Ile Pro Ser Ile Met  Asn Val Leu
       1325                 1330                 1335
   ctc gtc  tgc ctc atc ttc tgg  ctc atc ttc agc atc  atg ggc gtg
   4059
   Leu Val  Cys Leu Ile Phe Trp  Leu Ile Phe Ser Ile  Met Gly Val
       1340                 1345                 1350
   aac ctc  ttt gcg ggg aag ttt  ggg agg tgc atc aac  cag aca gag
   4104
   Asn Leu  Phe Ala Gly Lys Phe  Gly Arg Cys Ile Asn  Gln Thr Glu
       1355                 1360                 1365
   gga gac  ttg cct ttg aac tac  acc atc gtg aac aac  aag agc cag
   4149
   Gly Asp  Leu Pro Leu Asn Tyr  Thr Ile Val Asn Asn  Lys Ser Gln
       1370                 1375                 1380
   tgt gag  tcc ttg aac ttg acc  gga gaa ttg tac tgg  acc aag gtg
   4194
   Cys Glu  Ser Leu Asn Leu Thr  Gly Glu Leu Tyr Trp  Thr Lys Val
       1385                 1390                 1395
   aaa gtc  aac ttt gac aac gtg  ggg gcc ggg tac ctg  gcc ctt ctg
   4239
   Lys Val  Asn Phe Asp Asn Val  Gly Ala Gly Tyr Leu  Ala Leu Leu
       1400                 1405                 1410
   cag gtg  gca aca ttt aaa ggc  tgg atg gac att atg  tat gca gct
   4284
   Gln Val  Ala Thr Phe Lys Gly  Trp Met Asp Ile Met  Tyr Ala Ala
       1415                 1420                 1425
   gtg gac  tcc agg ggg tat gaa  gag cag cct cag tgg  gaa tac aac
   4329
   Val Asp  Ser Arg Gly Tyr Glu  Glu Gln Pro Gln Trp  Glu Tyr Asn
       1430                 1435                 1440
   ctc tac  atg tac atc tat ttt  gtc att ttc atc atc  ttt ggg tct
   4374
   Leu Tyr  Met Tyr Ile Tyr Phe  Val Ile Phe Ile Ile  Phe Gly Ser
       1445                 1450                 1455
   ttc ttc  acc ctg aac ctc ttt  att ggt gtc atc att  gac aac ttc
   4419
   Phe Phe  Thr Leu Asn Leu Phe  Ile Gly Val Ile Ile  Asp Asn Phe
       1460                 1465                 1470
   aac caa  cag aag aaa aag tta  ggg ggc cag gac atc  ttc atg aca
   4464
   Asn Gln  Gln Lys Lys Lys Leu  Gly Gly Gln Asp Ile  Phe Met Thr
       1475                 1480                 1485
   gag gag  cag aag aag tac tac  aat gcc atg aag aag  ctg ggc tcc
   4509
   Glu Glu  Gln Lys Lys Tyr Tyr  Asn Ala Met Lys Lys  Leu Gly Ser
       1490                 1495                 1500
   aag aag  ccc cag aag ccc atc  cca cgg ccc ctg aac  aag tac cag
   4554
   Lys Lys  Pro Gln Lys Pro Ile  Pro Arg Pro Leu Asn  Lys Tyr Gln
       1505                 1510                 1515
   ggc ttc  ata ttc gac att gtg  acc aag cag gcc ttt  gac gtc acc
   4599
   Gly Phe  Ile Phe Asp Ile Val  Thr Lys Gln Ala Phe  Asp Val Thr
       1520                 1525                 1530
   atc atg  ttt ctg atc tgc ttg  aat atg gtg acc atg  atg gtg gag
   4644
   Ile Met  Phe Leu Ile Cys Leu  Asn Met Val Thr Met  Met Val Glu
       1535                 1540                 1545
   aca gat  gac caa agt cct gag  aaa atc aac atc ttg  gcc aag atc
   4689
   Thr Asp  Asp Gln Ser Pro Glu  Lys Ile Asn Ile Leu  Ala Lys Ile
       1550                 1555                 1560
   aac ctg  ctc ttt gtg gcc atc  ttc aca ggc gag tgt  att gtc aag
   4734
   Asn Leu  Leu Phe Val Ala Ile  Phe Thr Gly Glu Cys  Ile Val Lys
       1565                 1570                 1575
   ctg gct  gcc ctg cgc cac tac  tac ttc acc aac agc  tgg aat atc
   4779
   Leu Ala  Ala Leu Arg His Tyr  Tyr Phe Thr Asn Ser  Trp Asn Ile
       1580                 1585                 1590
   ttc gac ttc gtg gtt gtc atc ctc tcc atc gtg ggc act gtg ctc
   4824
   Phe Asp Phe Val Val Val Ile Leu Ser Ile Val Gly Thr Val Leu
   1595 1600 1605
   tcg gac atc atc cag aag tac ttc ttc tcc ccg acg ctc ttc cga
   4869
   Ser Asp Ile Ile Gln Lys Tyr Phe Phe Ser Pro Thr Leu Phe Arg
   1610 1615 1620
   gtc atc cgc ctg gcc cga ata ggc cgc atc ctc aga ctg atc cga
   4914
   Val Ile Arg Leu Ala Arg Ile Gly Arg Ile Leu Arg Leu Ile Arg
   1625 1630 1635
   ggg gcc aag ggg atc cgc acg ctg ctc ttt gcc ctc atg atg tcc
   4959
   Gly Ala Lys Gly Ile Arg Thr Leu Leu Phe Ala Leu Met Met Ser
   1640 1645 1650
   ctg cct gcc ctc ttc aac atc ggg ctg ctg ctc ttc ctc gtc atg
   5004
   Leu Pro Ala Leu Phe Asn Ile Gly Leu Leu Leu Phe Leu Val Met
   1655 1660 1665
   ttc atc tac tcc atc ttt ggc atg gcc aac ttc gct tat gtc aag
   5049
   Phe Ile Tyr Ser Ile Phe Gly Met Ala Asn Phe Ala Tyr Val Lys
   1670 1675 1680
   tgg gag gct ggc atc gac gac atg ttc aac ttc cag acc ttc gcc
   5094
   Trp Glu Ala Gly Ile Asp Asp Met Phe Asn Phe Gln Thr Phe Ala
   1685 1690 1695
   aac agc atg ctg tgc ctc ttc cag atc acc acg tcg gcc ggc tgg
   5139
   Asn Ser Met Leu Cys Leu Phe Gln Ile Thr Thr Ser Ala Gly Trp
   1700 1705 1710
   gat ggc ctc ctc agc ccc atc ctc aac act ggg ccg ccc tac tgc
   5184
   Asp Gly Leu Leu Ser Pro Ile Leu Asn Thr Gly Pro Pro Tyr Cys
   1715 1720 1725
   gac ccc act ctg ccc aac agc aat ggc tct cgg ggg gac tgc ggg
   5229
   Asp Pro Thr Leu Pro Asn Ser Asn Gly Ser Arg Gly Asp Cys Gly
   1730 1735 1740
   agc cca gcc gtg ggc atc ctc ttc ttc acc acc tac atc atc atc
   5274
   Ser Pro Ala Val Gly Ile Leu Phe Phe Thr Thr Tyr Ile Ile Ile
   1745 1750 1755
   tcc ttc ctc atc gtg gtc aac atg tac att gcc atc atc ctg gag
   5319
   Ser Phe Leu Ile Val Val Asn Met Tyr Ile Ala Ile Ile Leu Glu
   1760 1765 1770
   aac ttc  agc gtg gcc acg gag  gag agc acc gag ccc  ctg agt gag
   5364
   Asn Phe  Ser Val Ala Thr Glu  Glu Ser Thr Glu Pro  Leu Ser Glu
       1775                 1780                 1785
   gac gac  ttc gat atg ttc tat  gag atc tgg gag aaa  ttt gac cca
   5409
   Asp Asp  Phe Asp Met Phe Tyr  Glu Ile Trp Glu Lys  Phe Asp Pro
       1790                 1795                 1800
   gag gcc  act cag ttt att gag  tat tcg gtc ctg tct  gac ttt gcc
   5454
   Glu Ala  Thr Gln Phe Ile Glu  Tyr Ser Val Leu Ser  Asp Phe Ala
       1805                 1810                 1815
   gac gcc  ctg tct gag cca ctc  cgt atc gcc aag ccc  aac cag ata
   5499
   Asp Ala  Leu Ser Glu Pro Leu  Arg Ile Ala Lys Pro  Asn Gln Ile
       1820                 1825                 1830
   agc ctc  atc aac atg gac ctg  ccc atg gtg agt ggg  gac cgc atc
   5544
   Ser Leu  Ile Asn Met Asp Leu  Pro Met Val Ser Gly  Asp Arg Ile
       1835                 1840                 1845
   cat tgc  atg gac att ctc ttt  gcc ttc acc aaa agg  gtc ctg ggg
   5589
   His Cys  Met Asp Ile Leu Phe  Ala Phe Thr Lys Arg  Val Leu Gly
       1850                 1855                 1860
   gag tct  ggg gag atg gac gcc  ctg aag atc cag atg  gag gag aag
   5634
   Glu Ser  Gly Glu Met Asp Ala  Leu Lys Ile Gln Met  Glu Glu Lys
       1865                 1870                 1875
   ttc atg  gca gcc aac cca tcc  aag atc tcc tac gag  ccc atc acc
   5679
   Phe Met  Ala Ala Asn Pro Ser  Lys Ile Ser Tyr Glu  Pro Ile Thr
       1880                 1885                 1890
   acc aca  ctc cgg cgc aag cac  gaa gag gtg tcg gcc  atg gtt atc
   5724
   Thr Thr  Leu Arg Arg Lys His  Glu Glu Val Ser Ala  Met Val Ile
       1895                 1900                 1905
   cag aga  gcc ttc cgc agg cac  ctg ctg caa cgc tct  ttg aag cat
   5769
   Gln Arg  Ala Phe Arg Arg His  Leu Leu Gln Arg Ser  Leu Lys His
       1910                 1915                 1920
   gcc tcc  ttc ctc ttc cgt cag  cag gcg ggc agc ggc  ctc tcc gaa
   5814
   Ala Ser  Phe Leu Phe Arg Gln  Gln Ala Gly Ser Gly  Leu Ser Glu
       1925                 1930                 1935
   gag gat  gcc cct gag cga gag  ggc ctc atc gcc tac  gtg atg agt
   5859
   Glu Asp  Ala Pro Glu Arg Glu  Gly Leu Ile Ala Tyr  Val Met Ser
       1940                 1945                 1950
   gag aac  ttc tcc cga ccc ctt  ggc cca ccc tcc agc  tcc tcc atc
   5904
   Glu Asn  Phe Ser Arg Pro Leu  Gly Pro Pro Ser Ser  Ser Ser Ile
       1955                 1960                 1965
   tcc tcc  act tcc ttc cca ccc  tcc tat gac agt gtc  act aga gcc
   5949
   Ser Ser  Thr Ser Phe Pro Pro  Ser Tyr Asp Ser Val  Thr Arg Ala
       1970                 1975                 1980
   acc agc  gat aac ctc cag gtg  cgg ggg tct gac tac  agc cac agt
   5994
   Thr Ser  Asp Asn Leu Gln Val  Arg Gly Ser Asp Tyr  Ser His Ser
       1985                 1990                 1995
   gaa gat  ctc gcc gac ttc ccc  cct tct ccg gac agg  gac cgt gag
   6039
   Glu Asp  Leu Ala Asp Phe Pro  Pro Ser Pro Asp Arg  Asp Arg Glu
       2000                 2005                 2010
   tcc atc  gtg tga
   6051
   Ser Ile  Val
       2015
<210> 6
   <211> 2016
   <212> PRT
   <213> Homo sapiens
<400> 6
   Met Ala Asn Phe Leu Leu Pro Arg Gly Thr Ser Ser Phe Arg Arg Phe
   1               5                   10                  15
   Thr Arg Glu Ser Leu Ala Ala Ile Glu Lys Arg Met Ala Glu Lys Gln
               20                  25                  30
   Ala Arg Gly Ser Thr Thr Leu Gln Glu Ser Arg Glu Gly Leu Pro Glu
           35                  40                  45
   Glu Glu Ala Pro Arg Pro Gln Leu Asp Leu Gln Ala Ser Lys Lys Leu
       50                  55                  60
   Pro Asp Leu Tyr Gly Asn Pro Pro Gln Glu Leu Ile Gly Glu Pro Leu
   65                  70                  75                  80
   Glu Asp Leu Asp Pro Phe Tyr Ser Thr Gln Lys Thr Phe Ile Val Leu
                   85                  90                  95
   Asn Lys Gly Lys Thr Ile Phe Arg Phe Ser Ala Thr Asn Ala Leu Tyr
               100                 105                 110
   Val Leu Ser Pro Phe His Pro Val Arg Arg Ala Ala Val Lys Ile Leu
           115                 120                 125
   Val His Ser Leu Phe Asn Met Leu Ile Met Cys Thr Ile Leu Thr Asn
       130                 135                 140
   Cys Val Phe Met Ala Gln His Asp Pro Pro Pro Trp Thr Lys Tyr Val
   145                 150                 155                 160
   Glu Tyr Thr Phe Thr Ala Ile Tyr Thr Phe Glu Ser Leu Val Lys Ile
                   165                 170                 175
   Leu Ala Arg Ala Phe Cys Leu His Ala Phe Thr Phe Leu Arg Asp Pro
               180                 185                 190
   Trp Asn Trp Leu Asp Phe Ser Val Ile Ile Met Ala Tyr Thr Thr Glu
           195                 200                 205
   Phe Val Asp Leu Gly Asn Val Ser Ala Leu Arg Thr Phe Arg Val Leu
       210                 215                 220
   Arg Ala Leu Lys Thr Ile Ser Val Ile Ser Gly Leu Lys Thr Ile Val
   225                 230                 235                 240
   Gly Ala Leu Ile Gln Ser Val Lys Lys Leu Ala Asp Val Met Val Leu
                   245                 250                 255
   Thr Val Phe Cys Leu Ser Val Phe Ala Leu Ile Gly Leu Gln Leu Phe
               260                 265                 270
   Met Gly Asn Leu Arg His Lys Cys Val Arg Asn Phe Thr Ala Leu Asn
           275                 280                 285
   Gly Thr Asn Gly Ser Val Glu Ala Asp Gly Leu Val Trp Glu Ser Leu
       290                 295                 300
   Asp Leu Tyr Leu Ser Asp Pro Glu Asn Tyr Leu Leu Lys Asn Gly Thr
   305                 310                 315                 320
   Ser Asp Val Leu Leu Cys Gly Asn Ser Ser Asp Ala Gly Thr Cys Pro
                   325                 330                 335
   Glu Gly Tyr Arg Cys Leu Lys Ala Gly Glu Asn Pro Asp His Gly Tyr
               340                 345                 350
   Thr Ser Phe Asp Ser Phe Ala Trp Ala Phe Leu Ala Leu Phe Arg Leu
   355 360 365
   Met Thr Gln Asp Cys Trp Glu Arg Leu Tyr Gln Gln Thr Leu Arg Ser
   370 375 380
   Ala Gly Lys Ile Tyr Met Ile Phe Phe Met Leu Val Ile Phe Leu Gly
   385 390 395 400
   Ser Phe Tyr Leu Val Asn Leu Ile Leu Ala Val Val Ala Met Ala Tyr
   405 410 415
   Glu Glu Gln Asn Gln Ala Thr Ile Ala Glu Thr Glu Glu Lys Glu Lys
   420 425 430
   Arg Phe Gln Glu Ala Met Glu Met Leu Lys Lys Glu His Glu Ala Leu
   435 440 445
   Thr Ile Arg Gly Val Asp Thr Val Ser Arg Ser Ser Leu Glu Met Ser
   450 455 460
   Pro Leu Ala Pro Val Asn Ser His Glu Arg Arg Ser Lys Arg Arg Lys
   465 470 475 480
   Arg Met Ser Ser Gly Thr Glu Glu Cys Gly Glu Asp Arg Leu Pro Lys
   485 490 495
   Ser Asp Ser Glu Asp Gly Pro Arg Ala Met Asn His Leu Ser Leu Thr
   500 505 510
   Arg Gly Leu Ser Arg Thr Ser Met Lys Pro Arg Ser Ser Arg Gly Ser
   515 520 525
   Ile Phe Thr Phe Arg Arg Arg Asp Leu Gly Ser Glu Ala Asp Phe Ala
   530 535 540
   Asp Asp Glu Asn Ser Thr Ala Arg Glu Ser Glu Ser His His Thr Ser
   545 550 555 560
   Leu Leu Val Pro Trp Pro Leu Arg Arg Thr Ser Ala Gln Gly Gln Pro
   565 570 575
   Ser Pro Gly Thr Ser Ala Pro Gly His Ala Leu His Gly Lys Lys Asn
   580 585 590
   Ser Thr Val Asp Cys Asn Gly Val Val Ser Leu Leu Gly Ala Gly Asp
   595 600 605
   Pro Glu Ala Thr Ser Pro Gly Ser His Leu Leu Arg Pro Val Met Leu
       610                 615                 620
   Glu His Pro Pro Asp Thr Thr Thr Pro Ser Glu Glu Pro Gly Gly Pro
   625                 630                 635                 640
   Gln Met Leu Thr Ser Gln Ala Pro Cys Val Asp Gly Phe Glu Glu Pro
                   645                 650                 655
   Gly Ala Arg Gln Arg Ala Leu Ser Ala Val Ser Val Leu Thr Ser Ala
               660                 665                 670
   Leu Glu Glu Leu Glu Glu Ser Arg His Lys Cys Pro Pro Cys Trp Asn
           675                 680                 685
   Arg Leu Ala Gln Arg Tyr Leu Ile Trp Glu Cys Cys Pro Leu Trp Met
       690                 695                 700
   Ser Ile Lys Gln Gly Val Lys Leu Val Val Met Asp Pro Phe Thr Asp
   705                 710                 715                 720
   Leu Thr Ile Thr Met Cys Ile Val Leu Asn Thr Leu Phe Met Ala Leu
                   725                 730                 735
   Glu His Tyr Asn Met Thr Ser Glu Phe Glu Glu Met Leu Gln Val Gly
               740                 745                 750
   Asn Leu Val Phe Thr Gly Ile Phe Thr Ala Glu Met Thr Phe Lys Ile
           755                 760                 765
   Ile Ala Leu Asp Pro Tyr Tyr Tyr Phe Gln Gln Gly Trp Asn Ile Phe
       770                 775                 780
   Asp Ser Ile Ile Val Ile Leu Ser Leu Met Glu Leu Gly Leu Ser Arg
   785                 790                 795                 800
   Met Ser Asn Leu Ser Val Leu Arg Ser Phe Arg Leu Leu Arg Val Phe
                   805                 810                 815
   Lys Leu Ala Lys Ser Trp Pro Thr Leu Asn Thr Leu Ile Lys Ile Ile
               820                 825                 830
   Gly Asn Ser Val Gly Ala Leu Gly Asn Leu Thr Leu Val Leu Ala Ile
           835                 840                 845
   Ile Val Phe Ile Phe Ala Val Val Gly Met Gln Leu Phe Gly Lys Asn
   850 855 860
   Tyr Ser Glu Leu Arg Asp Ser Asp Ser Gly Leu Leu Pro Arg Trp His
   865 870 875 880
   Met Met Asp Phe Phe His Ala Phe Leu Ile Ile Phe Arg Ile Leu Cys
   885 890 895
   Gly Glu Trp Ile Glu Thr Met Trp Asp Cys Met Glu Val Ser Gly Gln
   900 905 910
   Ser Leu Cys Leu Leu Val Phe Leu Leu Val Met Val Ile Gly Asn Leu
   915 920 925
   Val Val Leu Asn Leu Phe Leu Ala Leu Leu Leu Ser Ser Phe Ser Ala
   930 935 940
   Asp Asn Leu Thr Ala Pro Asp Glu Asp Arg Glu Met Asn Asn Leu Gln
   945 950 955 960
   Leu Ala Leu Ala Arg Ile Gln Arg Gly Leu Arg Phe Val Lys Arg Thr
   965 970 975
   Thr Trp Asp Phe Cys Cys Gly Leu Leu Arg His Arg Pro Gln Lys Pro
   980 985 990
   Ala Ala Leu Ala Ala Gln Gly Gln Leu Pro Ser Cys Ile Ala Thr Pro
   995 1000 1005
   Tyr Ser Pro Pro Pro Pro Glu Thr Glu Lys Val Pro Pro Thr Arg
   1010 1015 1020
   Lys Glu Thr Gln Phe Glu Glu Gly Glu Gln Pro Gly Gln Gly Thr
   1025 1030 1035
   Pro Gly Asp Pro Glu Pro Val Cys Val Pro Ile Ala Val Ala Glu
   1040 1045 1050
   Ser Asp Thr Asp Asp Gln Glu Glu Asp Glu Glu Asn Ser Leu Gly
   1055 1060 1065
   Thr Glu Glu Glu Ser Ser Lys Gln Gln Glu Ser Gln Pro Val Ser
   1070 1075 1080
   Gly Trp Pro Arg Gly Pro Pro Asp Ser Arg Thr Trp Ser Gln Val
   1085 1090 1095
   Ser Ala Thr Ala Ser Ser Glu Ala Glu Ala Ser Ala Ser Gln Ala
   1100 1105 1110
   Asp Trp Arg Gln Gln Trp Lys Ala Glu Pro Gln Ala Pro Gly Cys
   1115 1120 1125
   Gly Glu Thr Pro Glu Asp Ser Cys Ser Glu Gly Ser Thr Ala Asp
   1130 1135 1140
   Met Thr Asn Thr Ala Glu Leu Leu Glu Gln Ile Pro Asp Leu Gly
   1145 1150 1155
   Gln Asp Val Lys Asp Pro Glu Asp Cys Phe Thr Glu Gly Cys Val
   1160 1165 1170
   Arg Arg Cys Pro Cys Cys Ala Val Asp Thr Thr Gln Ala Pro Gly
   1175 1180 1185
   Lys Val Trp Trp Arg Leu Arg Lys Thr Cys Tyr His Ile Val Glu
   1190 1195 1200
   His Ser Trp Phe Glu Thr Phe Ile Ile Phe Met Ile Leu Leu Ser
   1205 1210 1215
   Ser Gly Ala Leu Ala Phe Glu Asp Ile Tyr Leu Glu Glu Arg Lys
   1220 1225 1230
   Thr Ile Lys Val Leu Leu Glu Tyr Ala Asp Lys Met Phe Thr Tyr
   1235 1240 1245
   Val Phe Val Leu Glu Met Leu Leu Lys Trp Val Ala Tyr Gly Phe
   1250 1255 1260
   Lys Lys Tyr Phe Thr Asn Ala Trp Cys Trp Leu Asp Phe Leu Ile
   1265 1270 1275
   Val Asp Val Ser Leu Val Ser Leu Val Ala Asn Thr Leu Gly Phe
   1280 1285 1290
   Ala Glu Met Gly Pro Ile Lys Ser Leu Arg Thr Leu Arg Ala Leu
   1295 1300 1305
   Arg Pro Leu Arg Ala Leu Ser Arg Phe Glu Gly Met Arg Val Val
   1310 1315 1320
   Val Asn Ala Leu Val Gly Ala Ile Pro Ser Ile Met Asn Val Leu
   1325 1330 1335
   Leu Val Cys Leu Ile Phe Trp Leu Ile Phe Ser Ile Met Gly Val
   1340 1345 1350
   Asn Leu Phe Ala Gly Lys Phe Gly Arg Cys Ile Asn Gln Thr Glu
   1355 1360 1365
   Gly Asp Leu Pro Leu Asn Tyr Thr Ile Val Asn Asn Lys Ser Gln
   1370 1375 1380
   Cys Glu Ser Leu Asn Leu Thr Gly Glu Leu Tyr Trp Thr Lys Val
   1385 1390 1395
   Lys Val Asn Phe Asp Asn Val Gly Ala Gly Tyr Leu Ala Leu Leu
   1400 1405 1410
   Gln Val Ala Thr Phe Lys Gly Trp Met Asp Ile Met Tyr Ala Ala
   1415 1420 1425
   Val Asp Ser Arg Gly Tyr Glu Glu Gln Pro Gln Trp Glu Tyr Asn
   1430 1435 1440
   Leu Tyr Met Tyr Ile Tyr Phe Val Ile Phe Ile Ile Phe Gly Ser
   1445 1450 1455
   Phe Phe Thr Leu Asn Leu Phe Ile Gly Val Ile Ile Asp Asn Phe
   1460 1465 1470
   Asn Gln Gln Lys Lys Lys Leu Gly Gly Gln Asp Ile Phe Met Thr
   1475 1480 1485
   Glu Glu Gln Lys Lys Tyr Tyr Asn Ala Met Lys Lys Leu Gly Ser
   1490 1495 1500
   Lys Lys Pro Gln Lys Pro Ile Pro Arg Pro Leu Asn Lys Tyr Gln
   1505 1510 1515
   Gly Phe Ile Phe Asp Ile Val Thr Lys Gln Ala Phe Asp Val Thr
   1520 1525 1530
   Ile Met Phe Leu Ile Cys Leu Asn Met Val Thr Met Met Val Glu
   1535 1540 1545
   Thr Asp Asp Gln Ser Pro Glu Lys Ile Asn Ile Leu Ala Lys Ile
   1550 1555 1560
   Asn Leu Leu Phe Val Ala Ile Phe Thr Gly Glu Cys Ile Val Lys
   1565 1570 1575
   Leu Ala Ala Leu Arg His Tyr Tyr Phe Thr Asn Ser Trp Asn Ile
   1580 1585 1590
   Phe Asp Phe Val Val Val Ile Leu Ser Ile Val Gly Thr Val Leu
   1595 1600 1605
   Ser Asp Ile Ile Gln Lys Tyr Phe Phe Ser Pro Thr Leu Phe Arg
   1610 1615 1620
   Val Ile Arg Leu Ala Arg Ile Gly Arg Ile Leu Arg Leu Ile Arg
   1625 1630 1635
   Gly Ala Lys Gly Ile Arg Thr Leu Leu Phe Ala Leu Met Met Ser
   1640 1645 1650
   Leu Pro Ala Leu Phe Asn Ile Gly Leu Leu Leu Phe Leu Val Met
   1655 1660 1665
   Phe Ile Tyr Ser Ile Phe Gly Met Ala Asn Phe Ala Tyr Val Lys
   1670 1675 1680
   Trp Glu Ala Gly Ile Asp Asp Met Phe Asn Phe Gln Thr Phe Ala
   1685 1690 1695
   Asn Ser Met Leu Cys Leu Phe Gln Ile Thr Thr Ser Ala Gly Trp
   1700 1705 1710
   Asp Gly Leu Leu Ser Pro Ile Leu Asn Thr Gly Pro Pro Tyr Cys
   1715 1720 1725
   Asp Pro Thr Leu Pro Asn Ser Asn Gly Ser Arg Gly Asp Cys Gly
   1730 1735 1740
   Ser Pro Ala Val Gly Ile Leu Phe Phe Thr Thr Tyr Ile Ile Ile
   1745 1750 1755
   Ser Phe Leu Ile Val Val Asn Met Tyr Ile Ala Ile Ile Leu Glu
   1760 1765 1770
   Asn Phe Ser Val Ala Thr Glu Glu Ser Thr Glu Pro Leu Ser Glu
       1775                    1780                1785
   Asp Asp Phe Asp Met Phe Tyr Glu Ile Trp Glu Lys Phe Asp Pro
       1790                    1795                1800
   Glu Ala Thr Gln Phe Ile Glu Tyr Ser Val Leu Ser Asp Phe Ala
       1805                    1810                1815
   Asp Ala Leu Ser Glu Pro Leu Arg Ile Ala Lys Pro Asn Gln Ile
       1820                    1825                1830
   Ser Leu Ile Asn Met Asp Leu Pro Met Val Ser Gly Asp Arg Ile
       1835                    1840                1845
   His Cys Met Asp Ile Leu Phe Ala Phe Thr Lys Arg Val Leu Gly
       1850                    1855                1860
   Glu Ser Gly Glu Met Asp Ala Leu Lys Ile Gln Met Glu Glu Lys
       1865                    1870                1875
   Phe Met Ala Ala Asn Pro Ser Lys Ile Ser Tyr Glu Pro Ile Thr
       1880                    1885                1890
   Thr Thr Leu Arg Arg Lys His Glu Glu Val Ser Ala Met Val Ile
       1895                    1900                1905
   Gln Arg Ala Phe Arg Arg His Leu Leu Gln Arg Ser Leu Lys His
       1910                    1915                1920
   Ala Ser Phe Leu Phe Arg Gln Gln Ala Gly Ser Gly Leu Ser Glu
       1925                    1930                1935
   Glu Asp Ala Pro Glu Arg Glu Gly Leu Ile Ala Tyr Val Met Ser
       1940                    1945                1950
   Glu Asn Phe Ser Arg Pro Leu Gly Pro Pro Ser Ser Ser Ser Ile
       1955                    1960                1965
   Ser Ser Thr Ser Phe Pro Pro Ser Tyr Asp Ser Val Thr Arg Ala
       1970                    1975                1980
   Thr Ser Asp Asn Leu Gln Val Arg Gly Ser Asp Tyr Ser His Ser
       1985                    1990                1995
   Glu Asp Leu Ala Asp Phe Pro Pro Ser Pro Asp Arg Asp Arg Glu
       2000                2005                2010
   Ser Ile Val
       2015
<210> 7
   <211> 405
   <212> DNA
   <213> Homo sapiens
<220>
   <221> CDS
   <222> (1)..(390)
   <223> KCNE1 cDNA (GenBank Acc. N° NM000219): nucleotides 626 to 1015
<400> 7
atg atc ctg tct aac acc aca gcg gtg acg ccc ttt ctg acc aag ctg
   48
   tgg cag gag aca gtt cag cag ggt ggc aac atg tcg ggc ctg gcc cgc
   96
   agg tcc ccc cgc agc agt gac ggc aag ctg gag gcc ctc tac gtc ctc
   144
   atg gta ctg gga ttc ttc ggc ttc ttc acc ctg ggc atc atg ctg agc
   192
   tac atc cgc tcc aag aag ctg gag cac tcg aac gac cca ttc aac gtc
   240
   tac atc gag tcc gat gcc tgg caa gag aag gac aag gcc tat gtc cag
   288
   gcc cgg gtc ctg gag agc tac agg tcg tgc tat gtc gtt gaa aac cat
   336
   ctg gcc ata gaa caa ccc aac aca cac ctt cct gag acg aag cct tcc
   384
   cca tga accccaccac tggct
   405
<210> 8
   <211> 129
   <212> PRT
   <213> Homo sapiens
<400> 8
<210> 9
   <211> 372
   <212> DNA
   <213> Homo sapiens
<220>
   <221> CDS
   <222> (1)..(372)
   <223> KCNE2 cDNA (GenBank Acc. N° NM000335): nucleotides 509 to 880
<400> 9
atg tct act tta tcc aat ttc aca cag acg ctg gaa gac gtc ttc cga
   48
   agg att ttt att act tat atg gac aat tgg cgc cag aac aca aca gct
   96
   gag caa gag gcc ctc caa gcc aaa gtt gat gct gag aac ttc tac tat
   144
   gtc atc ctg tac ctc atg gtg atg att gga atg ttc tct ttc atc atc
   192
   gtg gcc atc ctg gtg agc act gtg aaa tcc aag aga cgg gaa cac tcc
   240
   aat gac ccc tac cac cag tac att gta gag gac tgg cag gaa aag tac
   288
   aag agc caa atc ttg aat cta gaa gaa tcg aag gcc acc atc cat gag
   336
   aac att ggt gcg gct ggg ttc aaa atg tcc ccc tga
   372

<210> 10
   <211> 123
   <212> PRT
   <213> Homo sapiens
<400> 10
<210> 11
   <211> 9
   <212> DNA
   <213> Homo sapiens
<220>
   <221> mutation
   <222> (5)..(5)
   <223> KCNQ1; G136A
<400> 11
   cccgacggg
   9
<210> 12
   <211> 9
   <212> DNA
   <213> Homo sapiens
<220>
   <221> mutation
   <222> (6)..(6)
   <223> KCNQ1; 151-152insT
<400> 12
   cgctcttac
   9
<210> 13
   <211> 9
   <212> DNA
   <213> Homo sapiens
<220>
<221> mutation
   <222> (5)..(5)
   <223> KCNQ1; C409T
<400> 13
   ctgcttcat
   9
<210> 14
   <211> 9
   <212> DNA
   <213> Homo sapiens
<220>
   <221> mutation
   <222> (5)..(5)
   <223> KCNQ1; G436A
<400> 14
   catcaagca
   9
<210> 15
   <211> 9
   <212> DNA
   <213> Homo sapiens
<220>
   <221> mutation
   <222> (5)..(5)
   <223> KCNQ1; T518A
<400> 15
   gtggaccgc
   9
<210> 16
   <211> 9
   <212> DNA
   <213> Homo sapiens
<220>
   <221> mutation
   <222> (5)..(5)
   <223> KCNQ1; G521C
<400> 16
   gtccccctc
   9
<210> 17
   <211> 9
   <212> DNA
   <213> Homo sapiens
<220>
   <221> mutation
   <222> (5)..(5)
   <223> KCNQ1; C568T
<400> 17
   ggggtggct
   9
<210> 18
   <211> 9
   <212> DNA
   <213> Homo sapiens
<220>
   <221> mutation
   <222> (5)..(5)
   <223> KCNQ1; G575C
<400> 18
   ctgcccttt
   9
<210> 19
   <211> 9
   <212> DNA
   <213> Homo sapiens
<220>
   <221> misc_feature
   <223> KCNQ1; 584del
<400> 19
   gcccgaagc
   9
<210> 20
   <211> 9
   <212> DNA
   <213> Homo sapiens
<220>
   <221> mutation
   <222> (5)..(5)
   <223> KCNQ1; G604C
<400> 20
   catccgtga
   9
<210> 21
   <211> 9
   <212> DNA
   <213> Homo sapiens
<220>
   <221> mutation
   <222> (5)..(5)
   <223> KCNQ1; C612G
<400> 21
   tcatggtgg
   9
<210> 22
   <211> 9
   <212> DNA
   <213> Homo sapiens
<220>
   <221> mutation
   <222> (5)..(5)
   <223> KCNQ1; C626T
<400> 22
   gccttcatg
   9
<210> 23
   <211> 9
   <212> DNA
   <213> Homo sapiens
<220>
   <221> mutation
   <222> (5)..(5)
   <223> KCNQ1; G643A
<400> 23
   ctgcatggg
   9
<210> 24
   <211> 9
   <212> DNA
   <213> Homo sapiens
<220>
   <221> mutation
   <222> (5)..(5)
   <223> KCNQ1; G692A
<400> 24
   atccacttc
   9
<210> 25
   <211> 9
   <212> DNA
   <213> Homo sapiens
<220>
   <221> mutation
   <222> (5)..(5)
   <223> KCNQ1; T716C
<400> 25
   atgccacac
   9
<210> 26
   <211> 9
   <212> DNA
   <213> Homo sapiens
<220>
   <221> mutation
   <222> (5)..(5)
   <223> KCNQ1; G760T
<400> 26
   ctccttggt
   9
<210> 27
   <211> 9
   <212> DNA
   <213> Homo sapiens
<220>
   <221> mutation
   <222> (5)..(5)
   <223> KCNQ1; C772A
<400> 27
   catcaaccg
   9
<210> 28
   <211> 9
   <212> DNA
   <213> Homo sapiens
<220>
   <221> mutation
   <222> (5)..(5)
   <223> KCNQ1; A773G
<400> 28
   atccgccgc
   9
<210> 29
   <211> 10
   <212> DNA
   <213> Homo sapiens
<220>
   <221> mutation
   <222> (6)..(6)
   <223> KCNQ1; C784G
<400> 29
   aggaggtgat
   10
<210> 30
   <211> 9
   <212> DNA
   <213> Homo sapiens
<220>
   <221> misc_feature
   <223> KCNQ1; 796del
<400> 30
   cacctgtac
   9
<210> 31
   <211> 9
   <212> DNA
   <213> Homo sapiens
<220>
<221> mutation
   <222> (5)..(5)
   <223> KCNQ1; G815A
<400> 31
   ctggacctc
   9
<210> 32
   <211> 9
   <212> DNA
   <213> Homo sapiens
<220>
   <221> misc_feature
   <223> KCNQ1; 828-830del
<400> 32
   tctcgtact
   9
<210> 33
   <211> 9
   <212> DNA
   <213> Homo sapiens
<220>
   <221> mutation
   <222> (5)..(5)
   <223> KCNQ1; C830G
<400> 33
   tcctggtac
   9
<210> 34
   <211> 9
   <212> DNA
   <213> Homo sapiens
<220>
   <221> mutation
   <222> (5)..(5)
   <223> KCNQ1; T839A
<400> 34
   tttgagtac
   9
<210> 35
   <211> 9
   <212> DNA
   <213> Homo sapiens
<220>
   <221> mutation
   <222> (5)..(5)
   <223> KCNQ1; C860A
<400> 35
   gacgaggtg
   9
<210> 36
   <211> 9
   <212> DNA
   <213> Homo sapiens
<220>
   <221> mutation
   <222> (5)..(5)
   <223> KCNQ1; G904A
<400> 36
   agatacgct
   9
<210> 37
   <211> 9
   <212> DNA
   <213> Homo sapiens
<220>
   <221> mutation
   <222> (5)..(5)
   <223> KCNQ1; T923A
<400> 37
   caggacaca
   9
<210> 38
   <211> 9
   <212> DNA
   <213> Homo sapiens
<220>
   <221> mutation
   <222> (5)..(5)
   <223> KCNQ1; G947A
<400> 38
   tatgaggac
   9
<210> 39
   <211> 9
   <212> DNA
   <213> Homo sapiens
<220>
   <221> mutation
   <222> (5)..(5)
   <223> KCNQ1; C965T
<400> 39
   cagatgtgg
   9
<210> 40
   <211> 9
   <212> DNA
   <213> Homo sapiens
<220>
   <221> mutation
   <222> (5)..(5)
   <223> KCNQ1; C1028T
<400> 40
   ctcctagcg
   9
<210> 41
   <211> 9
   <212> DNA
   <213> Homo sapiens
<220>
   <221> mutation
   <222> (5)..(5)
   <223> KCNQ1; C1028G
<400> 41
   ctccgagcg
   9
<210> 42
   <211> 9
   <212> DNA
   <213> Homo sapiens
<220>
   <221> mutation
   <222> (5)..(5)
   <223> KCNQ1; T1045C
<400> 42
   tggcccggg
   9
<210> 43
   <211> 9
   <212> DNA
   <213> Homo sapiens
<220>
   <221> mutation
   <222> (5)..(5)
   <223> KCNQ1; G1048C
<400> 43
   ctcgcggtt
   9
<210> 44
   <211> 9
   <212> DNA
   <213> Homo sapiens
<220>
   <221> mutation
   <222> (5)..(5)
   <223> KCNQ1; T1052C
<400> 44
   gggtctgcc
   9
<210> 45
   <211> 9
   <212> DNA
   <213> Homo sapiens
<220>
   <221> misc_feature
   <223> KCNQ1; 1067-1072del
<400> 45
   gtgcagcag
   9
<210> 46
   <211> 9
   <212> DNA
   <213> Homo sapiens
<220>
   <221> mutation
   <222> (5)..(5)
   <223> KCNQ1; G1079C
<400> 46
   cagacgcag
   9
<210> 47
   <211> 9
   <212> DNA
   <213> Homo sapiens
<220>
   <221> mutation
   <222> (5)..(5)
   <223> KCNQ1; C1115A
<400> 47
   gcagactca
   9
<210> 48
   <211> 9
   <212> DNA
   <213> Homo sapiens
<220>
   <221> mutation
   <222> (5)..(5)
   <223> KCNQ1; A1178T
<400> 48
   tggatgatc
   9
<210> 49
   <211> 9
   <212> DNA
   <213> Homo sapiens
<220>
   <221> mutation
   <222> (5)..(5)
   <223> KCNQ1; 1291-1292insG
<400> 49
   gggggtgac
   9
<210> 50
   <211> 9
   <212> DNA
   <213> Homo sapiens
<220>
   <221> mutation
   <222> (5)..(5)
   <223> KCCNQ1; 1292-1293insG
<400> 50
   gggtggact
   9
<210> 51
   <211> 9
   <212> DNA
   <213> Homo sapiens
<220>
   <221> misc_feature
   <223> KCNQ1; 1486-1487del
<400> 51
   agactgctg
   9
<210> 52
   <211> 9
   <212> DNA
   <213> Homo sapiens
<220>
   <221> mutation
   <222> (5)..(5)
   <223> KCNQ1; 1514+1 G>A
<400> 52
   cacaatgag
   9
<210> 53
   <211> 9
   <212> DNA
   <213> Homo sapiens
<220>
   <221> misc_feature
   <223> KCNQ1; 1513-1514del
<400> 53
   ctcagtgag
   9
<210> 54
   <211> 9
   <212> DNA
   <213> Homo sapiens
<220>
   <221> misc_feature
   <223> KCNQ1, 1538delC
<400> 54
   ggccacatt
   9
<210> 55
   <211> 9
   <212> DNA
   <213> Homo sapiens
<220>
   <221> mutation
   <222> (5)..(5)
   <223> KCNQ1; C1552G
<400> 55
   cattggacg
   9
<210> 56
   <211> 9
   <212> DNA
   <213> Homo sapiens
<220>
   <221> mutation
   <222> (5)..(5)
   <223> KCNQ1; G1553C
<400> 56
   attccacgc
   9
<210> 57
   <211> 9
   <212> DNA
   <213> Homo sapiens
<220>
   <221> mutation
   <222> (5)..(5)
   <223> KCNQ1; G1643A
<400> 57
   caggaccac
   9
<210> 58
   <211> 9
   <212> DNA
   <213> Homo sapiens
<220>
   <221> mutation
   <222> (5)..(5)
   <223> KCNQ1; T1661C
<400> 58
   atggcgcgc
   9
<210> 59
   <211> 9
   <212> DNA
   <213> Homo sapiens
<220>
   <221> mutation
   <222> (5)..(5)
   <223> KCNQ1, T1700C
<400> 59
   tccactggg
   9
<210> 60
   <211> 9
   <212> DNA
   <213> Homo sapiens
<220>
   <221> misc_feature
   <223> KCNQ1; 1710delC
<400> 60
   aagcctcac
   9
<210> 61
   <211> 9
   <212> DNA
   <213> Homo sapiens
<220>
   <221> mutation
   <222> (5)..(5)
   <223> KCNQ1; C1719A
<400> 61
   tgttaatct
   9
<210> 62
   <211> 9
   <212> DNA
   <213> Homo sapiens
<220>
   <221> misc_feature
   <223> KCNQ1; 1725-1728del
<400> 62
   atctctcag
   9
<210> 63
   <211> 9
   <212> DNA
   <213> Homo sapiens
<220>
   <221> mutation
   <222> (5)..(5)
   <223> KCNQ1; G1748A
<400> 63
   gatcacggc
   9
<210> 64
   <211> 9
   <212> DNA
   <213> Homo sapiens
<220>
   <221> mutation
   <222> (5)..(5)
   <223> KCNQ1; A1756G
<400> 64
   cagcgacac
   9
<210> 65
   <211> 9
   <212> DNA
   <213> Homo sapiens
<220>
   <221> misc_feature
   <223> KCNQ1; 1893delC
<400> 65
   cccccagag
   9
<210> 66
   <211> 9
   <212> DNA
   <213> Homo sapiens
<220>
   <221> misc_feature
   <223> KCNQ1; 1909delC
<400> 66
   gggccacat
   9
<210> 67
   <211> 9
   <212> DNA
   <213> Homo sapiens
<220>
   <221> mutation
   <222> (5)..(5)
   <223> KCNH2; A128G
<400> 67
   atctgctgc
   9
<210> 68
   <211> 9
   <212> DNA
   <213> Homo sapiens
<220>
   <221> mutation
   <222> (5)..(5)
   <223> KCNH2; G146A
<400> 68
   ttctgcgag
   9
<210> 69
   <211> 9
   <212> DNA
   <213> Homo sapiens
<220>
   <221> mutation
   <222> (5)..(5)
   <223> KCNH2; A173C
<400> 69
   gccgcggtg
   9
<210> 70
   <211> 9
   <212> DNA
   <213> Homo sapiens
<220>
   <221> mutation
   <222> (5)..(5)
   <223> KCNH2; A173G
<400> 70
   gccggggtg
   9
<210> 71
   <211> 9
   <212> DNA
   <213> Homo sapiens
<220>
   <221> mutation
   <222> (5)..(5)
   <223> KCNH2; G174C
<400> 71
   ccgacgtga
   9
<210> 72
   <211> 9
   <212> DNA
   <213> Homo sapiens
<220>
   <221> mutation
   <222> (5)..(5)
   <223> KCNH2; T202C
<400> 72
   cgacctcct
   9
<210> 73
   <211> 10
   <212> DNA
   <213> Homo sapiens
<220>
   <221> misc_feature
   <223> KCNH2; G211C
<400> 73
   gcacgggccg
   10
<210> 74
   <211> 9
   <212> DNA
   <213> Homo sapiens
<220>
   <221> mutation
   <222> (5)..(5)
   <223> KCNH2; C221T
<400> 74
   cgcacgcag
   9
<210> 75
   <211> 8
   <212> DNA
   <213> Homo sapiens
<220>
   <221> misc_feature
   <223> KCNH2; 244-252del
<400> 75
   gcaggcac
   8
<210> 76
   <211> 9
   <212> DNA
   <213> Homo sapiens
<220>
   <221> mutation
   <222> (5)..(5)
   <223> KCNH2; 308-310ins ATG
<400> 76
   gatgatggt
   9
<210> 77
   <211> 9
   <212> DNA
   <213> Homo sapiens
<220>
   <221> misc_feature
   <223> KCNH2, 337-339del
<400> 77
   tgtgcccgt
   9
<210> 78
   <211> 10
   <212> DNA
   <213> Homo sapiens
<220>
   <221> mutation
   <222> (4)..(8)
   <223> KCNH2; 557-566del/Ins+TTCGC
<400> 78
   cggttcgccg
   10
<210> 79
   <211> 8
   <212> DNA
   <213> Homo sapiens
<220>
   <221> misc_feature
   <223> KCNH2; 576delG
<400> 79
   ccggggcc
   8
<210> 80
   <211> 9
   <212> DNA
   <213> Homo sapiens
<220>
   <221> misc_feature
   <223> KCNH2; 578-582del
<400> 80
   gggggtggt
   9
<210> 81
   <211> 9
   <212> DNA
   <213> Homo sapiens
<220>
   <221> mutation
   <222> (3)..(4)
   <223> KCNH2; 735-6InsCC
<400> 81
   gcccccggc
   9
<210> 82
   <211> 9
   <212> DNA
   <213> Homo sapiens
<220>
   <221> mutation
   <222> (5)..(5)
   <223> KCNH2; C751T
<400> 82
   atcgtcccg
   9

<210> 83
   <211> 8
   <212> DNA
   <213> Homo sapiens
<220>
   <221> mutation
   <222> (5)..(5)
   <223> KCNH2; C1171T
<400> 83
   gctgtagg
   8
<210> 84
   <211> 10
   <212> DNA
   <213> Homo sapiens
<220>
   <221> mutation
   <222> (6)..(6)
   <223> KCNH2; G1229C
<400> 84
   cgtgtcggac
   10
<210> 85
   <211> 10
   <212> DNA
   <213> Homo sapiens
<220>
   <221> mutation,
   <222> (7)..(7)
   <223> KCNH2; G1235A
<400> 85
   ggactgactc
   10
<210> 86
   <211> 10
   <212> DNA
   <213> Homo sapiens
<220>
   <221> mutation
   <222> (5)..(5)
   <223> KCNH2; C1277A
<400> 86
   cacacactac
   10
<210> 87
   <211> 10
   <212> DNA
   <213> Homo sapiens
<220>
   <221> mutation
   <222> (5)..(5)
   <223> KCNH2; T1279C
<400> 87
   accccactcg
   10
<210> 88
   <211> 10
   <212> DNA
   <213> Homo sapiens
<220>
   <221> mutation
   <222> (6)..(6)
   <223> KCNH2; C1283T
<400> 88
   ctacttggct
   10
<210> 89
   <211> 10
   <212> DNA
   <213> Homo sapiens
<220>
   <221> mutation
   <222> (5)..(5)
   <223> KCNH2; G1378T
<400> 89
   cgtgtacatc
   10
<210> 90
   <211> 10
   <212> DNA
   <213> Homo sapiens
<220>
   <221> mutation
   <222> (5)..(5)
   <223> KCNH2; G1501C
<400> 90
   catccacatg
   10
<210> 91
   <211> 10
   <212> DNA
   <213> Homo sapiens
<220>
   <221> mutation
   <222> (5)..(5)
   <223> KCNH2, G1601T
<400> 91
   ggtgctcgtg
   10
<210> 92
   <211> 9
   <212> DNA
   <213> Homo sapiens
<220>
   <221> misc_feature
   <223> KCNH2; 1613-1619del
<400> 92
   cggatcgct
   9
<210> 93
   <211> 9
   <212> DNA
   <213> Homo sapiens
<220>
   <221> mutation
   <222> (5)..(5)
   <223> KCNH2; G1697C
<400> 93
   gcctccatc
   9
<210> 94
   <211> 9
   <212> DNA
   <213> Homo sapiens
<220>
   <221> misc_feature
   <223> KCNH2; 1701delC
<400> 94
   tgcattggt
   9
<210> 95
   <211> 10
   <212> DNA
   <213> Homo sapiens
<220>
   <221> mutation
   <222> (5)..(5)
   <223> KCNH2; T1702C
<400> 95
   catccggtac
   10
<210> 96
   <211> 9
   <212> DNA
   <213> Homo sapiens
<220>
   <221> mutation
   <222> (5)..(5)
   <223> KCNH2; A1711G
<400> 96
   cgccgtcgc
   9
<210> 97
   <211> 9
   <212> DNA
   <213> Homo sapiens
<220>
   <221> mutation
   <222> (6)..(6)
   <223> KCNH2; G1715A
<400> 97
   catcgacaa
   9
<210> 98
   <211> 9
   <212> DNA
   <213> Homo sapiens
<220>
   <221> mutation
   <222> (5)..(5)
   <223> KCNH2; G1745T
<400> 98
   tcactcatc
   9
<210> 99
   <211> 9
   <212> DNA
   <213> Homo sapiens
<220>
   <221> mutation
   <222> (4)..(4)
   <223> KCNH2; G1825C
<400> 99
   aagcacaag
   9
<210> 100
   <211> 9
   <212> DNA
   <213> Homo sapiens
<220>
   <221> mutation
   <222> (4)..(4)
   <223> KCNH2; C1843T
<400> 100
   gcgttctac
   9
<210> 101
   <211> 9
   <212> DNA
   <213> Homo sapiens
<220>
   <221> mutation
   <222> (5)..(5)
   <223> KCNH2; C1863G
<400> 101
   gcaggctca
   9
<210> 102
   <211> 9
   <212> DNA
   <213> Homo sapiens
<220>
   <221> mutation
   <222> (5)..(5)
   <223> KCNH2; G1877C
<400> 102
   cccgccagc
   9
<210> 103
   <211> 9
   <212> DNA
   <213> Homo sapiens
<220>
   <221> mutation
   <222> (6)..(6)
   <223> KCNH2; G1911C
<400> 103
   tcagacaag
   9
<210> 104
   <211> 9
   <212> DNA
   <213> Homo sapiens
<220>
   <221> mutation
   <222> (5)..(5)
   <223> KCNH2; G1930T
<400> 104
   ctgcttcat
   9
<210> 105
   <211> 9
   <212> DNA
   <213> Homo sapiens
<220>
   <221> mutation
   <222> (5)..(5)
   <223> KCNH2; T1967G
<400> 105
   atctgcggc
   9
<210> 106
   <211> 9
   <212> DNA
   <213> Homo sapiens
<220>
   <221> mutation
   <222> (4)..(4)
   <223> KCNH2; C1979T
<400> 106
   tgttggcca
   9
<210> 107
   <211> 9
   <212> DNA
   <213> Homo sapiens
<220>
   <221> mutation
   <222> (5)..(5)
   <223> KCNH2; G2087C
<400> 107
   cagcccctc
   9
<210> 108
   <211> 9
   <212> DNA
   <213> Homo sapiens
<220>
   <221> misc_feature
   <223> KCNH2; 2164-2181dup
<400> 108
   tgacgagtg
   9
<210> 109
   <211> 9
   <212> DNA
   <213> Homo sapiens
<220>
   <221> misc_feature
   <223> KCNH2; 2231delG
<400> 109
   cttccaggg
   9
<210> 110
   <211> 9
   <212> DNA
   <213> Homo sapiens
<220>
   <221> mutation
   <222> (5)..(5)
   <223> KCNH5; G2398+3A>G
<400> 110
   gggtgtggg
   9
<210> 111
   <211> 9
   <212> DNA
   <213> Homo sapiens
<220>
   <221> mutation
   <222> (5)..(5)
   <223> KCNH2; G2398+3A>T
<400> 111
   gggtttggg
   9
<210> 112
   <211> 9
   <212> DNA
   <213> Homo sapiens
<220>
   <221> mutation
   <222> (5)..(5)
   <223> KCNH2; G2398T
<400> 112
   cctgtgtat
   9
<210> 113
   <211> 9
   <212> DNA
   <213> Homo sapiens
<220>
   <221> mutation
   <222> (5)..(5)
   <223> KCNH2; T2452C
<400> 113
   aagccgaac
   9
<210> 114
   <211> 9
   <212> DNA
   <213> Homo sapiens
<220>
   <221> mutation
   <222> (5)..(5)
   <223> KCNH2; A2494T
<400> 114
   tggctagtc
   9
<210> 115
   <211> 9
   <212> DNA
   <213> Homo sapiens
<220>
   <221> mutation
   <222> (4)..(4)
   <223> KCNH2; A2581C
<400> 115
   ttccacctg
   9
<210> 116
   <211> 9
   <212> DNA
   <213> Homo sapiens
<220>
   <221> misc_feature
   <223> KCNH2; 2638-2648del
<400> 116
   gggtgcaac
   9
<210> 117
   <211> 9
   <212> DNA
   <213> Homo sapiens
<220>
   <221> misc_feature
   <223> KCNH2; 2676-2682del
<400> 117
   tccgacgga
   9
<210> 118
   <211> 9
   <212> DNA
   <213> Homo sapiens
<220>
   <221> misc_feature
   <223> KCNH2; 2732-2766del
<400> 118
   ccggccggg
   9
<210> 119
   <211> 9
   <212> DNA
   <213> Homo sapiens
<220>
   <221> mise feature
   <223> KCNH2; 2738-2739insCGGGC
<400> 119
   ggcgggccg
   9
<210> 120
   <211> 9
   <212> DNA
   <213> Homo sapiens
<220>
   <221> misc_feature
   <223> KCNH2; 2775delG
<400> 120
   ggggccgtg
   9
<210> 121
   <211> 9
   <212> DNA
   <213> Homo sapiens
<220>
   <221> mutation
   <222> (5)..(5)
   <223> KCNH2; G2781A
<400> 121
   cgtgagggg
   9
<210> 122
   <211> 9
   <212> DNA
   <213> Homo sapiens
<220>
   <221> misc_feature
   <223> KCNH2; 2895-2905del
<400> 122
   cccgggtgg
   9
<210> 123
   <211> 9
   <212> DNA
   <213> Homo sapiens
<220>
   <221> mutation
   <222> (5)..(5)
   <223> KCNH2; C2903T
<400> 123
   ccgctgggt
   9
<210> 124
   <211> 11
   <212> DNA
   <213> Homo sapiens
<220>
   <221> mutation
   <222> (6)..(6)
   <223> KCNH2; C3045A
<400> 124
   cgatgacccg c
   11
<210> 125
   <211> 9
   <212> DNA
   <213> Homo sapiens
<220>
   <221> misc_feature
   <223> KCNH2; 3093-3106del
<400> 125
   cccggggcg
   9
<210> 126
   <211> 9
   <212> DNA
   <213> Homo sapiens
<220>
   <221> mutation
   <222> (4)..(8)
   <223> KCNH2; 3093-3099del/insTTCGC
<400> 126
   gggttcgcc
   9
<210> 127
   <211> 9
   <212> DNA
   <213> Homo sapiens
<220>
   <221> misc_feature
   <223> KCNH2; 3099delG
<400> 127
   ggcgccccg
   9
<210> 128
   <211> 9
   <212> DNA
   <213> Homo sapiens
<220>
   <221> misc_feature
   <223> KCNH2; 3100delC
<400> 128
   gcggcccgg
   9
<210> 129
   <211> 9
   <212> DNA
   <213> Homo sapiens
<220>
   <221> misc_feature
   <223> KCNH2; 3154delC
<400> 129
   caggtggag
   9
<210> 130
   <211> 9
   <212> DNA
   <213> Homo sapiens
<220>
   <221> mutation
   <222> (4)..(4)
   <223> KCNH2; 3304InsC
<400> 130
   ccccaccct
   9
<210> 131
   <211> 9
   <212> DNA
   <213> Homo sapiens
<220>
   <221> misc_feature
   <223> KCNH2; 3397-3398del
<400> 131
   cccacgacg
   9
<210> 132
   <211> 9
   <212> DNA
   <213> Homo sapiens
<220>
   <221> mutation
   <222> (4)..(4)
   <223> KCNH2; C3457T
<400> 132
   ctgtacaga
   9
<210> 133
   <211> 10
   <212> DNA
   <213> Homo sapiens
<220>
   <221> mutation
   <222> (6)..(6)
   <223> SNC5A; C1238A
<400> 133
   ggtcgaaatg
   10
<210> 134
   <211> 9
   <212> DNA
   <213> Homo sapiens
<220>
   <221> mutation
   <222> (5)..(5)
   <223> SNC5A; G1237A
<400> 134
   ggtcacaat
   9
<210> 135
   <211> 9
   <212> DNA
   <213> Homo sapiens
<220>
   <221> mutation
   <222> (5)..(5)
   <223> KCNH2; C1717G
<400> 135
   tgccgaggg
   9
<210> 136
   <211> 9
   <212> DNA
   <213> Homo sapiens
<220>
   <221> mutation
   <222> (5)..(5)
   <223> SNC5A; G1735A
<400> 136
   tcccagaac
   9
<210> 137
   <211> 9
   <212> DNA
   <213> Homo sapiens
<220>
   <221> mutation
   <222> (5)..(5)
   <223> SNC5A; G2066A
<400> 137
   aaccatctc
   9
<210> 138
   <211> 10
   <212> DNA
   <213> Homo sapiens
<220>
   <221> mutation
   <222> (6)..(6)
   <223> SNC5A; T4493C
<400> 138
   tgccacgaag
   10
<210> 139
   <211> 11
   <212> DNA
   <213> Homo sapiens
<220>
   <221> mutation
   <222> (7)..(7)
   <223> SNC5A; G4877C
<400> 139
   tcttcccagt c
   11
<210> 140
   <211> 10
   <212> DNA
   <213> Homo sapiens
<220>
   <221> mutation
   <222> (5)..(5)
   <223> SNC5A; C4930T
<400> 140
   gatctgcacg
   10
<210> 141
   <211> 9
   <212> DNA
   <213> Homo sapiens
<220>
   <221> mutation
   <222> (5)..(5)
   <223> SNC5A; A4978G
<400> 141
   caacgtcgg
   9
<210> 142
   <211> 9
   <212> DNA
   <213> Homo sapiens
<220>
   <221> mutation
   <222> (5)..(5)
   <223> SNC5A; A5300G
<400> 142
   atgtgcatt
   9
<210> 143
   <211> 9
   <212> DNA
   <213> Homo sapiens
<220>
   <221> mutation
   <222> (5)..(5)
   <223> SNC5A; G5360A
<400> 143
   caccaagcc
   9
<210> 144
   <211> 9
   <212> DNA
   <213> Homo sapiens
<220>
   <221> mutation
   <222> (5)..(5)
   <223> SNC5A; A5369G
<400> 144
   gagggcgac
   9
<210> 145
   <211> 9
   <212> DNA
   <213> Homo sapiens
<220>
   <221> mutation
   <222> (5)..(5)
   <223> SNC5A; G5738A
<400> 145
   ttccacagg
   9
<210> 146
   <211> 9
   <212> DNA
   <213> Homo sapiens
<220>
   <221> mutation
   <222> (5)..(5)
   <223> KCNE1; G107A
<400> 146
   ccccacagc
   9
<210> 147
   <211> 9
   <212> DNA
   <213> Homo sapiens
<220>
   <221> mutation
   <222> (5)..(5)
   <223> KCNE2; T158G
<400> 147
   ggatgcttc
   9
<210> 148
   <211> 9
   <212> DNA
   <213> Homo sapiens
<220>
   <221> mutation
   <222> (4)..(4)
   <223> KCNE2; A166G
<220>
   <221> mutation
   <222> (7)..(9)
   <223> KCNE2; 169InsATG
<400> 148
   gtggtgatg
   9
<210> 149
   <211> 9
   <212> DNA
   <213> Homo sapiens
<220>
   <221> misc_feature
   <223> KCNE2; 156-161del
<400> 149
   ctgtaggtg
   9
<210> 150
   <211> 21
   <212> DNA
   <213> Homo sapiens
<220>
   <221> misc_feature
   <223> KCNQ1; Primer ID KV11.1
<400> 150
   cactcaaggc cgagcctgcc t
   21
<210> 151
   <211> 20
   <212> DNA
   <213> Homo sapiens
<220>
   <221> misc_feature
   <223> KCNQ1; Primer ID KV5NA
<400> 151
   gccccacacc atctccttcg
   20
<210> 152
   <211> 18
   <212> DNA
   <213> Homo sapiens
<220>
   <221> misc_feature
   <223> KCNQ1; Primer ID KV6NI
<400> 152
   taccctaacc cgggccac 18
<210> 153
   <211> 20
   <212> DNA
   <213> Homo sapiens
<220>
   <221> misc_feature
   <223> KCNQ1; Primer ID KVDFn
<400> 153
   gaggagaagt gatgcgtgtc 20
<210> 154
   <211> 20
   <212> DNA
   <213> Homo sapiens
<220>
   <221> misc_feature
   <223> KCNQ1; Primer ID KVDRn
<400> 154
   ggcaggacct gggcaccctc 20
<210> 155
   <211> 20
   <212> DNA
   <213> Homo sapiens
<220>
   <221> misc_feature
   <223> KCNQ1; Primer ID KV1A1F
<400> 155
   cttcgctgca gctcccggtg 20
<210> 156
   <211> 20
   <212> DNA
   <213> Homo sapiens
<220>
   <221> misc_feature
   <223> KCNQ1; Primer ID KV1A2R
<400> 156
   acgcgcgggt ctaggctcac
   20
<210> 157
   <211> 21
   <212> DNA
   <213> Homo sapiens
<220>
   <221> misc_feature
   <223> KCNQ1; Primer ID KK2F
<400> 157
   gactgccgtg tccctgtctt g
   21
<210> 158
   <211> 21
   <212> DNA
   <213> Homo sapiens
<220>
   <221> misc_feature
   <223> KCNQ1; Primer ID KK2R
<400> 158
   gccatgcctt cagatgctac g
   21
<210> 159
   <211> 20
   <212> DNA
   <213> Homo sapiens
<220>
   <221> misc_feature
   <223> KCNQ1; Primer ID KK12Rn
<400> 159
   ctgagggcag gaaggctcag
   20
<210> 160
   <211> 20
   <212> DNA
   <213> Homo sapiens
<220>
   <221> misc_feature
   <223> KCNQ1; Primer ID KK14F1

<400> 160
   ctgtctgtcc cacagacgac
   20
<210> 161
   <211> 20
   <212> DNA
   <213> Homo sapiens
<220>
   <221> misc_feature
   <223> KCNQ1; Primer ID KK14Rn
<400> 161
   ctgggcccag agtaactgac
   20
<210> 162
   <211> 21
   <212> DNA
   <213> Homo sapiens
<220>
   <221> misc_feature
   <223> KCNQ1; Primer ID KK15Fn
<400> 162
   cggcccaccc cagcacttgg c
   21
<210> 163
   <211> 21
   <212> DNA
   <213> Homo sapiens
<220>
   <221> misc_feature
   <223> KCNQ1; Primer ID KK15Rn
<400> 163
   gaaccaccgc aggccggcgc g
   21
<210> 164
   <211> 18
   <212> DNA
   <213> Homo sapiens
<220>
   <221> misc_feature
   <223> KCNQ1; Primer ID KK16Fn
<400> 164
   cgtctgcctt tgtccccg
   18
<210> 165
   <211> 18
   <212> DNA
   <213> Homo sapiens
<220>
   <221> misc_feature
   <223> KCNQ1; Primer ID KK16Rn
<400> 165
   cactcttggc ctcccctc
   18
<210> 166
   <211> 19
   <212> DNA
   <213> Homo sapiens
<220>
   <221> misc_feature
   <223> KCNE1; Primer ID MINK F
<400> 166
   ctgcagcagt ggaccctta
   19
<210> 167
   <211> 20
   <212> DNA
   <213> Homo sapiens
<220>
   <221> misc_feature
   <223> KCNE1; Primer ID MINK 1R
<400> 167
   agcttcttgg agcggatgta
   20
<210> 168
   <211> 20
   <212> DNA
   <213> Homo sapiens
<220>
   <221> misc_feature
   <223> KCNE1; Primer ID MINK 2F
<400> 168
   gtcctcatgg tactgggatt
   20
<210> 169
   <211> 19
   <212> DNA
   <213> Homo sapiens
<220>
   <221> misc_feature
   <223> KCNE1; Primer ID MINK R
<400> 169
   tttagccagt ggtggggtt
   19
<210> 170
   <211> 24
   <212> DNA
   <213> Homo sapiens
<220>
   <221> misc_feature
   <223> KCNE2; Primer ID MINK2-F2
<400> 170
   ccgttttcct aaccttgttc gcct
   24
<210> 171
   <211> 26
   <212> DNA
   <213> Homo sapiens
<220>
   <221> misc_feature
   <223> KCNE2; Primer ID MINK2-R2
<400> 171
   gccacgatga tgaaagagaa cattcc
   26
<210> 172
   <211> 23
   <212> DNA
   <213> Homo sapiens
<220>
   <221> misc_feature
   <223> KCNE2; Primer ID MINK2-F3
<400> 172
   gtcatcctgt acctcatggt gat
   23
<210> 173
   <211> 24
   <212> DNA
   <213> Homo sapiens
<220>
   <221> misc_feature
   <223> KCNE2; Primer ID MINK2-R3
<400> 173
   tggacgtcag atgttagctt ggtg
   24
<210> 174
   <211> 21
   <212> DNA
   <213> Homo sapiens
<220>
   <221> misc_feature
   <223> SNC5A; Primer ID SCN2.1Fnew
<400> 174
   ccctgctctc tgtccctggg c
   21
<210> 175
   <211> 20
   <212> DNA
   <213> Homo sapiens
<220>
   <221> misc_feature
   <223> SNC5A; Primer ID SCN2.1Rnew
<400> 175
   gcagcccctc tcggctctcc
   20
<210> 176
   <211> 21
   <212> DNA
   <213> Homo sapiens
<220>
   <221> misc_feature
   <223> SNC5A; Primer ID SCN2.2Fnew
<400> 176
   catggcagag aagcaagccc g
   21
<210> 177
   <211> 22
   <212> DNA
   <213> Homo sapiens
<220>
   <221> misc_feature
   <223> SNC5A; Primer ID SCN6Fnew
<400> 177
   cctcctctga ctgtgtgtct cc
   22
<210> 178
   <211> 21
   <212> DNA
   <213> Homo sapiens
<220>
   <221> misc_feature
   <223> SNC5A; Primer ID SCN10Fnew
<400> 178
   ccagtgaggg tgacctctgc c
   21
<210> 179
   <211> 21
   <212> DNA
   <213> Homo sapiens
<220>
   <221> misc_feature
   <223> SNC5A; Primer ID SCN10Rnew
<400> 179
   ggcttagagg ctcctcggtg g
   21
<210> 180
   <211> 21
   <212> DNA
   <213> Homo sapiens
<220>
   <221> misc_feature
   <223> SNC5A; Primer ID SCN16F
<400> 180
   gagccagaga ccttcacaag g
   21
<210> 181
   <211> 24
   <212> DNA
   <213> Homo sapiens
<220>
   <221> misc_feature
   <223> SNC5A; Primer ID
<220>
   <221> misc_feature
   <223> SNC5A; Primer ID SCN17.1Fnew
<400> 181
   gcttggcatg gtgcagtgcc ttgg
   24
<210> 182
   <211> 22
   <212> DNA
   <213> Homo sapiens
<220>
   <221> misc_feature
   <223> SNC5A; Primer ID SCN17.1Rnew
<400> 182
   gaggcacctt ctccgtctct gg
   22
<210> 183
   <211> 24
   <212> DNA
   <213> Homo sapiens
<220>
   <221> misc_feature
   <223> SNC5A; Primer ID SCN20Fnew
<400> 183
   cattagatgt gggcattcac aggc
   24
<210> 184
   <211> 21
   <212> DNA
   <213> Homo sapiens
<220>
   <221> misc_feature
   <223> SNC5A; Primer ID SCN20Rnew
<400> 184
   ccagccgtcc ctgccacaac c
   21
<210> 185
   <211> 21
   <212> DNA
   <213> Homo sapiens
<220>
   <221> misc_feature
   <223> SNC5A; Primer ID SCN21Fnew
<400> 185
   ggtccaggct tcatgtccac c
   21
<210> 186
   <211> 22
   <212> DNA
   <213> Homo sapiens
<220>
   <221> misc_feature
   <223> SNCSA; Primer ID
<220>
   <221> misc_feature
   <223> SNC5A; Primer ID SCN21Rnew
<400> 186
   ggcaatgggt ttctccttcc tg
   22
<210> 187
   <211> 22
   <212> DNA
   <213> Homo sapiens
<220>
   <221> misc_feature
   <223> SNC5A; Primer ID SCN22Fnew
<400> 187
   ggggagctgt tcccatcctc cc
   22
<210> 188
   <211> 21
   <212> DNA
   <213> Homo sapiens
<220>
   <221> misc_feature
   <223> SNC5A; Primer ID SCN22Rnew
<400> 188
   cgcctcccac tccctggtgg g
   21
<210> 189
   <211> 23
   <212> DNA
   <213> Homo sapiens
<220>
   <221> misc_feature
   <223> SNC5A; Primer ID SCN23.1F
<400> 189
   ttgaaaagga aatgtgctct ggg
   23
<210> 190
   <211> 22
   <212> DNA
   <213> Homo sapiens
<220>
   <221> misc_feature
   <223> SNC5A; Primer ID SCN23.1R
<400> 190
   ttgttcacga tggtgtagtt ca
   22
<210> 191
   <211> 21
   <212> DNA
   <213> Homo sapiens
<220>
   <221> mise feature
   <223> SNC5A; Primer ID SCN23.2F
<400> 191
   ccagacagag ggagacttgc c
   21
<210> 192
   <211> 22
   <212> DNA
   <213> Homo sapiens
<220>
   <221> misc_feature
   <223> SNC5A; Primer ID SCN25Fnew
<400> 192
   cccagcctgt ctgatctccc tg
   22
<210> 193
   <211> 21
   <212> DNA
   <213> Homo sapiens
<220>
   <221> misc_feature
   <223> SNC5A; Primer ID SCN25Rnew
<400> 193
   ccaccctacc cagcccagtg g
   21
<210> 194
   <211> 20
   <212> DNA
   <213> Homo sapiens
<220>
   <221> misc_feature
   <223> KCNH2; Primer ID HM1F
<400> 194
   catgggctca ggatgccggt
   20
<210> 195
   <211> 22
   <212> DNA
   <213> Homo sapiens
<220>
   <221> misc_feature
   <223> KCNH2; Primer ID KCNH2-1R
<400> 195
   cattgactcg cacttgccga cg
   22
<210> 196
   <211> 20
   <212> DNA
   <213> Homo sapiens
<220>
   <221> misc_feature
   <223> KCNH2; Primer ID H2F
<400> 196
   cgctcacgcg cactctcctc
   20
<210> 197
   <211> 20
   <212> DNA
   <213> Homo sapiens
<220>
   <221> misc_feature
   <223> KCNH2; Primer ID KH2R
<400> 197
   ttgaccccgc ccctggtcgt
   20
<210> 198
   <211> 21
   <212> DNA
   <213> Homo sapiens
<220>
   <221> misc_feature
   <223> KCNH2; Primer ID H3F
<400> 198
   ccactgagtg ggtgccaagg g
   21
<210> 199
   <211> 21
   <212> DNA
   <213> Homo sapiens
<220>
   <221> misc_feature
   <223> KCNH2; Primer ID H3R
<400> 199
   gagaccacga acccctgagc c
   21
<210> 200
   <211> 21
   <212> DNA
   <213> Homo sapiens
<220>
   <221> misc_feature
   <223> KCNH2; Primer ID H4.1F

<400> 200
   cccacgacca cgtgcctctc c
   21
<210> 201
   <211> 18
   <212> DNA
   <213> Homo sapiens
<220>
   <221> misc_feature
   <223> KCNH2; Primer ID H8R
<400> 201
   gcctgccacc cactggcc
   18
<210> 202
   <211> 22
   <212> DNA
   <213> Homo sapiens
<220>
   <221> misc_feature
   <223> KCNH2; Primer ID KH9F
<400> 202
   atggtggagt agagtgtggg tt
   22
<210> 203
   <211> 21
   <212> DNA
   <213> Homo sapiens
<220>
   <221> misc_feature
   <223> KCNH2; Primer ID
<220>
   <221> misc_feature
   <223> KCNH2; Primer ID KH9R
<400> 203
   agaaggctcg cacctcttga g
   21
<210> 204
   <211> 21
   <212> DNA
   <213> Homo sapiens
<220>
   <221> misc_feature
   <223> KCNH2; Primer ID KH10F
<400> 204
   gagaaggtgc ctgctgcctg g
   21
<210> 205
   <211> 21
   <212> DNA
   <213> Homo sapiens
<220>
   <221> misc_feature
   <223> KCNH2; Primer ID KH10R
<400> 205
   acagctggaa gcaggaggat g
   21
<210> 206
   <211> 22
   <212> DNA
   <213> Homo sapiens
<220>
   <221> misc_feature
   <223> KCNH2; Primer ID H11F
<400> 206
   ggcaggagag cactgaaagg gc
   22
<210> 207
   <211> 22
   <212> DNA
   <213> Homo sapiens
<220>
   <221> misc_feature
   <223> KCNH2; Primer ID H11R
<400> 207
   ggtaaagcag acacggccca cc
   22
<210> 208
   <211> 22
   <212> DNA
   <213> Homo sapiens
<220>
   <221> misc_feature
   <223> KCNH2; Primer ID H12A F
<400> 208
   gttctcctcc cctctctgag gc
   22
<210> 209
   <211> 21
   <212> DNA
   <213> Homo sapiens
<220>
   <221> misc_feature
   <223> KCNH2; Primer ID H12B R
<400> 209
   gggtagacgc accaccgctg c
   21
<210> 210
   <211> 21
   <212> DNA
   <213> Homo sapiens
<220>
   <221> misc_feature
   <223> KCNH2; Primer ID H13F
<400> 210
   gcagcggtgg tgcgtctacc c
   21
<210> 211
   <211> 22
   <212> DNA
   <213> Homo sapiens
<220>
   <221> misc_feature
   <223> KCNH2; Primer ID H13R
<400> 211
   gacctggacc agactccagg gc
   22
<210> 212
   <211> 20
   <212> DNA
   <213> Homo sapiens
<220>
   <221> misc_feature
   <223> KCNH2; Primer ID H14R
<400> 212
   gggtacatcg aggaagcagg
   20